# EUROPEAN PATENT SPECIFICATION

(11) **EP 3 525 584 B1**
(45) Date of publication and mention of the grant of the patent: **03.05.2023**
(21) Application number: 17859545.0
(22) Date of filing: 10.10.2017
(51) Int. Cl.: A01N 1/02, C12N 5/02, C12N 5/07, C12Q 1/02, G01N 33/15, G01N 33/50

(54) **MULTI-ORGAN "BODY ON A CHIP" APPARATUS UTILIZING A COMMON MEDIA**
MULTIORGAN- KÖRPER-AUF-EINEM-CHIP -VORRICHTUNG UNTER VERWENDUNG EINES GEMEINSAMEN MEDIUMS
APPAREIL "CORPS-SUR-PUCE" MULTI-ORGANE UTILISANT UN MILIEU COMMUN

(30) Priority: 14.10.2016 US 201662408113 P; 20.06.2017 US 201762522318 P
(43) Date of publication of application: 21.08.2019
(73) Proprietor: Wake Forest University Health Sciences, Winston-Salem, NC 27157 (US)
(72) Inventor: SKARDAL, Aleksander, Clemmons, North Carolina 27012 (US); SHUPE, Thomas, Mocksville, North Carolina 27028 (US); ATALA, Anthony, Winston-Salem, North Carolina 27104 (US)
(74) Representative: Marks & Clerk LLP
(86) International application number: PCT/US2017/055823
(87) International publication number: WO 2018/071354

(56) References cited:
- WO-A1-2013/086502
- WO-A1-2016/049363
- WO-A1-2016/064648
- WO-A1-2016/100227
- WO-A1-2017/059171
- JONG HWAN SUNG ET AL: "A microfluidic device for a pharmacokinetic-pharmacodynamic (PK-PD) model on a chip", LAB ON A CHIP, vol. 10, no. 4, 1 January 2010 (2010-01-01), page 446, XP055184167, ISSN: 1473-0197, DOI: 10.1039/b917763a
- SKARDAL ALEKSANDER ET AL: "Organoid-on-a-chip and body-on-a-chip systems for drug screening and disease modeling", DRUG DISCOVERY TODAY, ELSEVIER, AMSTERDAM, NL, vol. 21, no. 9, 12 July 2016 (2016-07-12), pages 1399-1411, XP029741819, ISSN: 1359-6446, DOI: 10.1016/J.DRUDIS.2016.07.003

## Description

### Field of the Invention

The present invention generally relates to multi-tissue body-on-a-chip apparatus.

### Background of the Invention

The total cost for drug screening and the development process for effective and safe therapeutic agents can exceed 2 billion USD. This includes costs dedicated to target identification, drug screening, regulatory studies, and therapeutic agent manufacturing for clinical trials. Despite extensive preclinical testing and high costs, 90% of drugs that enter Phase I clinical trials fail ¹. There is a critical need for improved model systems that can effectively test the effects of drugs, chemicals, and biological agents on the human body ^{2,3}.

Traditional *in vitro* 2D cultures, currently the norm for early drug compound screening, fail to recapitulate the 3D microenvironment of *in vivo* tissues ^{4,5}. Standard tissue cultures have three major differences from native tissue microenvironments: substrate topography, substrate stiffness, and most importantly, a 2D rather than 3D architecture. As a consequence, 2D culture places a selective pressure on cells, substantially altering their original phenotypic properties. Drug diffusion kinetics are also not accurately modeled in 2D tissue culture, and drug doses effective in 2D are often ineffective when scaled to patients^{4,6,7}. Animal models serve as the gold standard for biological testing, but have several drawbacks, including uncertainties in interpretation of the results. The main weakness of animal models is that responses to external stimuli in animals are not necessarily predictive of those in humans⁸. Due to interspecies differences in metabolism and immunology, animal models are often poor predictors of human efficacy and toxicology, contributing to drug attrition rates.

Advancements in cell aggregate culture systems, and microengineering/microfluidics technologies have contributed to the evolution of 3D human tissue-on-a chip models⁹. Currently, many organs-on-chip systems exist^{10,11}, as well as several on-chip disease models¹⁰. Despite advances to date, organs-on-chip models still lack many of the elements of normal human tissue.

### Summary of the Invention

The invention is set out in the appended claims and disclosure directed to a multi-tissue body-on-a-chip apparatus. In some embodiments, the apparatus comprises: at least a first, second, and third chamber in fluid communication with one another; liver tissue in said first chamber; cardiac muscle tissue in said second chamber; lung tissue in said third chamber; and a common aqueous growth media in said first, second, and third chamber. In some embodiments, the apparatus comprises: at least a first, second, third, fourth, fifth, and sixth chamber in fluid communication with one another; liver tissue in said first chamber, heart tissue in said second chamber, lung tissue in said third chamber, brain tissue in said fourth chamber, colon tissue in said fifth chamber, and testis or ovary tissue in said sixth chamber; and a common aqueous growth media in said first, second, third, fourth, fifth and sixth chambers.

Methods of using an apparatus of the present disclosure may comprise: *(a)* providing an apparatus of the present invention; and *(b)* circulating a common aqueous growth media through all of the chambers of the apparatus for a time of at least 1, 4, 8, or 11 days. The methods may include adding a test compound to the common aqueous growth media; and then detecting a pharmacological or toxicological response to the test compound in at least one, or a plurality of tissues present in the apparatus.

Methods of *in vitro* drug screening, toxicology screening, and/or disease modeling may comprise providing an apparatus of the present disclosure; and detecting a pharmacological or toxicological response in at least one, or a plurality of tissues present in the apparatus.

It is noted that aspects of the disclosure described with respect to one embodiment, may be incorporated in a different embodiment although not specifically described relative thereto. That is, all embodiments and/or features of any embodiment can be combined in any way and/or combination. These and other objects and/or aspects of the present disclosure are explained in detail in the specification set forth below. Further features, advantages and details of the present disclosure will be appreciated by those of ordinary skill in the art from a reading of the figures and the detailed description of the preferred embodiments that follow, such description being merely illustrative of the present disclosure.

### Brief Description of the Drawings

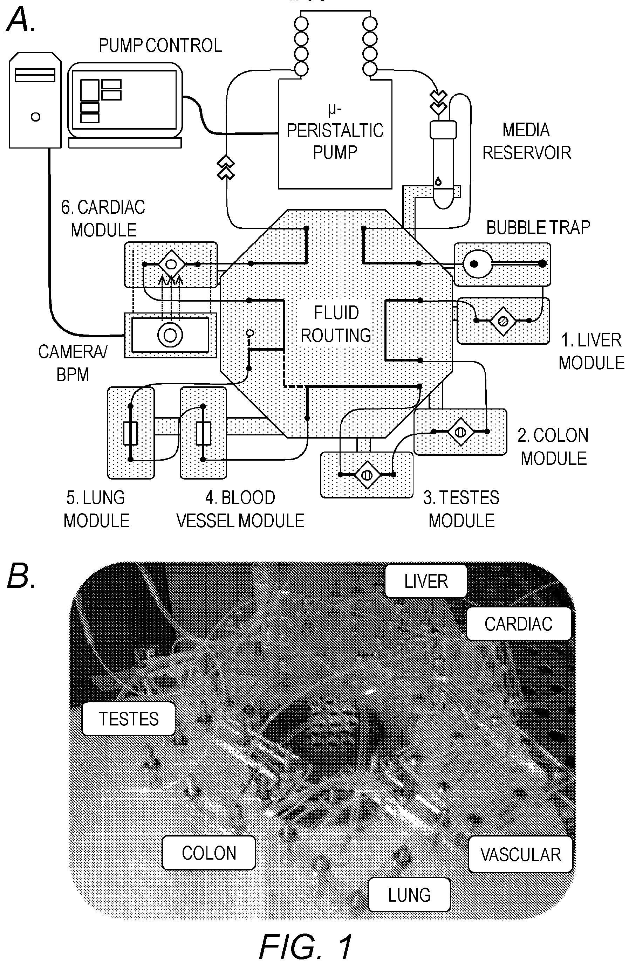

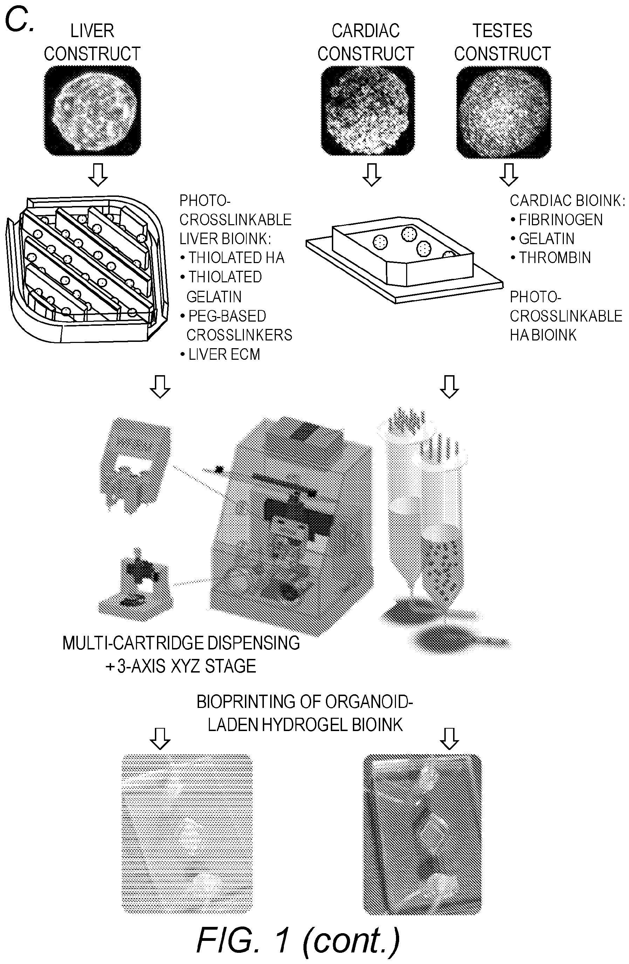

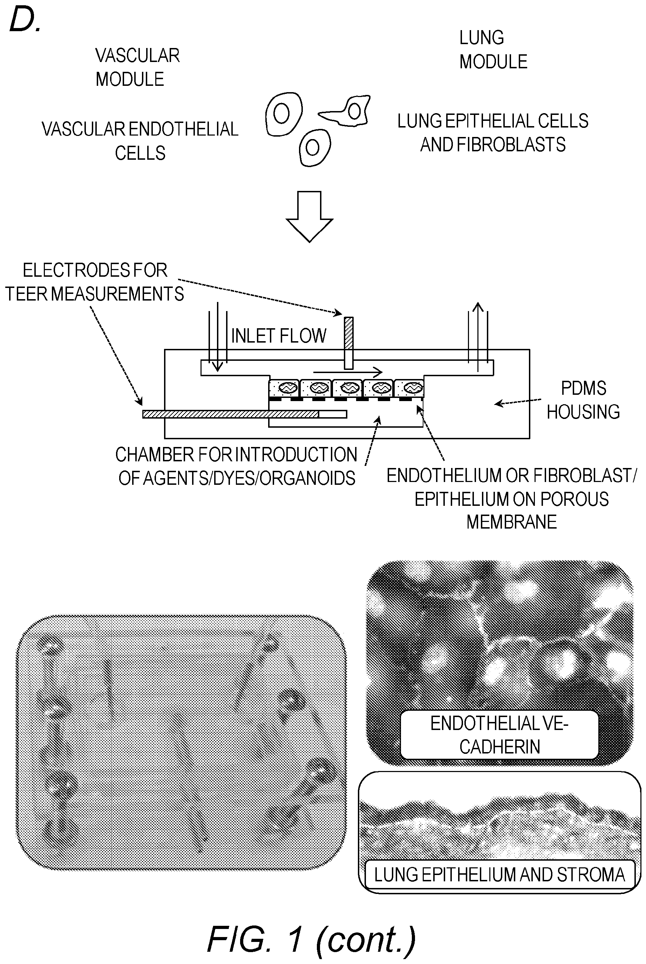

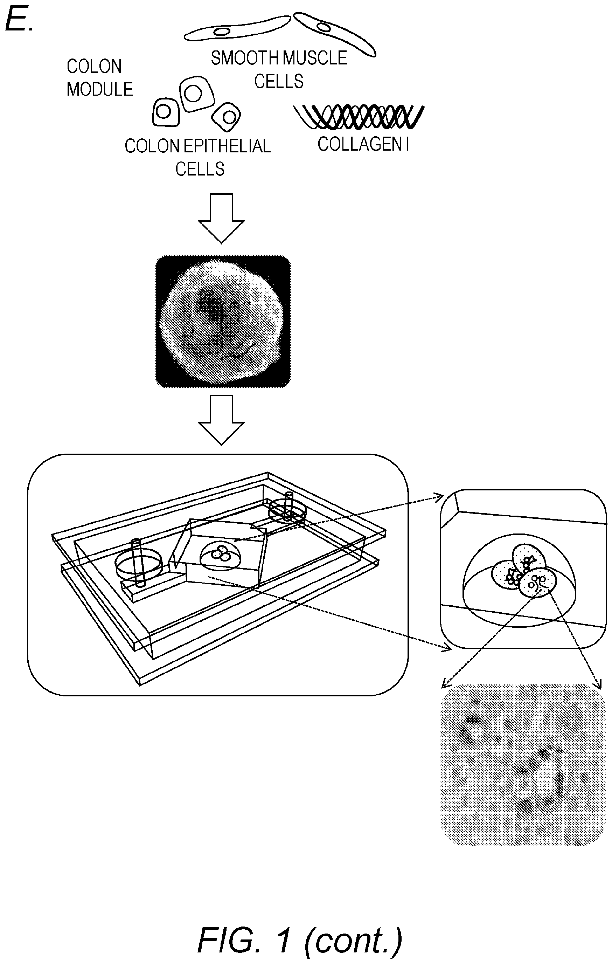

**Fig. 1****.** Overall design and implementation strategy for the 6-tissue-representative body-on-a-chip system using a variety of biofabrication approaches. Panels A-B) Illustration and photograph of the modular body-on-a-chip hardware system set up for maintenance of 6 tissue model. Individual microfluidic microreactor units house each organoid or tissue model, and are connected via a central fluid routing breadboard, allowing for straightforward "plug-and-play" system preparation initialization. Panels C-E) General overview of how each tissue type is prepared for the system. Panel C) Liver, cardiac, and testes modules are created by bioprinting spherical organoids within customized bioinks, resulting in 3D hydrogel constructs that are placed into the microreactor devices. Panel D) Vascular and lung modules are formed by creating layers of cells over porous membranes within microfluidic devices. Introduction of TEER (trans-endothelial [or epithelial] electrical resistance sensors allows monitoring of tissue barrier function integrity overtime. Panel E) Colon modules are created by encapsulating colon smooth muscle cells and colon epithelial cells within collagen type I in polymer molds. The smooth muscle cells align the collagen and contract the organoids, after which the organoids take on a donut-like shape. The individual organoids are then encapsulated in a hyaluronic acid hydrogel in order to immobilize them within the microfluidic devices.

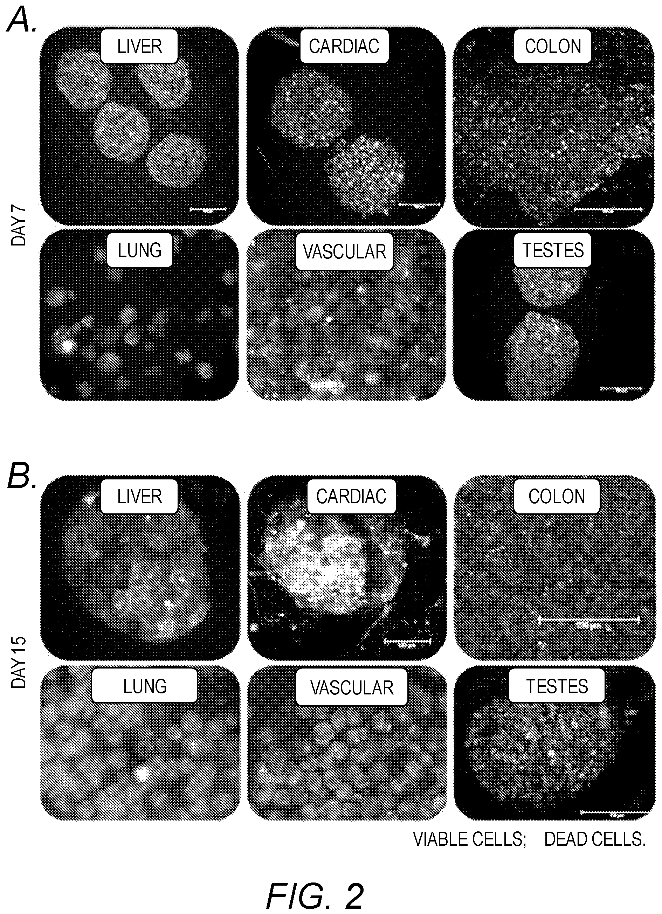

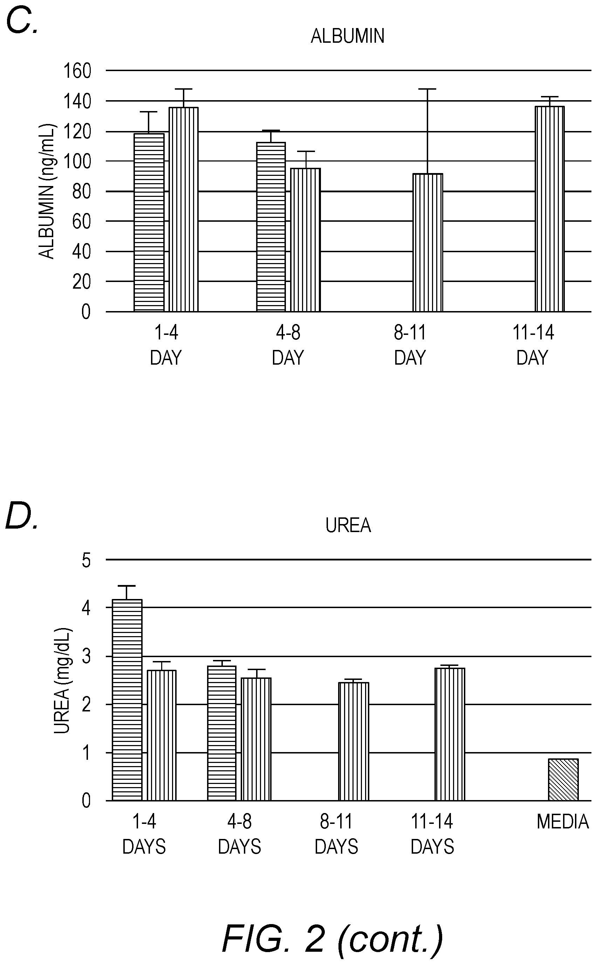

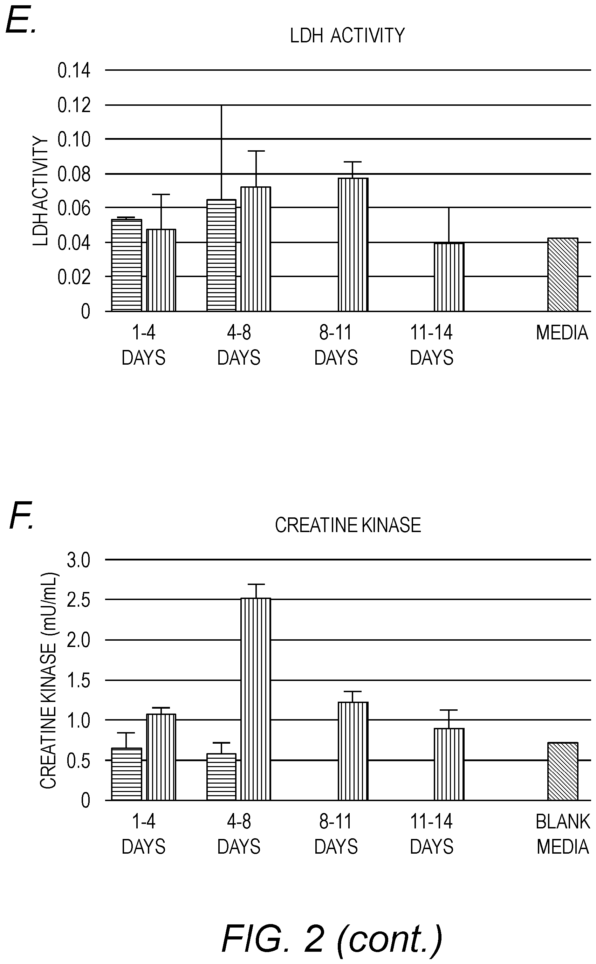

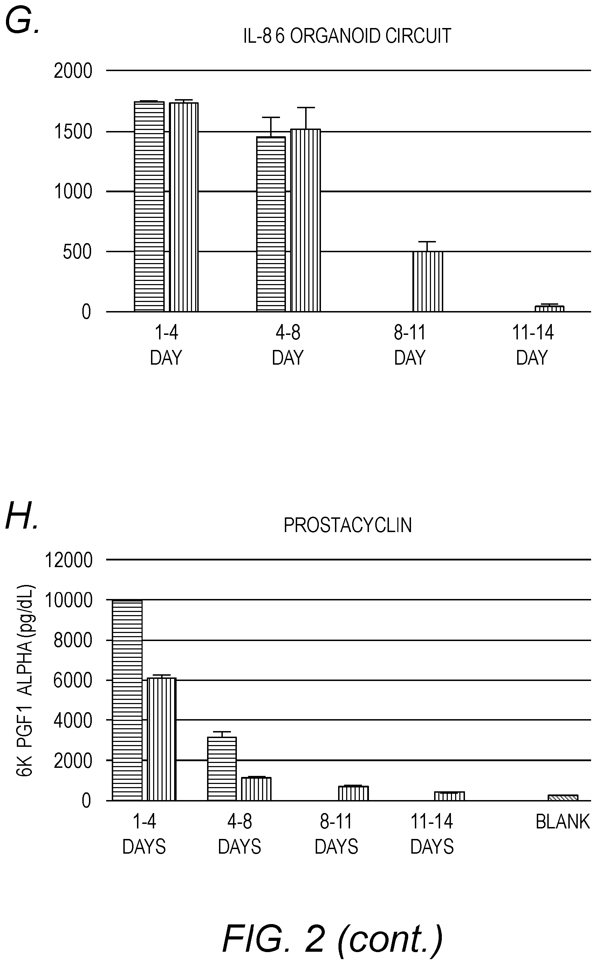

**Fig. 2****.** Six organoid maintenance under a common media the body-on-a-chip system. Panel A) 7-day and panel B) 14-day viability of liver, cardiac, colon, lung, vascular, and testes tissue models in the integrated system. Green - Calcein AM-stained viable cells; Red - Ethidium homodimer-stained dead cells. Panels C-H) Biomarkers in solution show that the system reaches an apparent equilibrium state. Panels C-D) Albumin and urea quantification shows relatively consistent secreted albumin and urea in the media over time, that is significantly higher than media controls. Panel E) LDH, a general marker of lysed cells and toxicity, remains at levels near that of media controls, indicating relatively low cell death. Panel F) Creatine kinase, a biomarker released by lysed cardiomyocytes upon cell death remains relatively low (with the exception of a spike on day 8 quantification), but returns to a low concentration near media control levels. Panels G-H) IL-8 and prostacyclin, proteins released from lung epithelium and vascular endothelium, respectively, initially increase, but decrease to baseline levels over time, suggesting that the lung and vascular constructs experienced stress upon system initiation, but equilibrate to a steady state over time.

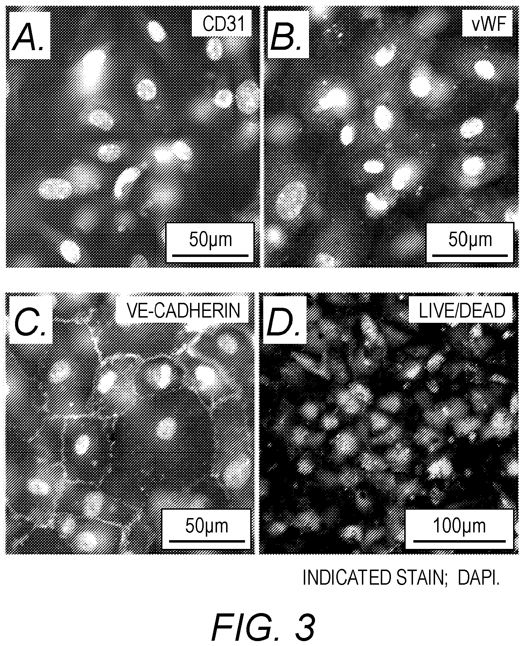

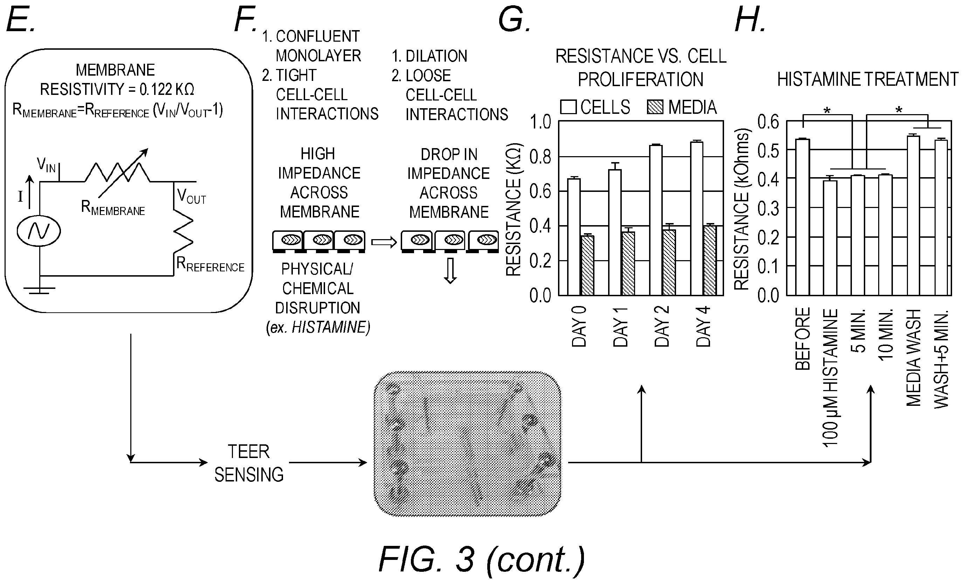

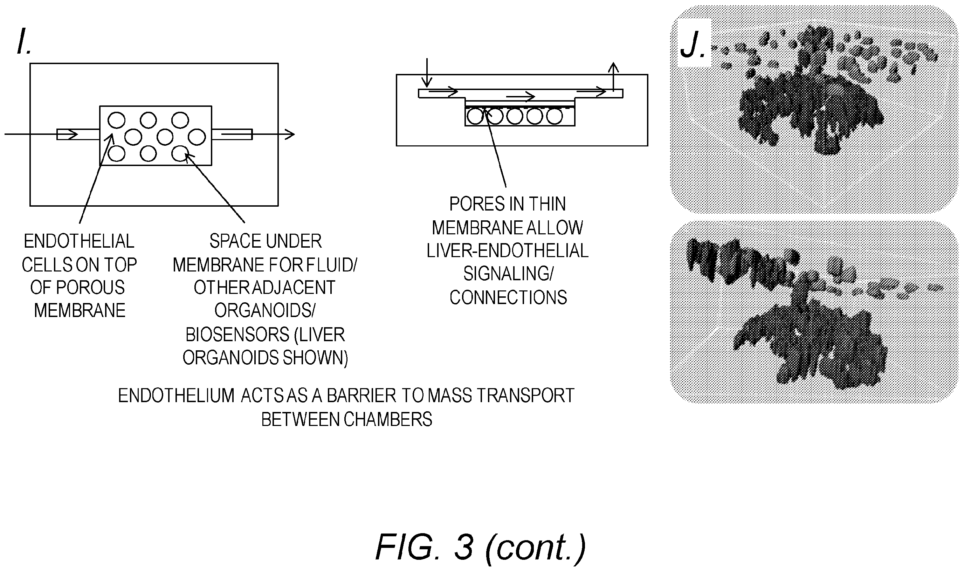

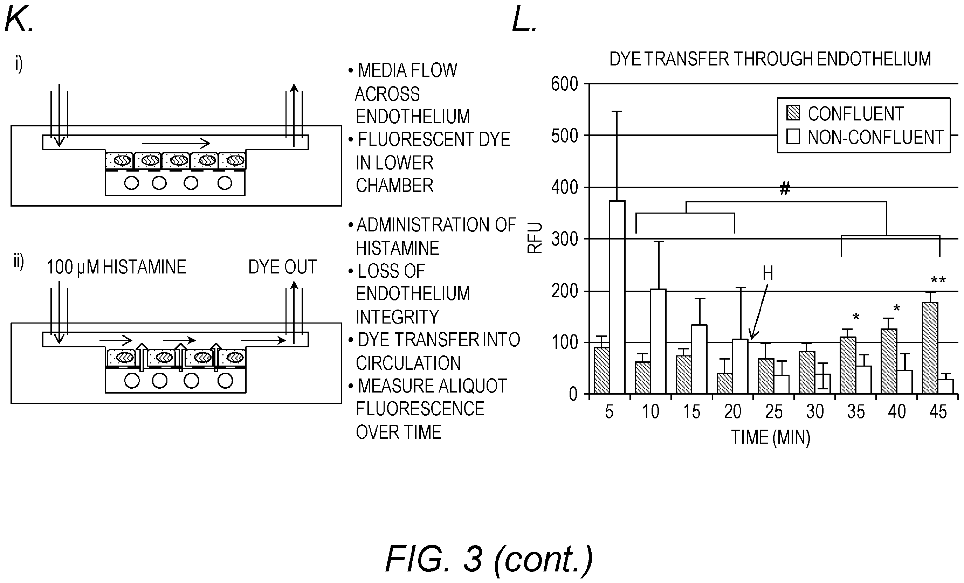

**Fig. 3****.** Vasculature-on-a-chip: Endothelium marker expression; trans-endothelial electrical resistance (TEER) measurement responses; and mass transport changes. Panels a-c) Immunostaining of endothelial markers; panel a) CD31, panel b) vWF, and panel c) VE-cadherin. Panel d) Live/Dead viability/cytotoxicity staining of vascular module endothelium. Green - Calcein AM-stained viable cells; Red - Ethidium homodimer-stained dead cells. Panel e) A circuit diagram of the TEER measurement system. Panel f) Under baseline conditions, the endothelium maintains integrity, resulting in relatively high resistance across the monolayer. Under injury, or certain stimuli, the endothelium becomes discontinuous, resulting in a drop in resistance across the monolayer. Inset image - Vascular module with TEER sensors. Panel g) After seeding of cells onto the semi-porous membrane, daily TEER measurements show an increase in cellularity in the monolayer over time in culture. Statistical significance in panel g): * < 0.05. Panel b) Functional response of the endothelium to histamine administration. Histamine causes disruption of the endothelium, resulting in a significant decrease in resistance across the membrane. Washing out with fresh medium results in recovery of resistance. Statistical significance in panel h): * < 0.05. Panel i) A depiction of the vasculature-on-a-chip device in which liver organoids are situated in the liver bioink underneath the endothelium, while flow is preserved above the endothelial layer. Panel j) Two macro-confocal 3D reconstruction images showing the close interactions of the endothelium (visible primarily in blue) and liver organoid cells (visible primarily in red). Panel k) Endothelial patency-based dye exclusion and transfer as an effect of histamine administration. Fluorescent 15 kDa soluble gelatin dye is held in the lower chamber away from the circulation under normal media conditions. Upon histamine administration, dye can permeate through the endothelium and into circulation. Panel l) Fluorescent intensity readings of aliquots taken from the module before and after histamine administration. Fluorescence was read using excitation wavelength 584 nm and emission wavelength 612 nm. Statistical significance in panel i): * and ** denote p < 0.05 and p < 0.01, respectively, between the confluent and non-confluent conditions at the indicated time point. # denotes that within the confluent experimental group, fluorescent intensity at time-points 35, 40, and 45 minutes is significantly greater (p < 0.05) than at time points 10, 15, and 20, showing the presence of positive dye transfer post-histamine administration at 20 minutes.

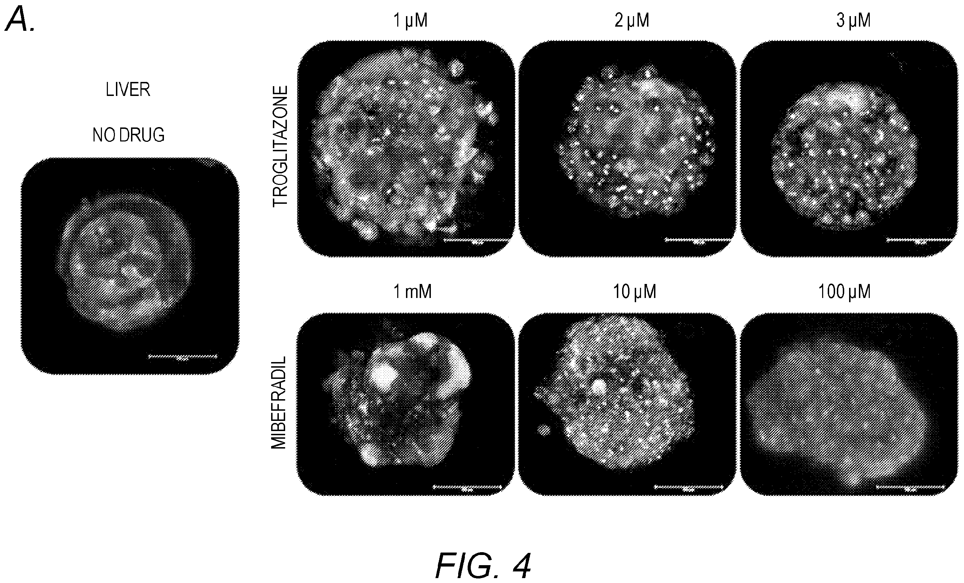

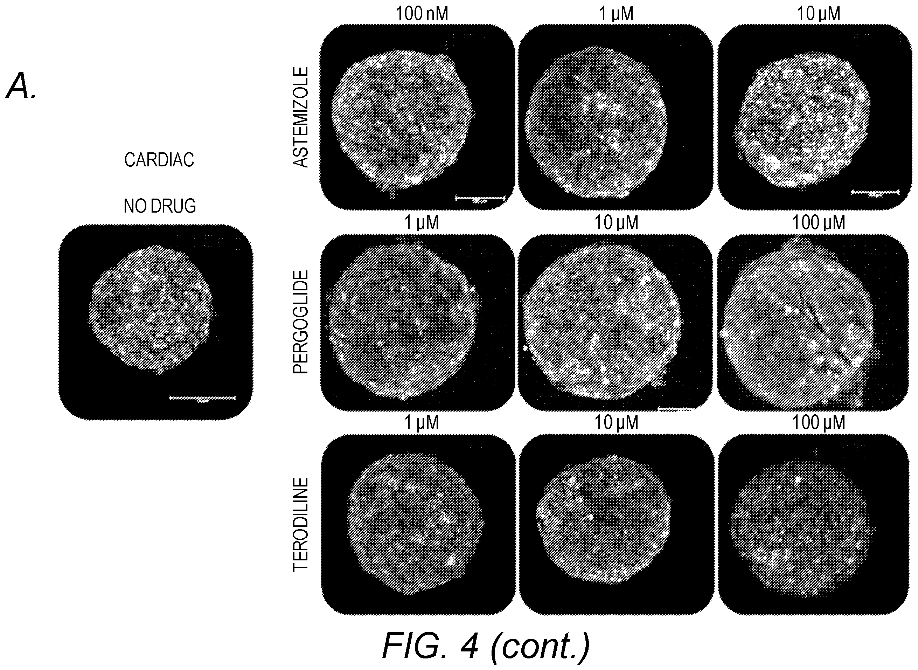

**Fig. 4****.** LIVE/DEAD staining of 3D liver and cardiac organoids in response to screens with FDA-recalled drugs. Panel a) Liver: Troglitazone and mibefradil. Panel b) Cardiac: Astemizole, pergoglide, and terodiline. Green: calcein AM-stained viable cells; Red: ethidium homodimer-stained dead cell.

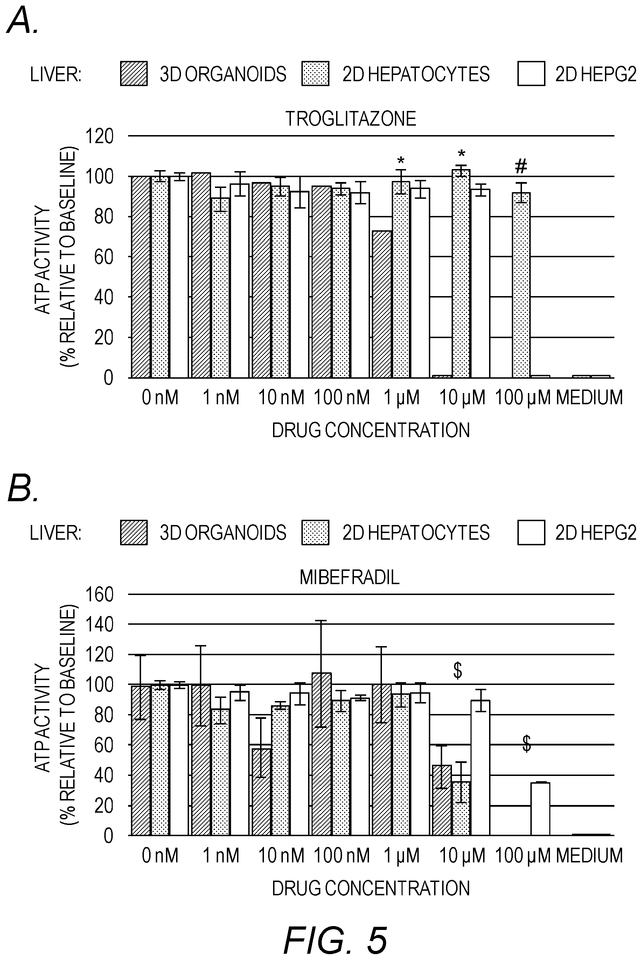

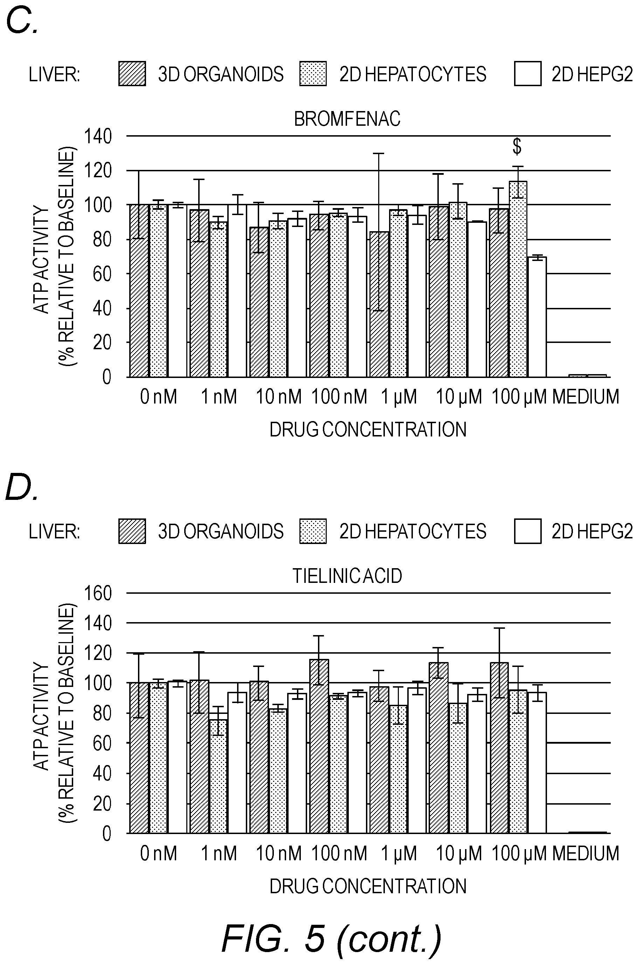

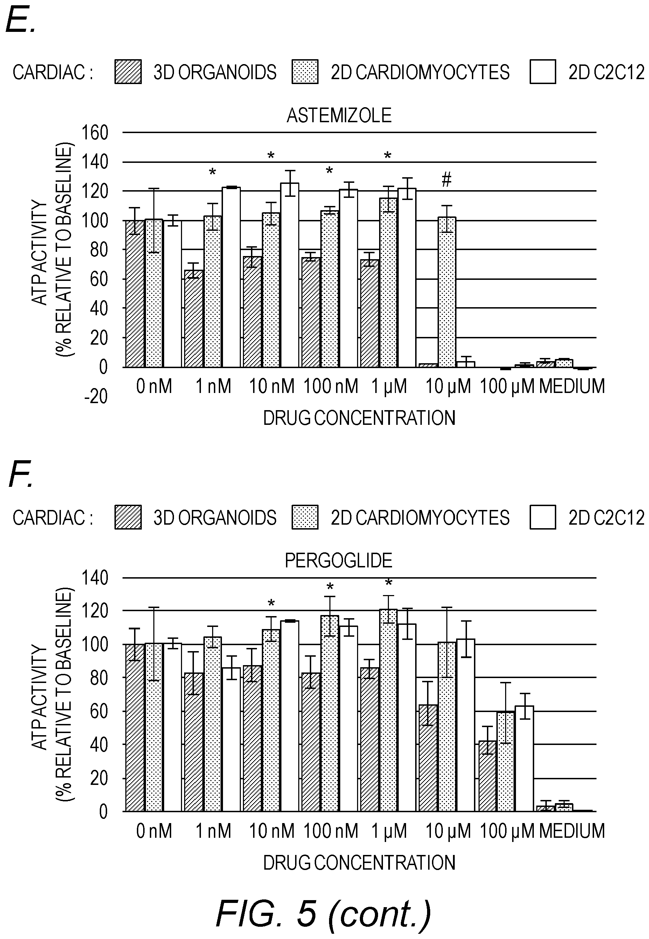

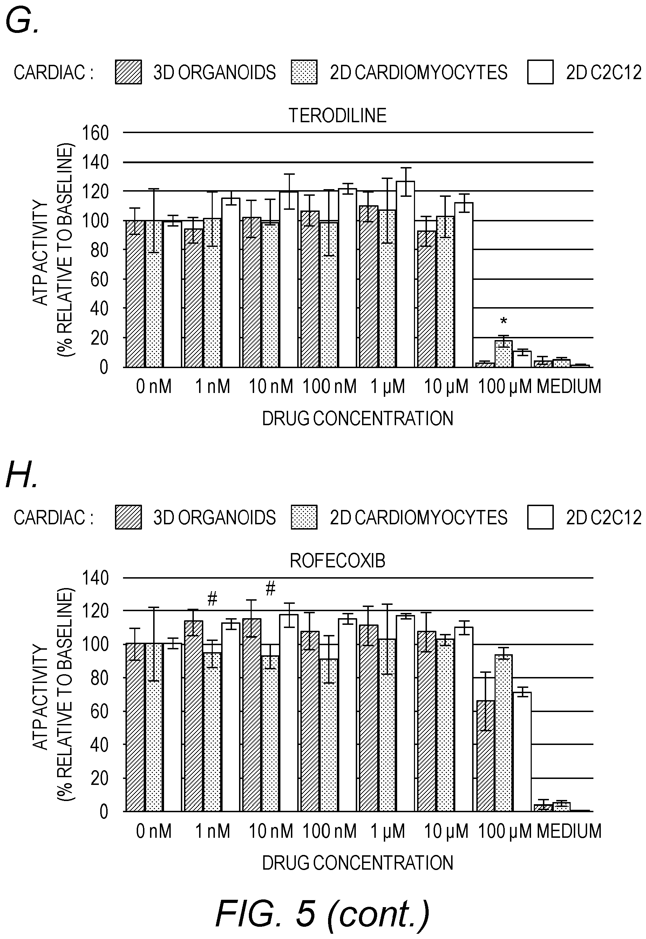

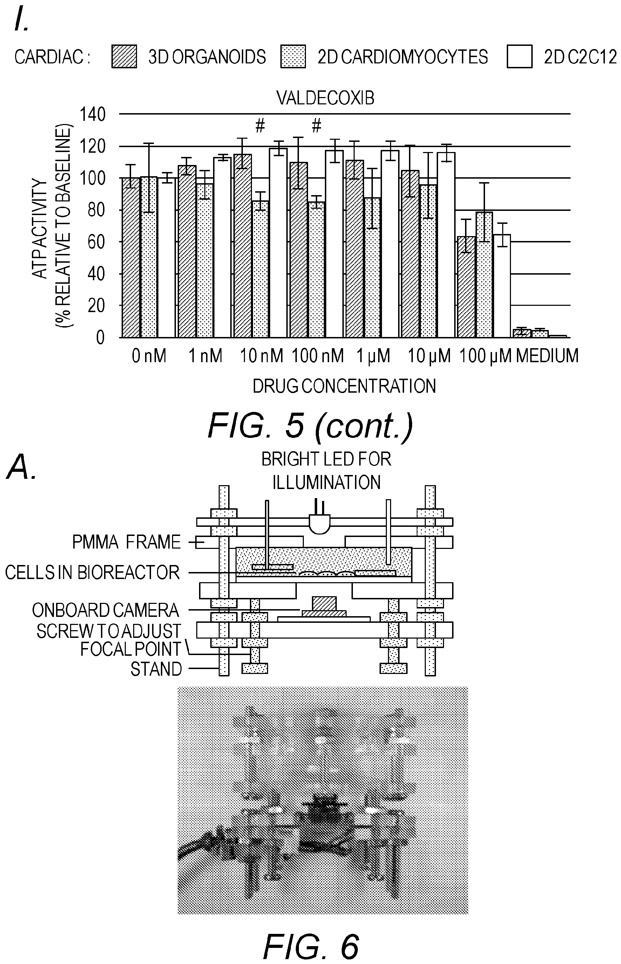

**Fig. 5****.** Comparison of ATP activity in response to drug screens of FDA-recalled drugs between 3D liver and cardiac organoids, 2D hepatocyte and cardiomyocyte cultures, and 2D HepG2 and C2C12 cell line cultures. Panels a-d) ATP activity of liver organoids, 2D primary human hepatocytes, and 2D hepG2 cell line cultures in response to panel a) troglitazone, panel b) mibefradil, panel c) bromfenac, and panel d) tielinic acid. Panels e-i) ATP activity of cardiac organoids, 2D iPS-derived cardiomyocytes, and 2D C2C12 cell line cultures in response to panel e) astemizole, panel f) pergoglide, panel g) terodiline, panel h) rofecoxib, and panel i) valdecoxib. Comparisons between groups were normalized to the no drug baseline ATP activity levels. Statistical significance: * p < 0.05, 3D organoids versus both 2D cultures; # p < 0.05, 2D hepatocytes/cardiomyocytes versus both 3D organoids and 2D cell lines; $ p < 0.05, 2D cell line versus both 3D organoids and 2D hepatocytes/cardiomyocytes.

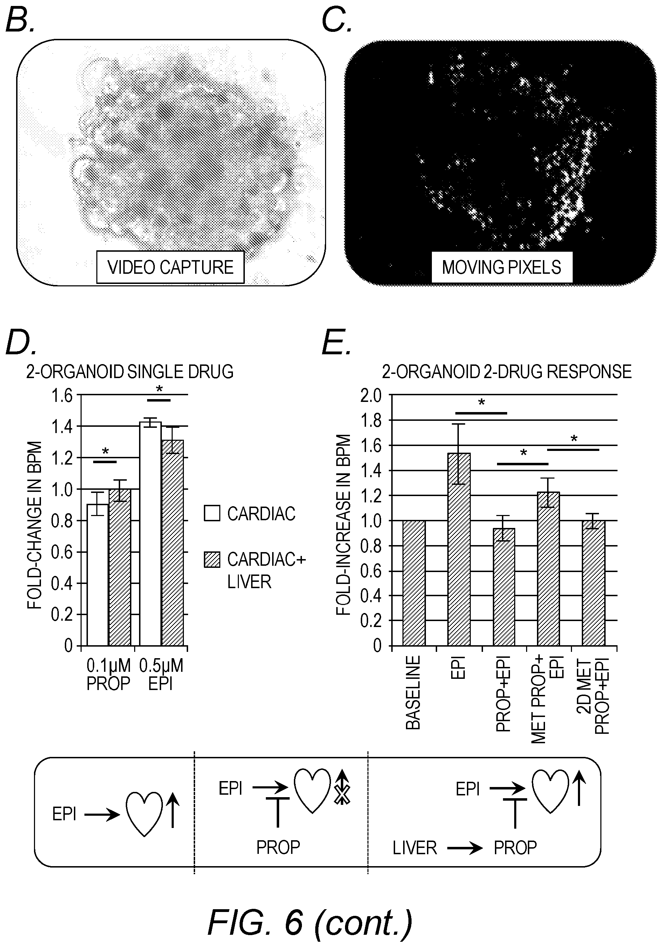

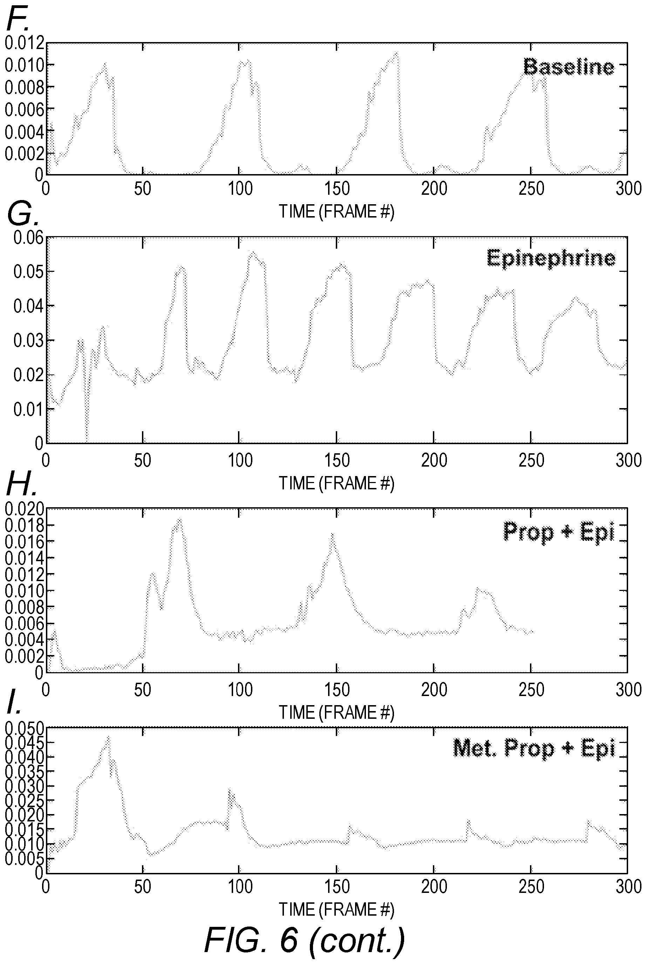

**Fig. 6****.** Multi-organoid interaction: Combining liver and cardiac modules results in a biological system capable of an integrated response to drugs. Panel a) A depiction of the on-chip camera system used to capture real-time beating data of cardiac organoids during culture within the ECHO platform. Panel b) Screen capture from a video of a beating cardiac organoid within the microfluidic system, and Panel c) screen capture of thresholded pixel movement conversion of the beating cardiac organoid (generated by custom written MatLab code) allowing quantification of beat rates. Panel d) Incorporation of liver organoids results in an altered response of the cardiac organoids to both 0.1 µM propranolol and 0.5 µM epinephrine. Panel e) The effects of liver metabolic activity on downstream cardiac beating rates. BPM values increase from baseline with epinephrine 0.5 µM; Increases from epinephrine are blocked by 0.1 µM propranolol. When liver organoids are present and permitted to metabolically inactivate 0.1 µM propranolol, 0.1 µM epinephrine is capable of inducing an increased BPM value. Interestingly, if 2D cultured hepatocytes are substituted for the liver organoids, this effect is not observed, indicating that in 2D culture, hepatocyte drug metabolism is greatly reduced. Statistical significance: * < 0.05. Panels f-i) Cardiac organoid beat peak plots corresponding to the values presented in panel e).

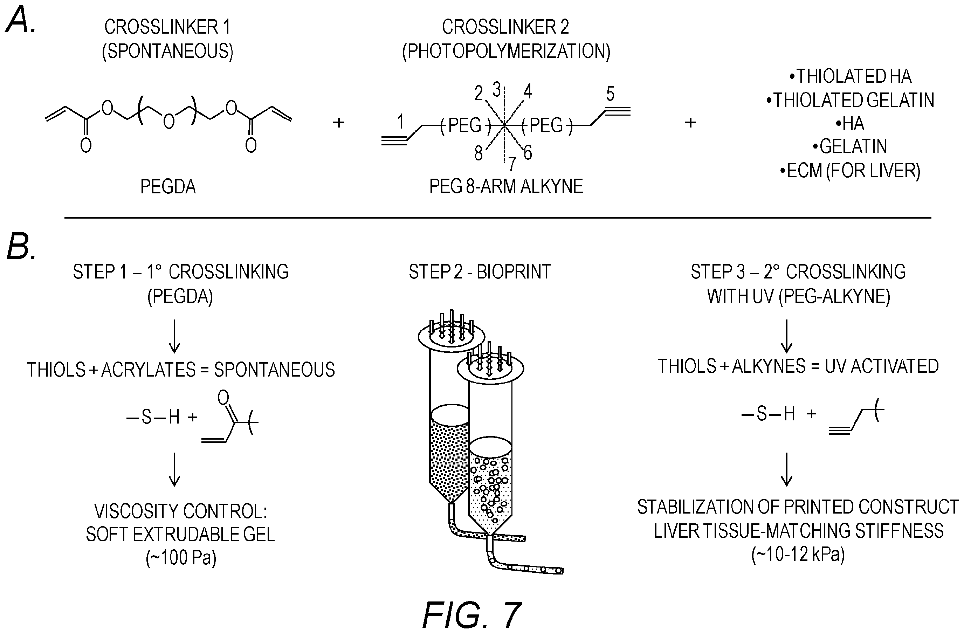

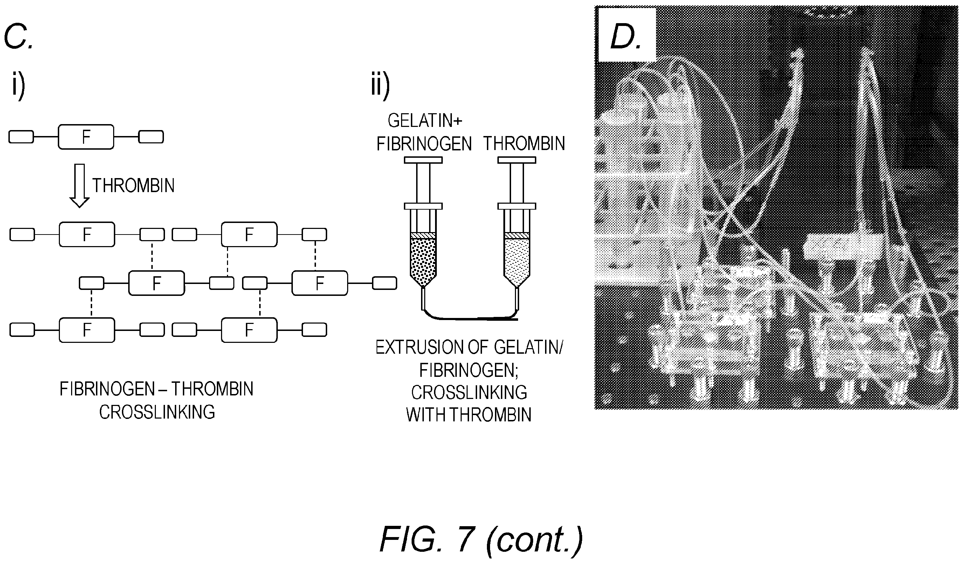

**Fig. 7****.** Bioprinting of hydrogel bioinks strategy, fluidic system overview, and additional phenotype assessment onboard fluidic devices. Panels a-b) Liver bioink formation and implementation. a) Strategy of formulation of printable bioinks comprised of acrylate-based crosslinkers (crosslinker 1), alkyne-based crosslinkers (crosslinker 2), thiolated HA, thiolated gelatin, and unmodified hyaluronic acid (HA) and gelatin, b) Implementation of bioprintable hydrogel bioinks. The bioink formulation is prepared and spontaneously crosslinks through thiol-acrylate binding, resulting in a soft, extrudable material. Bioprinting is performed. Lastly, the bioprinted structures are fused, stabilized, and brought to the target stiffness. Panel c) Cardiac bioink formation and implementation. Panel d) An operational three organoid-on-a-chip fluidic system. Three fluid circuits support three organoid devices, a parallel circuit microfluidic pump, media reservoirs, and bubble traps (not visible).

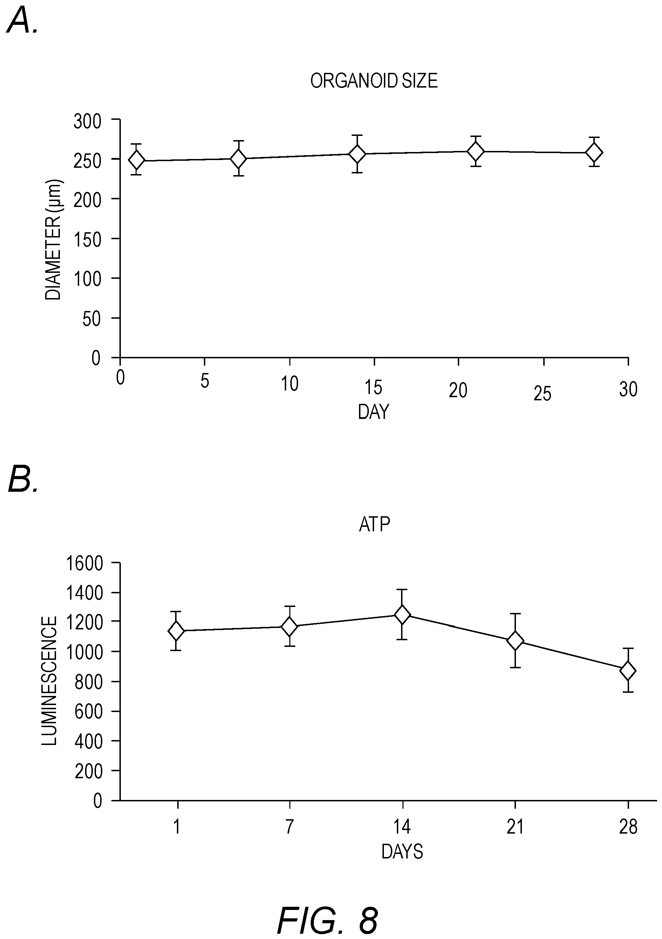

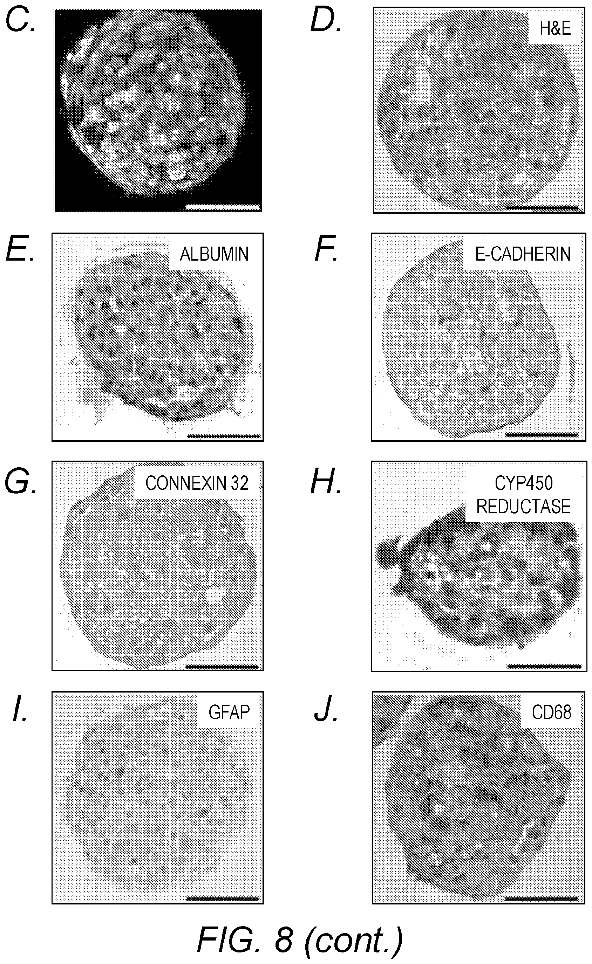

**Fig. 8****.** Liver organoids exhibit liver-specific markers and remain stable and viable long term. Panel a) Average organoid diameter remains consistent over 28 days. Panel b) Liver organoids remain metabolically active over 28 days, as determined by luminescence readings of ATPase. Panel c) LIVE/DEAD staining (shown at 14 days) shows high cell viability in the organoids. Green - calcein AM-stained viable cells; Red - ethidium homodimer-1-stained dead cells; Diameter 261*µ*m. Panels d-j) Histological and immunohistochemical staining depict organoid structure and organization, d) H&E staining shows overall organoid morphology. Primary human hepatocytes are identified by e) albumin expression, and exhibit epithelial organization shown through panel f) E-cadherin expression around the cell membrane, and also express panel g) connexin 32 and panel h) cytochrome P450 reductase. Hepatic Stellate and Kupffer cells are identified by panel i) GFAP, and panel j) CD68, respectively. Purple - hemotoxylin-stained nuclei; Pink - cell cytoplasm; Brown - indicated stain; Scale bar - 100 *µ*m.

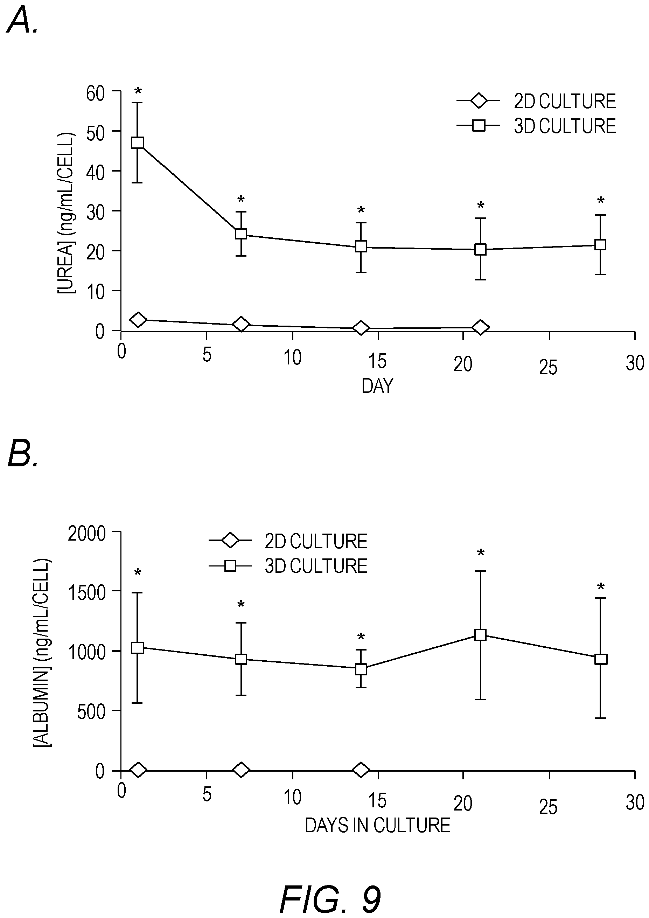

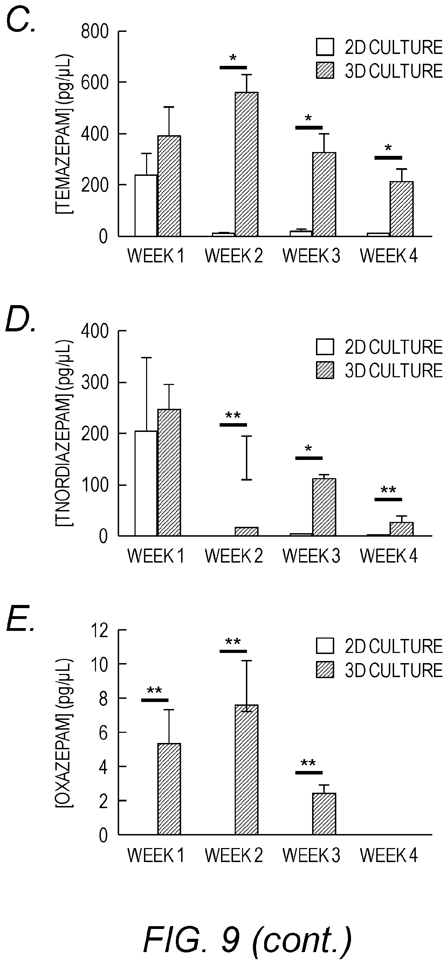

**Fig. 9****.** Liver organoids retain dramatically increased baseline liver function and metabolism compared to 2-D hepatocyte cultures. Normalized albumin (panel b) and (panel a) urea secretion into media, analyzed by ELISA and colorimetric assays show dramatically increased functional output in the 3-D organoid format in comparison to 2-D hepatocyte sandwich cultures. Quantification of the diazepam metabolites panel c) temazepam, panel d) noridazepam, and panel e) oxazepam primarily by CYP2C19 and CYP3A4. Statistical significance: * p < 0.05 between 3-D and 2-D comparisons at each time point.

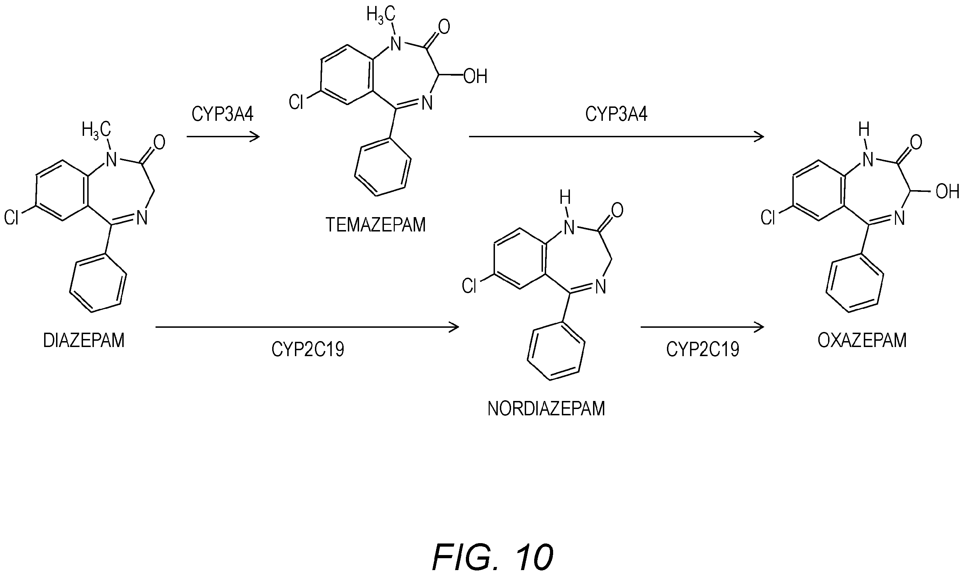

**Fig. 10****.** Liver organoid metabolism of diazepam into temazepam, nordiazepam, and oxazepan by cytochrome p450 isoforms.

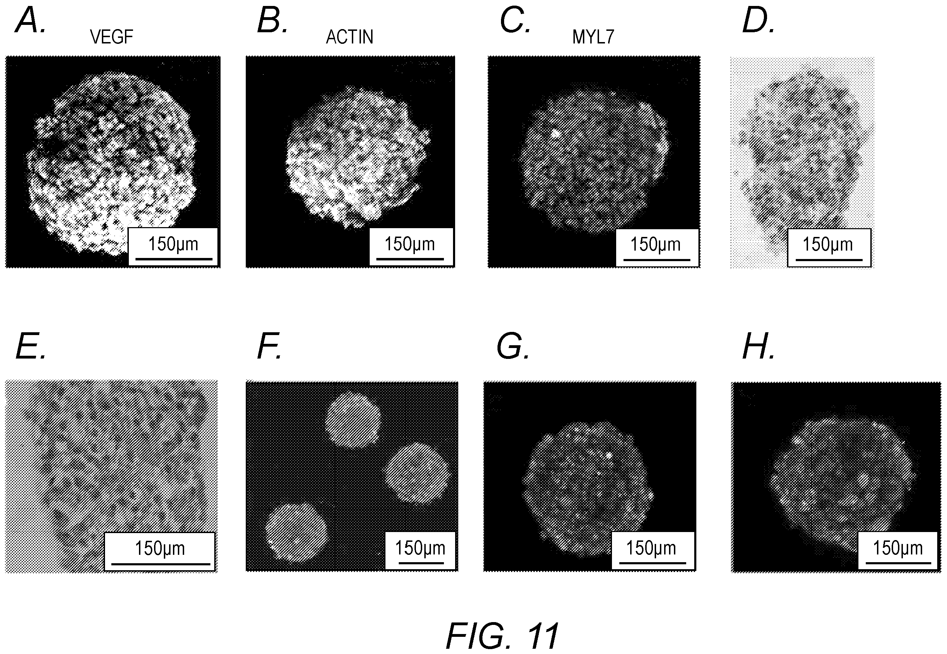

**Fig. 11****.** Cardiac organoids exhibit cardiac-specific markers and remain stable and viable over time. Cardiac organoids were stained for panel a) VEGF, panel b) actinin, panel c) low levels of myosin regulatory light chain 7 (MYI,7) (Red - indicated stain; Blue - DAPI), panel d) cardiac troponin-T (brown) with hemotoxylin counterstain, panel e) H&E, and panels f-h) Live/Dead viability/cytoxocity stains on panel f) day 1, panel g) day 28, and panel h) day 35 of culture. Green - calcein AM-stained viable cells; Red - ethidium homodimer-stained dead cell nuclei. Scale bars - 150 *µ*m.

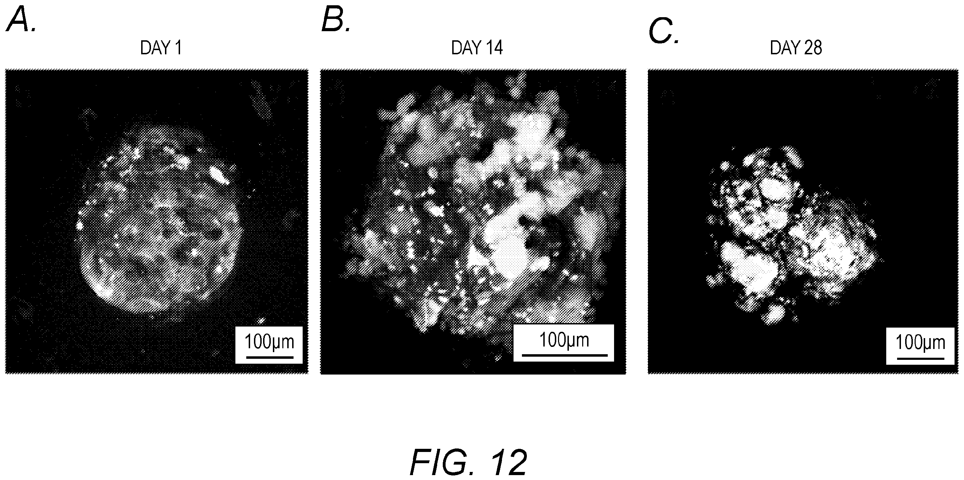

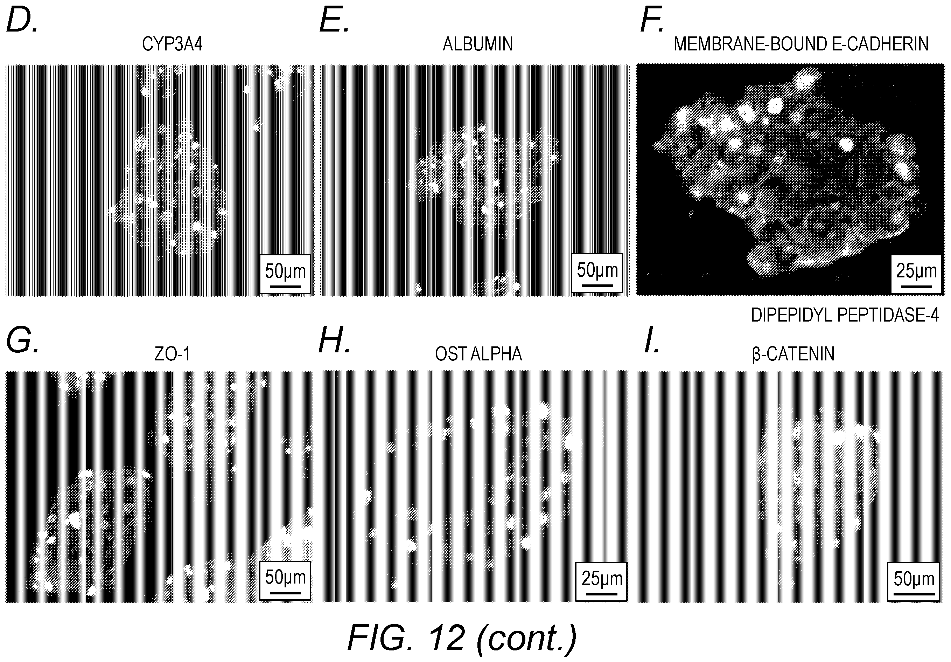

**Fig. 12****.** On-chip liver organoid viability and phenotype analysis by immunohistochemistry. Primary liver organoids cultured onboard the fluidic system remain viable panels a-c) for up to 28 days, and express panel d) CYP3A7 (green), panel e) albumin (green), panel f) E-cadherin (red) and DPP-4 (green), panel g) ZO-1 (green), panel h) Ost-α (green), and panel i) β-catenin (green). Blue staining indicates DAPI-stained nuclei.

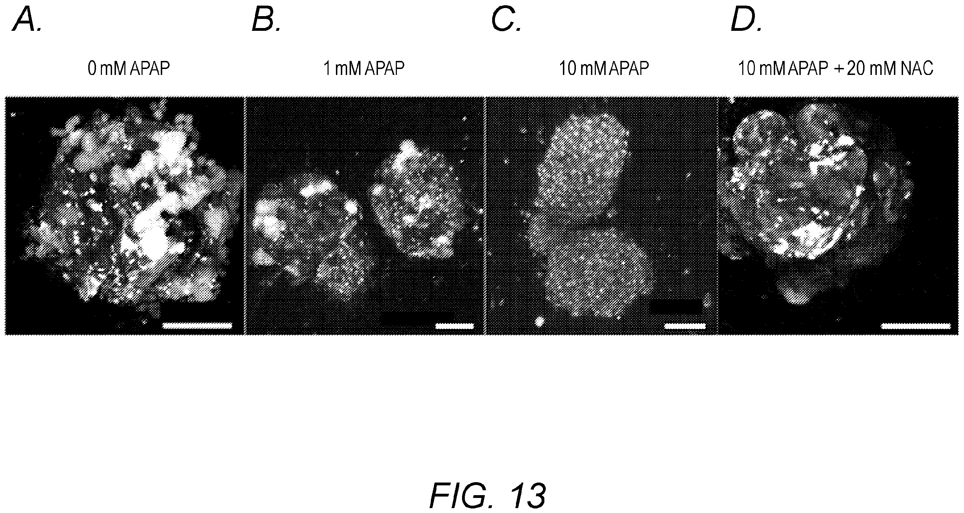

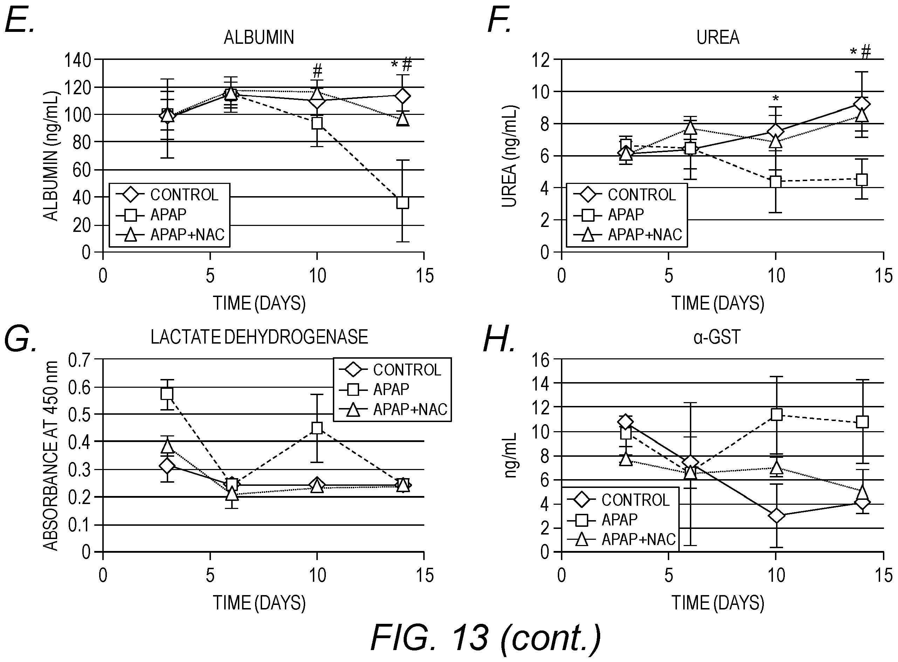

**Fig. 13****.** Liver on a chip: On-chip response to acetaminophen, and N-acetyl-L-cysteine countermeasure. Panels a-d) Liver organoids respond to acetaminophen toxicity and are rescued by NAC. Viability as determined by LIVE/DEAD staining on day 14. Organoids were exposed to panel a) a 0 mM APAP control, panel b) 1 mM APAP, panel c) 10 mM APAP, or panel d) 10 mM APAP with 20 mM N-acetyl-L-cysteine. Green - Calcein AM-stained viable cells; Red - Ethidium homodimer-stained dead cells. Panels e-h) Analysis of media aliquots indicate that APAP induces loss of function and cell death, while NAC has the capability to mitigate these negative effects. Quantification of panel e) human albumin, panel f) urea, panel g) lactate dehydrogenase, and panel h) alpha-GST. Albumin and urea output are negatively affected by APAP treatments, while NAC decreases this reduction in secretion. LDH and alpha-GST are low in control and APAP+NAC groups demonstrating functional cells, while APAP induces elevated levels, indicating apoptosis and release of LDH and alpha-GST into the media. Statistical significance: * p < 0.05 between control and APAP; # p < 0.05 APAP+NAC and APAP.

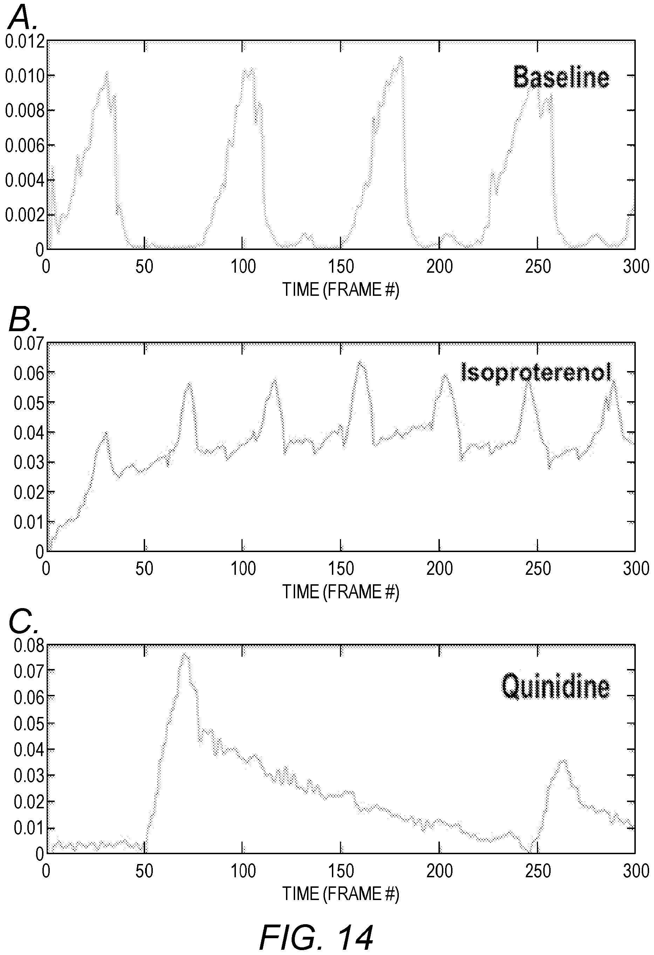

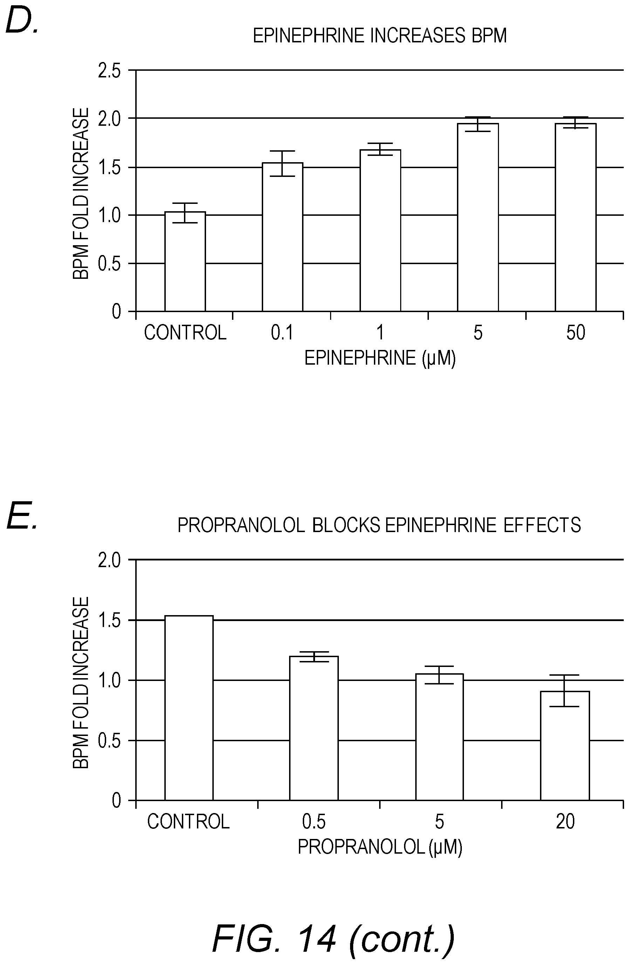

**Fig. 14****.** Cardiac organoids modulate beat rate as response to drug treatment. Panel a) Beating output under baseline conditions, from which beating rate is determined. Panels b-e) Change in cardiac organoid beat rate resulting from exposure to panel b) isoproterenol, or panel c) quinidine. Panels d-e) Cardiac organoid response to epinephrine and propranolol. Cardiac organoids produce a dose dependent increase in beat rate ranging from 1 to 2-fold with increasing epinephrine concentration before reaching a plateau with 5 µM epinephrine and higher. Initial incubation with propranolol concentrations ranging from 0 to 20 µM results in a dose dependent decrease in beating rate after administration of 5 µM epinephrine.

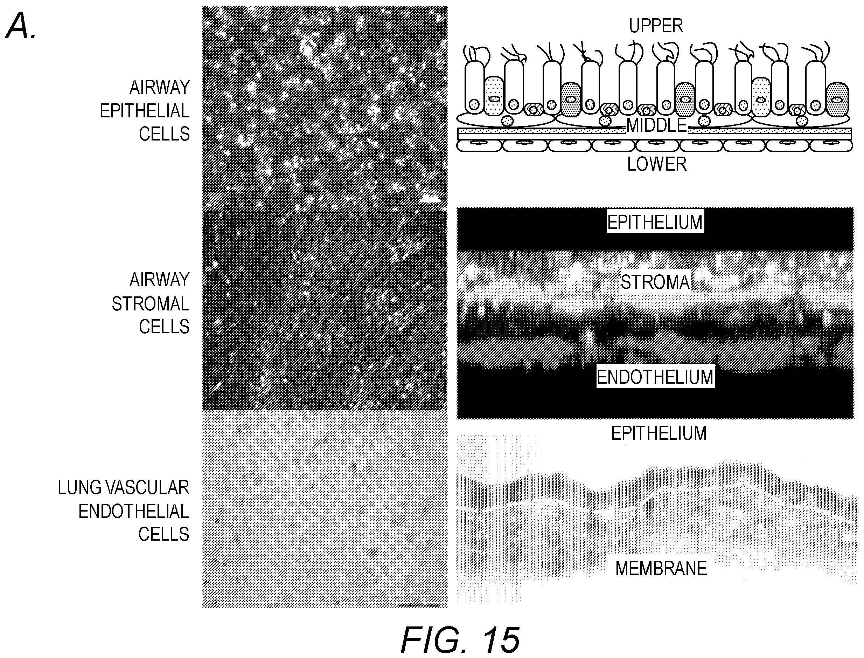

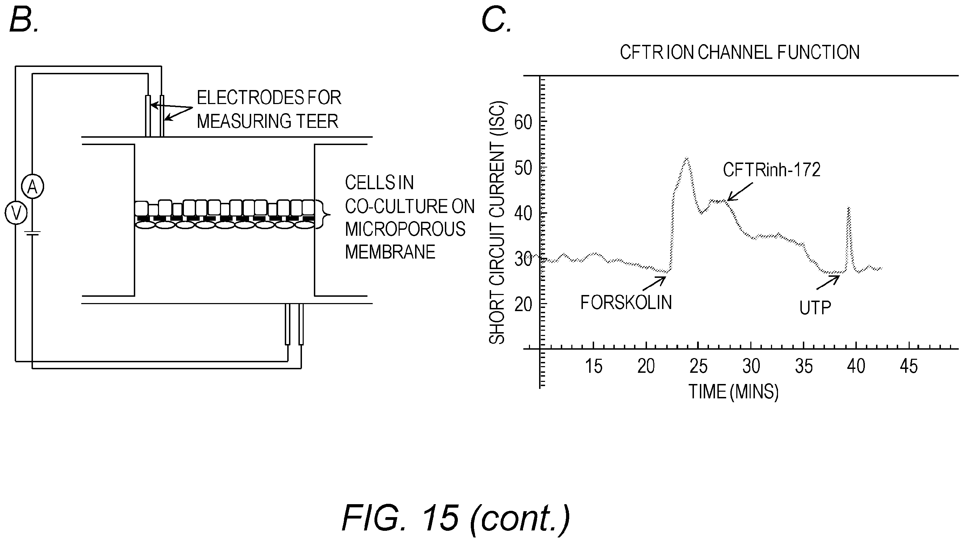

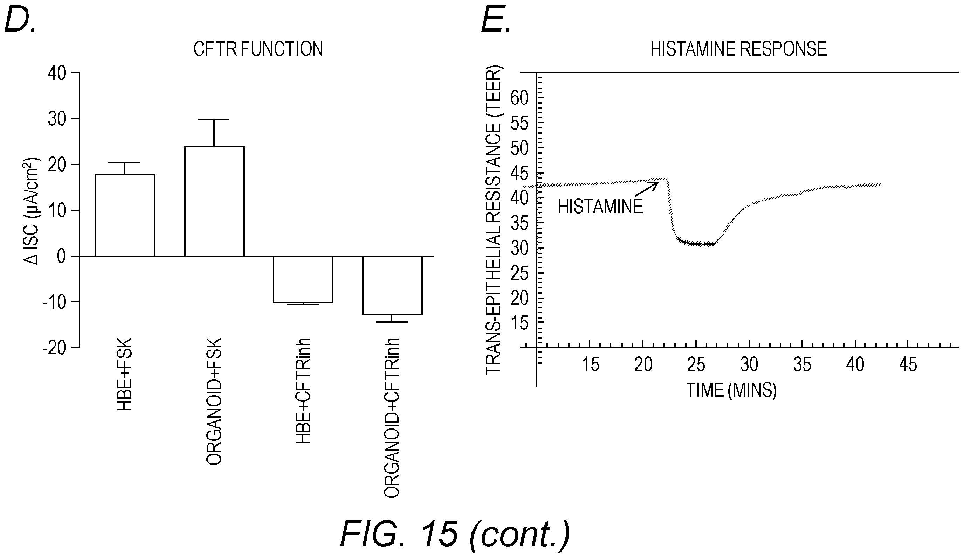

**Fig. 15****.** Generation of 3D lung organoids for disease modeling, drug development and testing. Panel A) 3D lung organoids are comprised of airway epithelial cells, airway stromal cells, and lung microvascular endothelial cells. The layered 3D organoid rapidly produces a cellular organization similar to that seen in native airway tissue, with a polarized epithelial surface exposed to the air-liquid interface, a stromal component providing tissue structure, and an endothelium forming a thin vascular barrier exposed to liquid media. Layered organoids can be maintained in culture for over 4 weeks with maintenance of transepithelial resistance and cell viability. The advantage of the layered technique of organoid formation is the ability to rapidly establish an organized tissue representing normal airway structure. Panel B) Trans-epithelial electrical resistance monitoring. Panels C-D) 3D lung organoids demonstrate physiological responses to CFTR activating or inhibitory pharmaceuticals and panel E) histamine as demonstrated by assessing CFTR ion channel activity by short circuit current (Isc) and TEER.

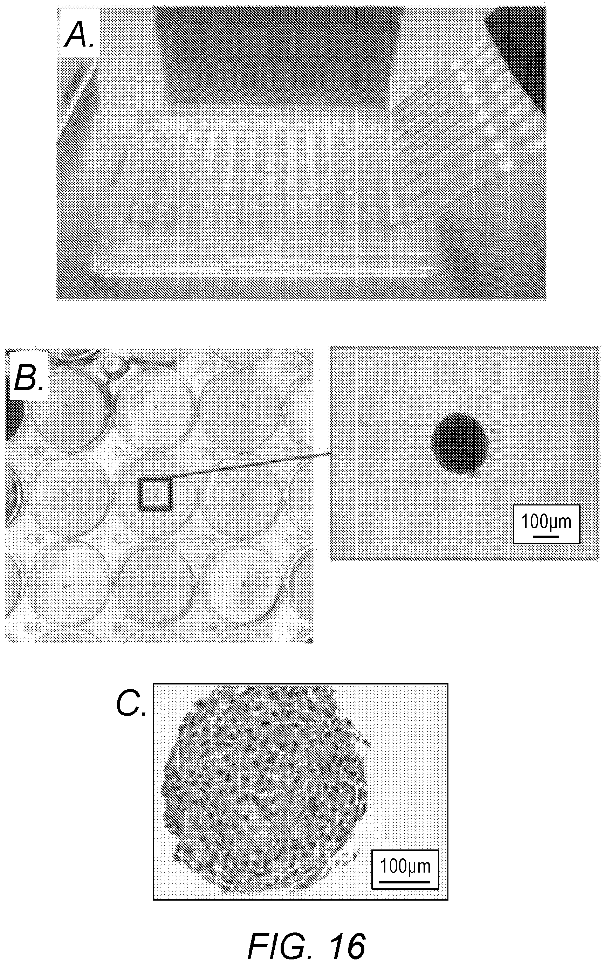

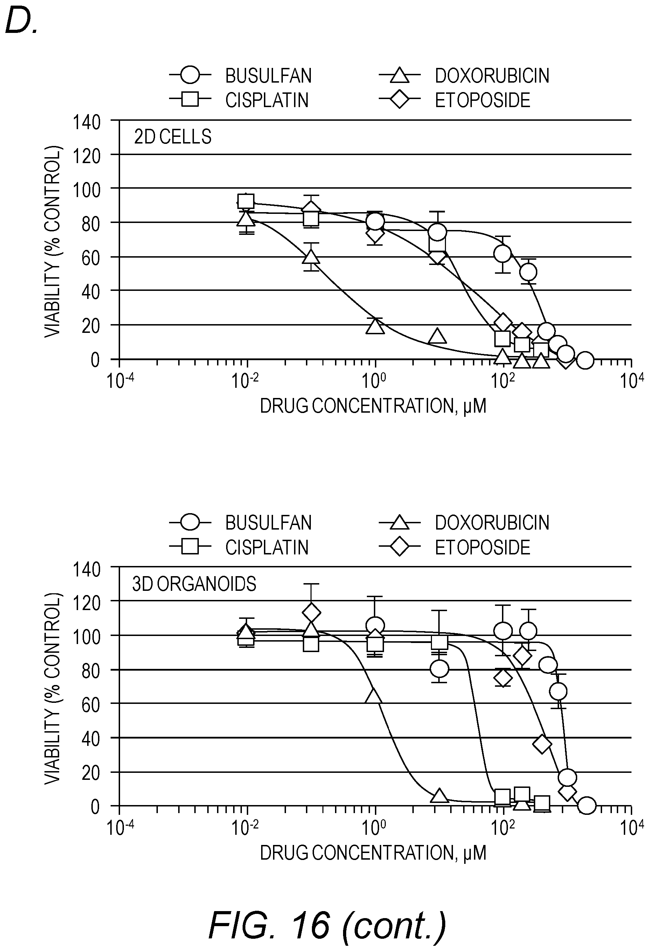

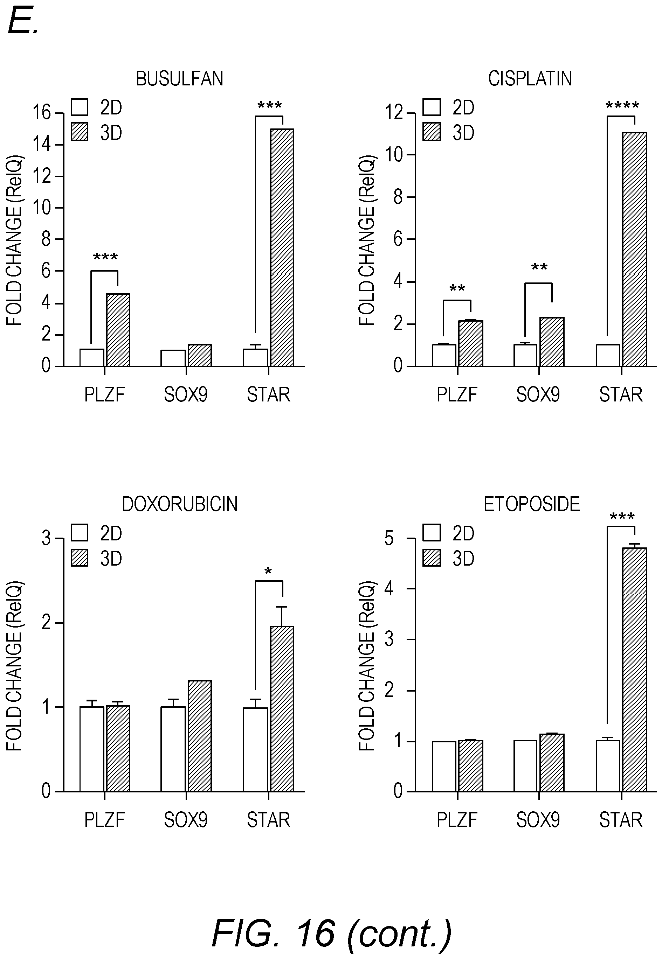

**Fig. 16****.** Generation of 3D testis organoids and drug toxicity testing. Panel (A) Perfecta3D^{®} 96-Well Hanging Drop Plate. These culture plates provide a versatile high-throughput system for 3D culture formation and subsequent evaluation of cellular constructs. Panel (B) Visualization of testicular organoids within wells of 96-well ultra-low adhesion culture plates. Insert picture represents phase contrast image of fully formed testicular organoid after 48 hours in hanging drop culture. Panel (C) Human testis organoid stained with hematoxylin and eosin for internal morphologic characterization. Panel (D) Drug effect on human testicular cells. Cells (2D culture in upper panel versus 3D organoids in lower panel) exposed to drugs for 48 hours prior to determination of ATP content via CellTiter-Glo^{®} Luminescent Cell Viability assays. Dose response curves were generated (n = 3). Data presented as mean ± SD. Panel (E) Testicular organoid gene expression changes following acute drug treatment. The fold change (RelQ, 2-ΔΔCT) for spermatogonia (PLZF (ZTBT16)), Leydig (CYP11A1, STAR) and Sertoli (SOX9) cell markers was measured to compare 3D cultured testicular organoids to corresponding 2D testicular cell culture controls for four clinically relevant chemotherapeutic drugs. A. Busulfan. B. Cisplatin. C. Doxorubicin. D. Etoposide (n = 3). Data presented as mean ± SD. Significance: *p < 0.05; **p < 0.01; ***p < 0.001.

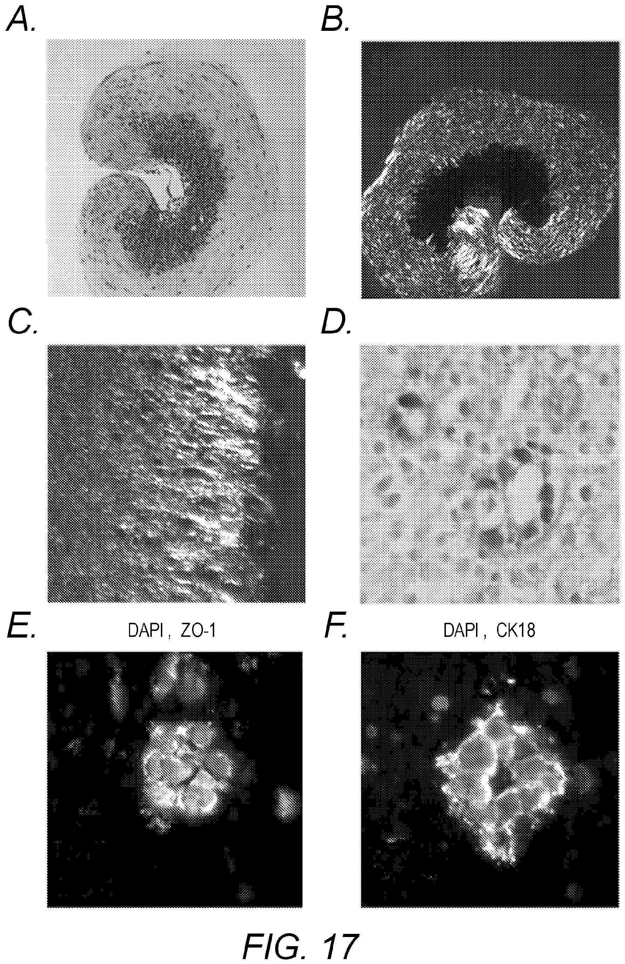

**Fig. 17****.** Colon construct morphology, collagen fiber alignment, and epithelial acini formation. Panel A) shows the cross-sectional morphology of colon constructs where smooth muscle cells aggregate to one side and cause inward contraction. Panel B) is picrosirius red staining of colon constructs where orange/red signal indicates highly bundled fibers and green signal indicates reticular fibers. Panel C) is a magnified image of collagen fiber bundles. Pictured in Panel D) is the formation of epithelial acini within the colon construct - developed acini do not form without smooth muscle remodeling (not shown). Panels E and F) show epithelial specific staining of acini demonstrating the cell-cell interaction between acini cells typically found in colon epithelium.

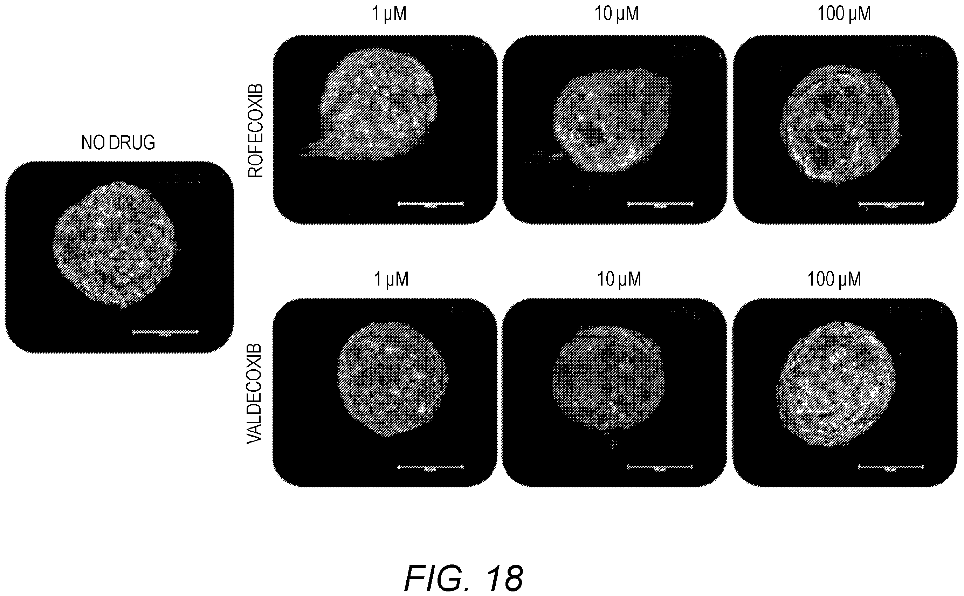

**Fig. 18****.** Viability assessment of cardiac organoids in response to rofecoxib and valdecoxib drug screens. Green - Calcein AM-stained viable cells; Red - Ethidium homodimer-stained dead cells.

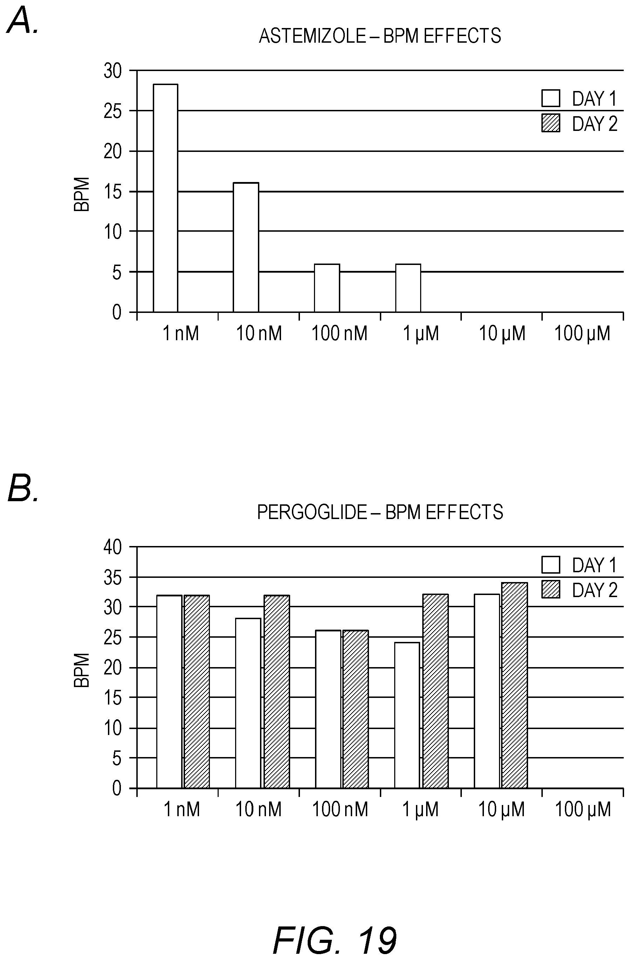

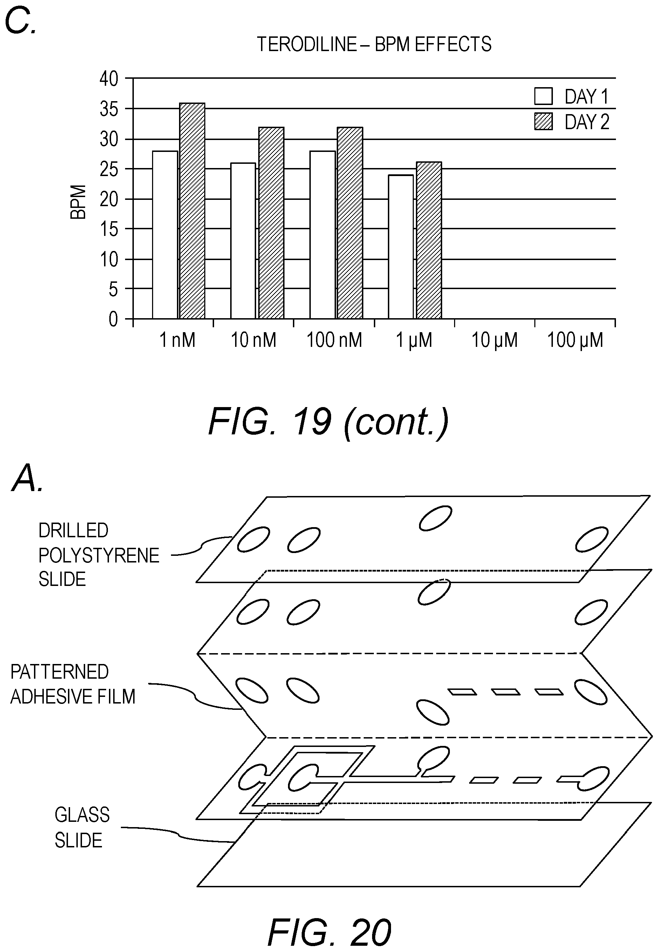

**Fig. 19****.** Assessment of cardiac organoid beating kinetics in response to panel a) astemizole, panel b) pergoglide, and panel c) terodiline.

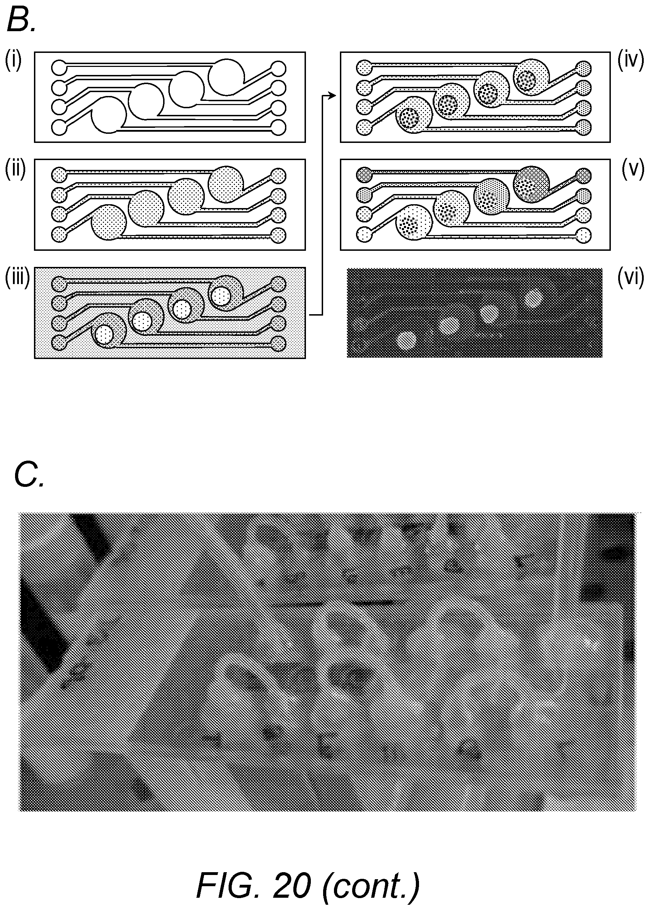

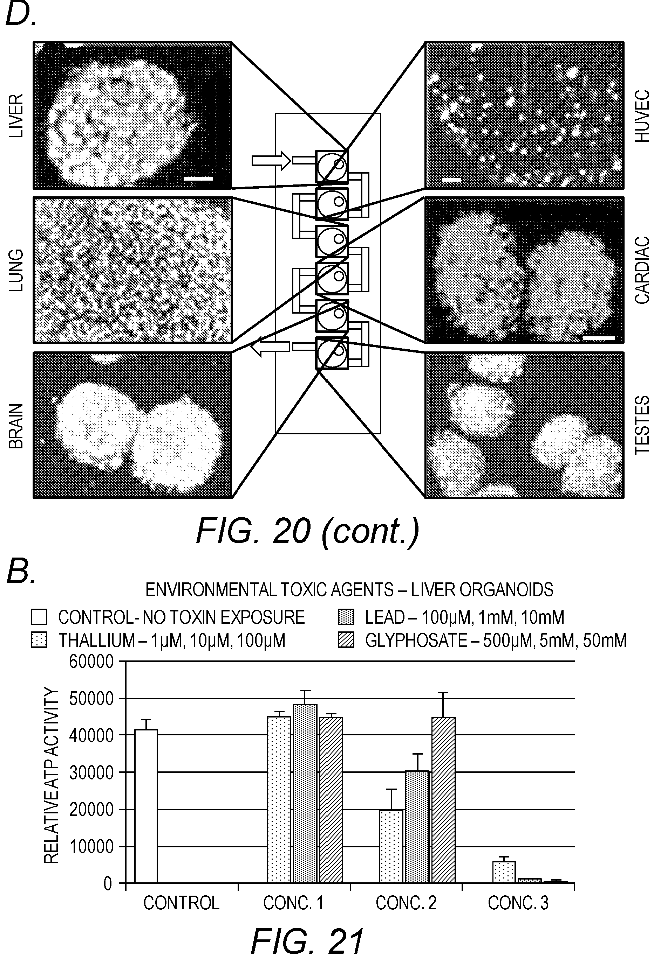

**Fig. 20****.** Microfluidic device fabrication and organoid formation. Panel A) Patterned adhesive films are layered between a glass slide (bottom) and a polystyrene slide drilled with fluid inlets and outlets (top) to form microfluidic channels quickly and easily. Panel B) *In situ* 3D cell culture microconstruct formation workflow. All fluidic channels (i) are filled with the mixture of photocurable hydrogel precursor, target cells, and additional components (light red, ii). A printed transparency photomask or patterned foil (grey) is employed to define construct shape (iii). Following UV exposure, cross-linked hydrogel (dashed lines) are formed in the channels (iv) and the remaining solution is replaced with clean buffer (v). Epifluorescence imaging (vi) demonstrates construct formation. Panel C) A miniaturized 6-tissue chip supporting liver, lung, cardiac, brain, vascular, and testes tissues. Panel D) High viability in the 6 tissues onboard. Green - viable cells; Red - dead cells.

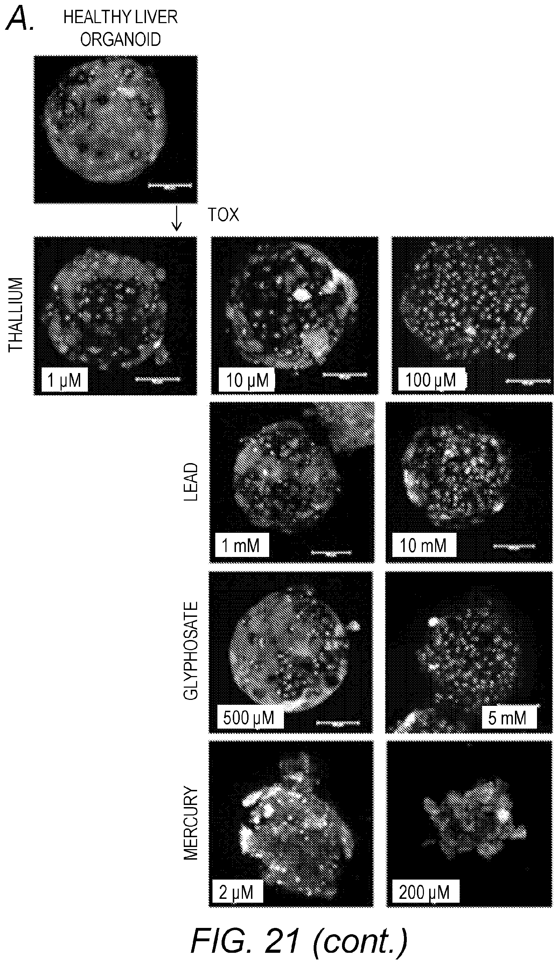

**Fig. 21****.** Preliminary results showing response of liver organoids to thallium, lead, glyphosate, and mercury. Panel A) LIVE/DEAD staining (green - viable cells; red - dead cells) and panel B) ATP activity.

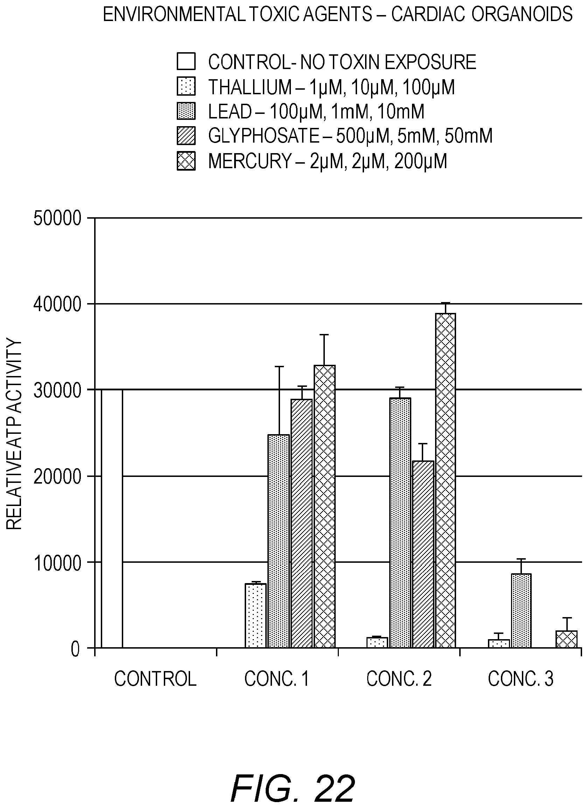

**Fig. 22****.** Preliminary results showing cardiac organoid decreases in ATP activity after exposure to thallium, lead, glyphosate, and mercury.

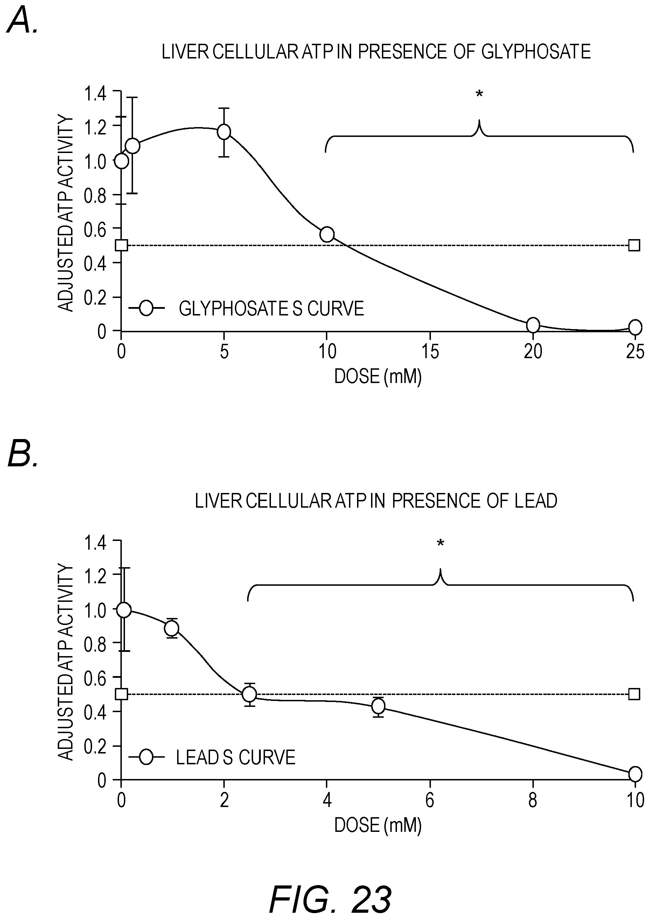

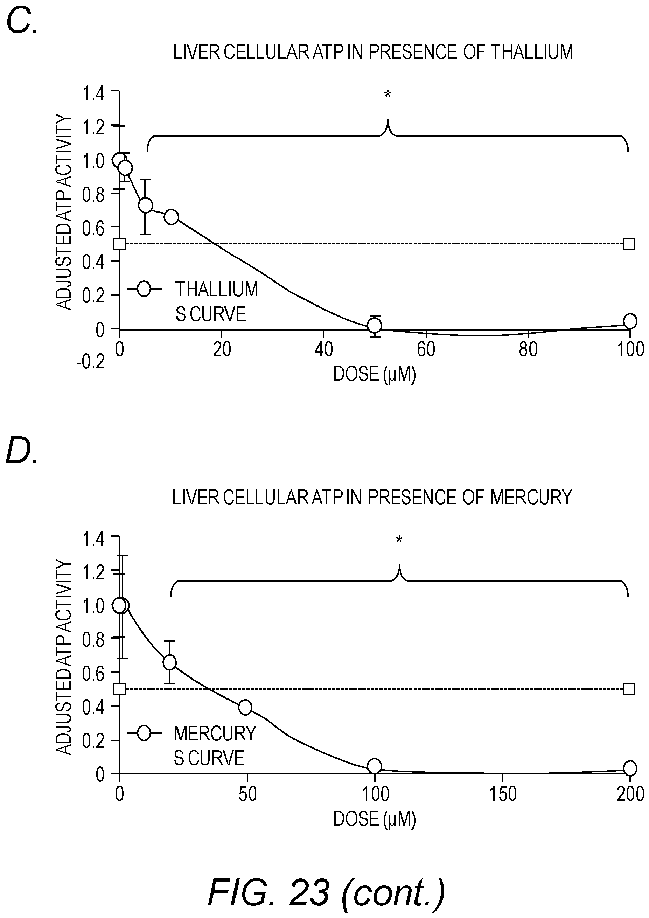

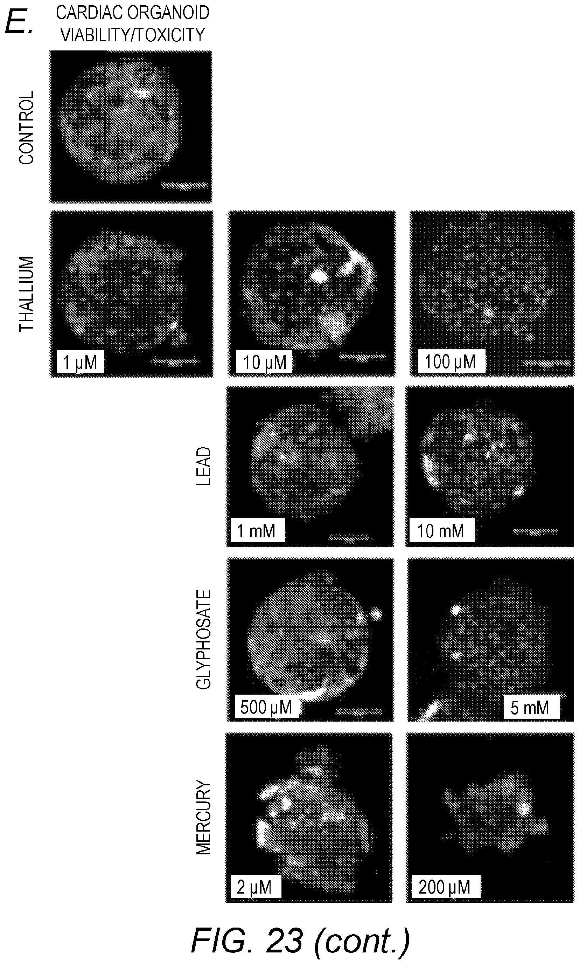

**Fig. 23****.** Preliminary liver organoid environmental toxin screens. Panels A-D) Normalized ATP activity and IC50 value estimation, and panel E) LIVE/DEAD staining and macro-confocal imaging of liver organoids treated with glyphosate, lead, thallium, and mercury. Green - viable cells; Red - dead cells. * p < 0.05 in comparison to the no drug condition for each toxin screen.

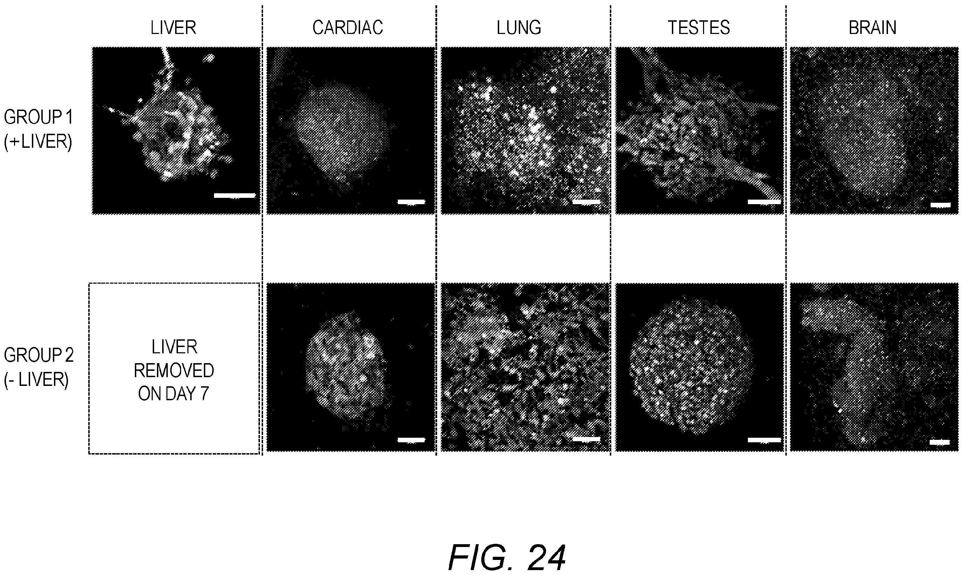

**Fig. 24** shows images of LIVE/DEAD results, which show that liver organoids are required to metabolize capecitabine to the active 5-FU form of the drug, inducing increased cell death in cardiac and lung organoids.

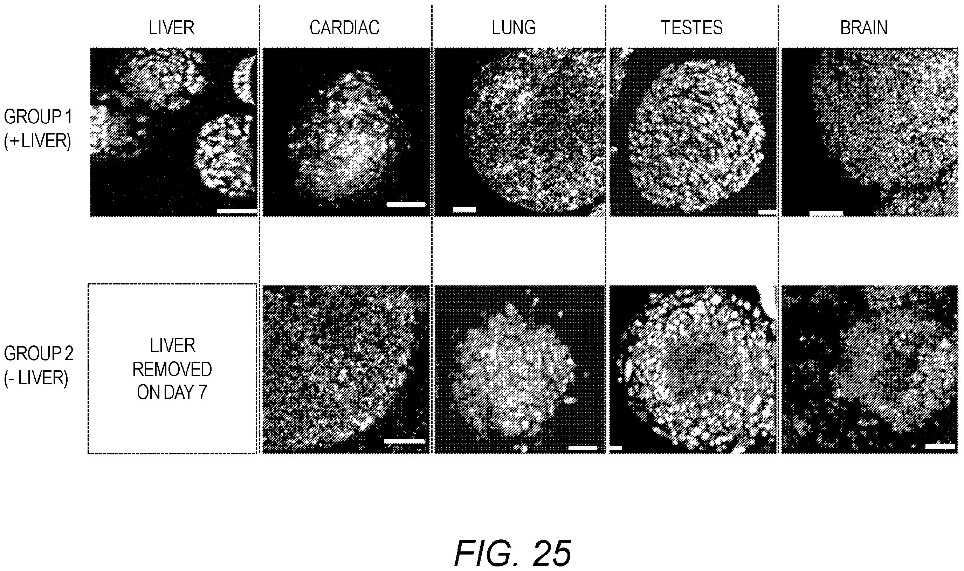

**Fig. 25** shows images of LIVE/DEAD results in the miniaturized system and show that, with liver present, capecitabine is metabolized into the toxic drug 5-FU, causing heart and lung toxicity. Without liver, this metabolism does not occur, and cardiac and lung organoid viability does not decrease.

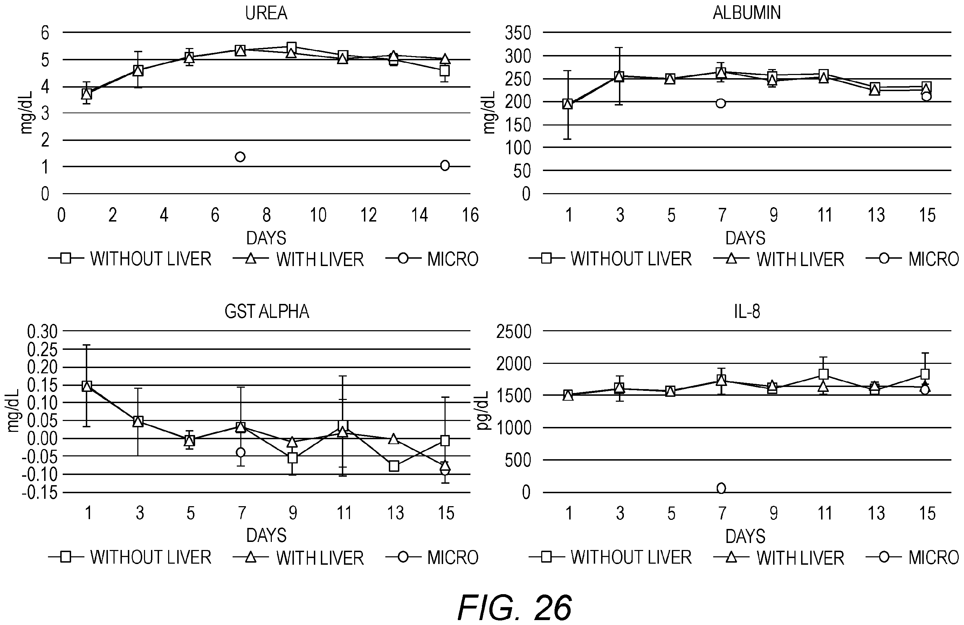

**Fig. 26** shows graphs of the initial biomarker analysis in the standard size system and the miniaturized system. Red and blue data points are from the standard size system, while the yellow "Micro" data points are from the miniaturized system.

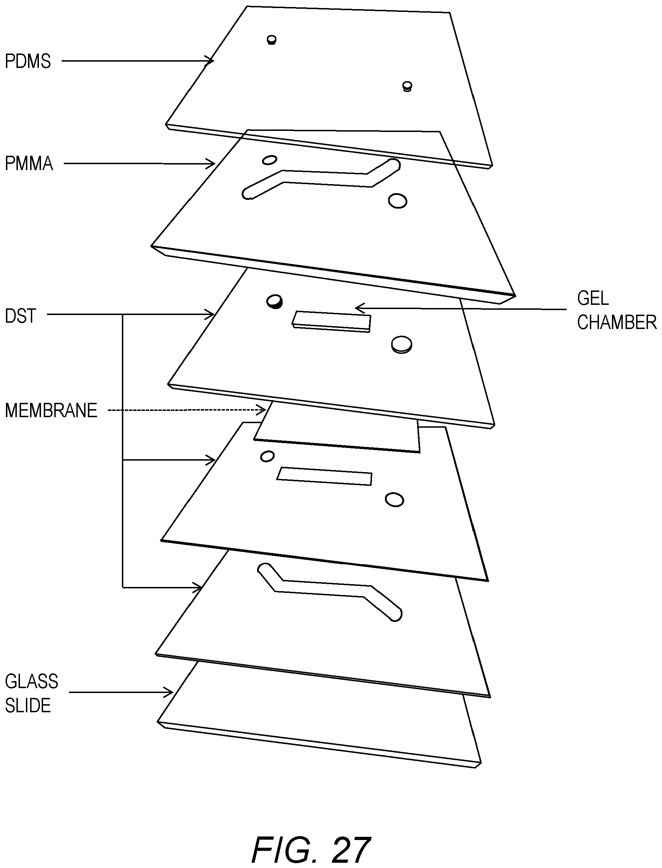

**Fig. 27** shows an illustration of the hybrid microreactor fabrication strategy incorporating tape fluidics and adhesive films and/or double sided tape (DST), poly(methyl methacrylate) (PMMA), and polydimethylsiloxane (PDMS) components.

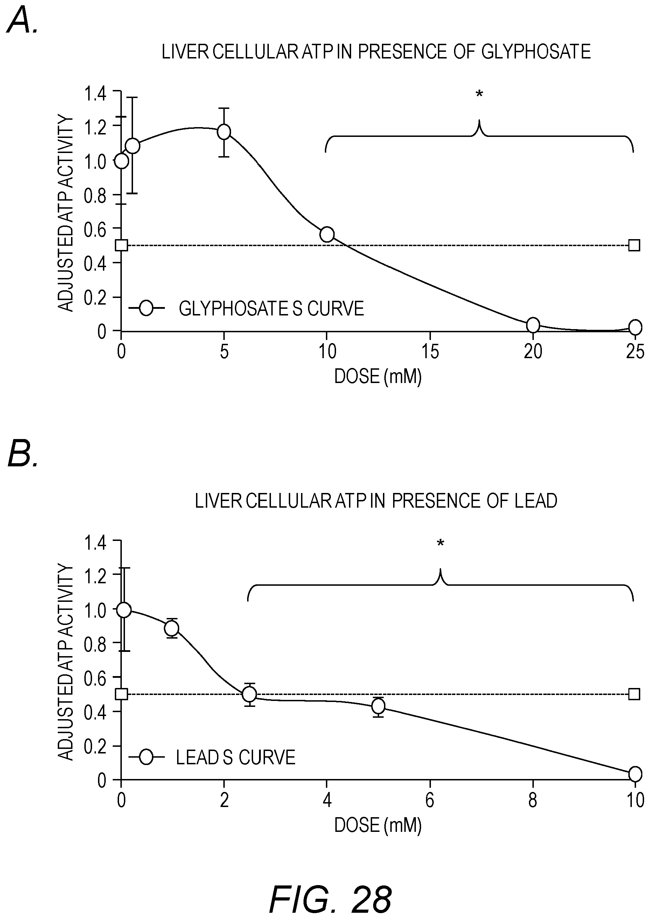

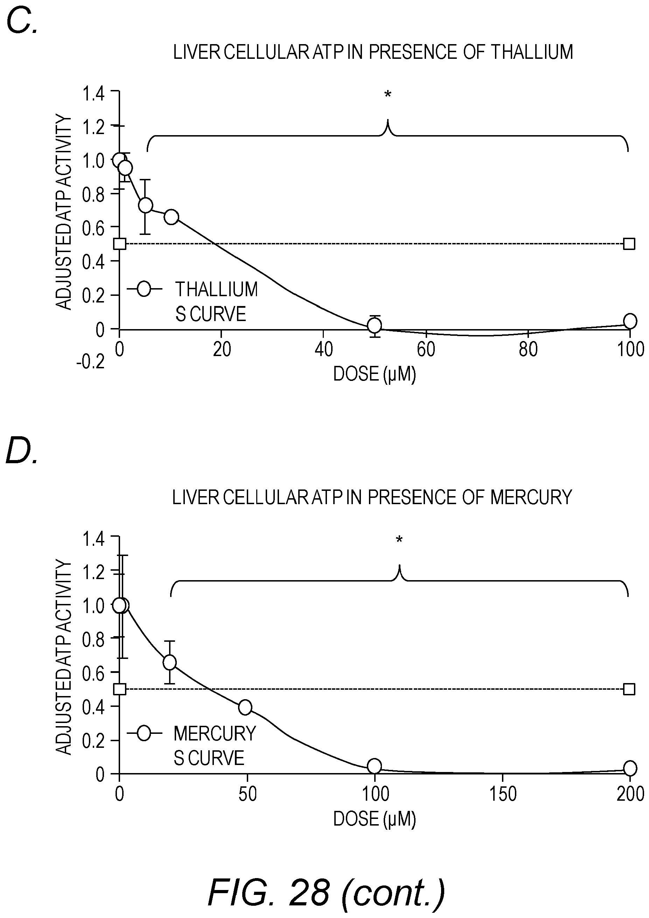

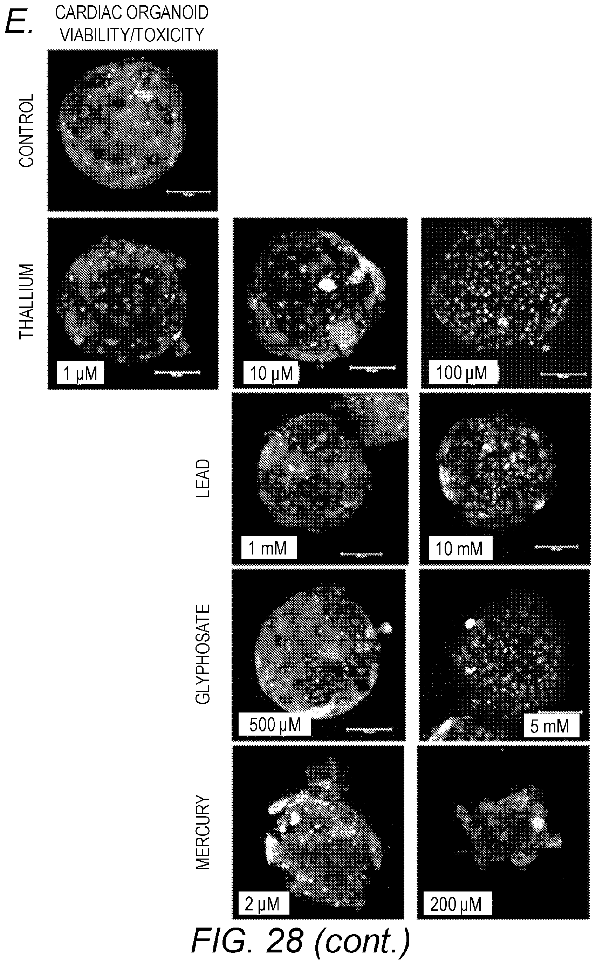

**Fig. 28** shows the results from the liver organoid environmental toxin screens with panels A-D showing the normalized ATP activity and IC50 value estimation, and panel E showing the LIVE/DEAD staining and macro-confocal imaging of liver organoids treated with glyphosate, lead, thallium, and mercury. * p < 0.05 in comparison to the no drug condition for each toxin screen.

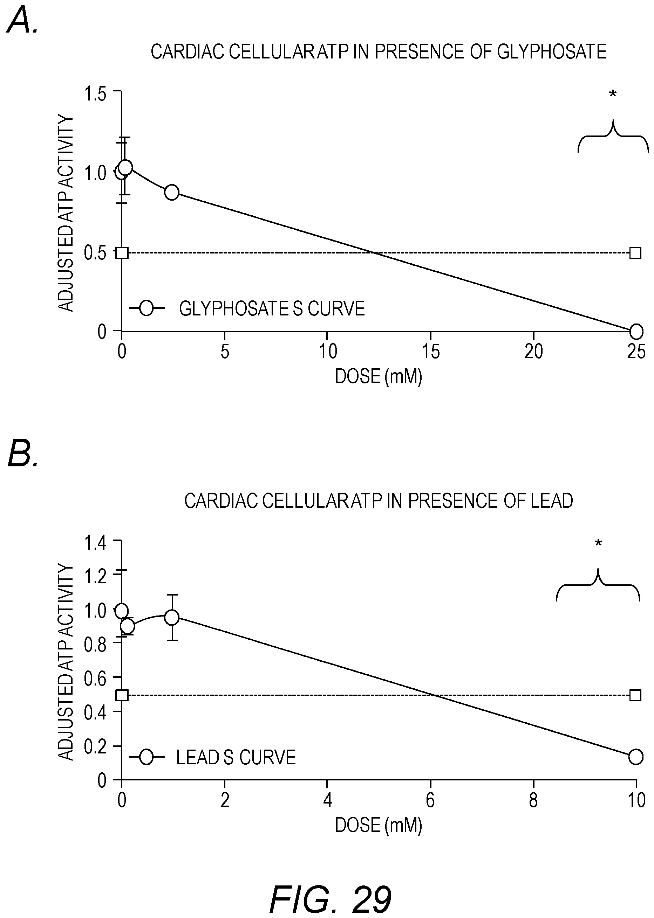

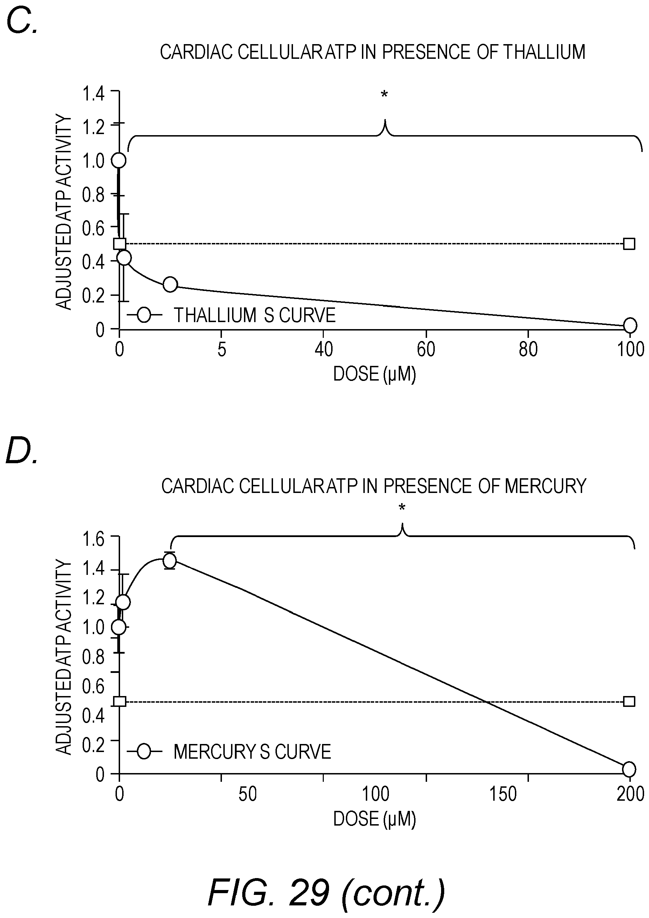

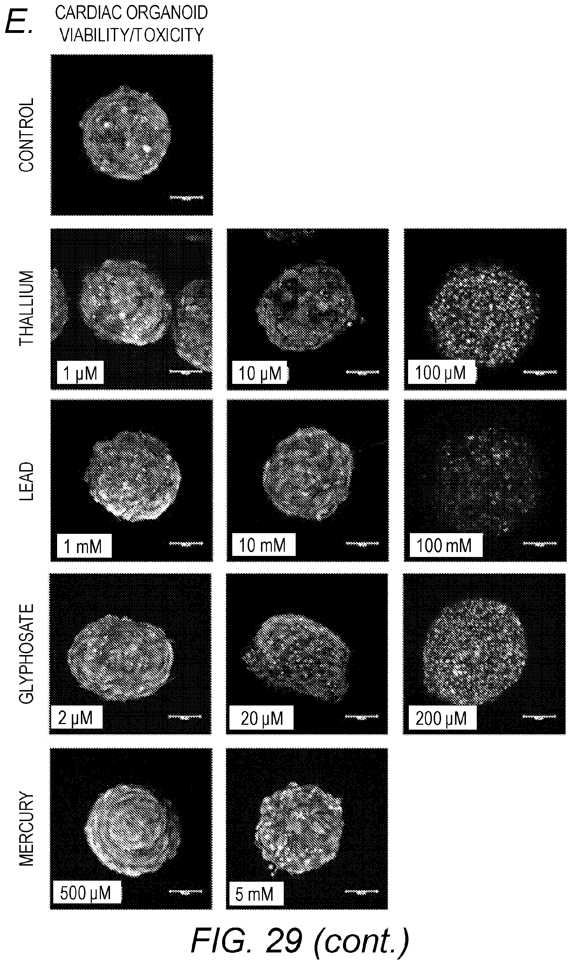

**Fig. 29** shows the results from the cardiac organoid environmental toxin screens with panels A-D showing the normalized ATP activity and IC50 value estimation, and panel E showing the LIVE/DEAD staining and macro-confocal imaging of liver organoids treated with glyphosate, lead, thallium, and mercury. * p < 0.05 in comparison to the no drug condition for each toxin screen.

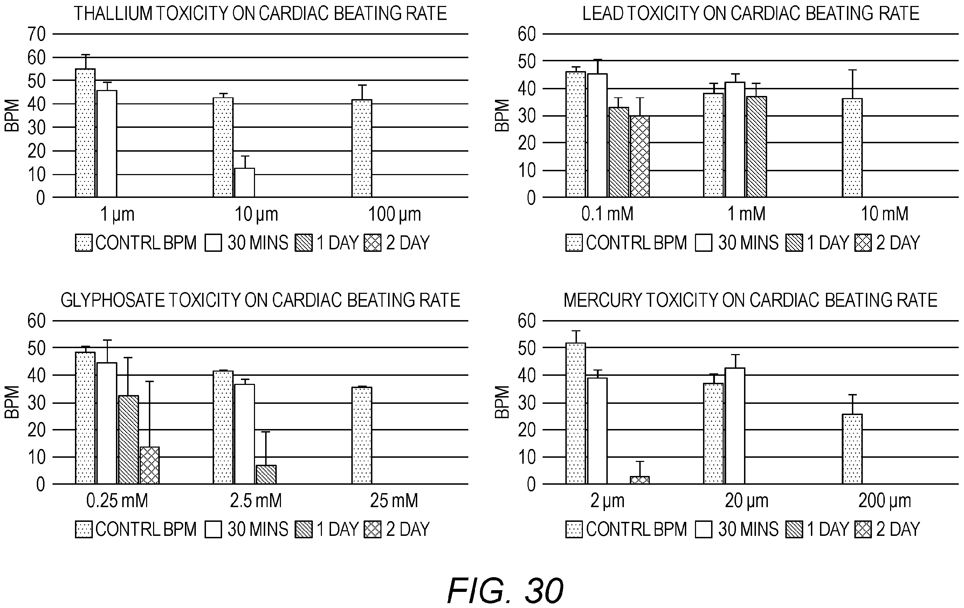

**Fig. 30** shows changes in cardiac organoid beat rates as an effect of environmental toxin exposure.

### Detailed Description of Illustrative Embodiments

The present disclosure is now described more fully hereinafter with reference to the accompanying drawings, in which embodiments of the disclosure are shown. This disclosure may, however, be embodied in many different forms and should not be construed as limited to the embodiments set forth herein; rather these embodiments are provided so that this disclosure will be thorough and complete and will fully convey the scope of the disclosure to those skilled in the art.

The terminology used herein is for the purpose of describing particular embodiments only and is not intended to be limiting of the invention/disclosure. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof. As used herein, the term "and/or" includes any and all combinations of one or more of the associated listed items.

It will be understood that when an element is referred to as being "on," "attached" to, "connected" to, "coupled" with, "contacting," etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

Spatially relative terms, such as "under," "below," "lower," "over," "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is inverted, elements described as "under" or "beneath" other elements or features would then be oriented "over" the other elements or features. Thus, the exemplary term "under" can encompass both an orientation of "over" and "under." The device may be otherwise oriented (rotated 90 degrees or at other orientations) and the spatially relative descriptors used herein interpreted accordingly. Similarly, the terms "upwardly," "downwardly," "vertical," "horizontal" and the like are used herein for the purpose of explanation only unless specifically indicated otherwise.

It will be understood that, although the terms "first," "second," etc. may be used herein to describe various elements, these elements should not be limited by these terms. These terms are only used to distinguish one element from another. Thus, a "first" element discussed below could also be termed a "second" element without departing from the teachings of the present invention. The sequence of operations (or steps) is not limited to the order presented in the claims or figures unless specifically indicated otherwise.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity. The terminology used in the description of the disclosure herein is for the purposes of describing particular embodiments only and is not intended to be limiting of the disclosure. In case of a conflict in terminology, the present specification is controlling.

"Cells" used in the present invention are, in general, animal cells, particularly mammalian and primate cells, examples of which include but are not limited to human, dog, cat, rabbit, monkey, chimpanzee, cow, pig, goat. The cells may be differentiated at least in part to a particular cell or tissue type, such as liver, intestine, pancreas, lymph node, smooth muscle, skeletal muscle, central nerve, peripheral nerve, skin, immune system, etc. Some cells may be cancer cells, as discussed further below, in which case they optionally may express (naturally, or by recombinant techniques) a detectable compound, as also discussed further below.

"Three dimensional tissue construct" and "organoid" are used interchangeably herein and, as used herein, refer to a composition of live cells, typically in a carrier media, arranged in a three-dimensional or multi-layered configuration (as opposed to a monolayer). Suitable carrier media include hydrogels, such as cross-linked hydrogels as described below. Additional example hydrogels include, but are not limited to, those described in PCT/US2015/055699. Such constructs may comprise one differentiated cell type, or two or more differentiated cell types, depending upon the particular tissue or organ being modeled or emulated. Some organoids may comprise cancer cells, as discussed further below. Where the constructs comprise cancer cells, they may include tissue cells, and/or may include a tissue mimic without cells, such as an extracellular matrix (or proteins or polymers derived therefrom), hyaluronic acid, gelatin, collagen, alginate, etc., including combinations thereof. Thus, in some embodiments, cells are mixed together with the extracellular matrix, or cross-linked matrix, to form the construct, while in other embodiments cell aggregates such as spheroids or organoids may be pre-formed and then combined with the extracellular matrix. In some embodiments, the tissue construct and/or organoid comprise cells that are human-derived cells, and, in some embodiments, the tissue construct and/or organoid comprise cells that consist of human-derived cells. A tissue construct and/or organoid of the present disclosure may express and/or produce one or more biomarkers (e.g., 1, 2, 3, 4, or more) that are the same as a biomarker produced by the cells *in vivo.* For example, liver cells *in vivo* produce albumin and an organoid of the present disclosure comprising liver cells may express albumin. In some embodiments, a tissue construct and/or organoid may express a biomarker in the same amount or in an amount that is ±20%, ±10%, or ±5% of the average amount produced and/or expressed by corresponding cells *in vivo.* Example biomarkers include, but are not limited to, albumin, urea, glutathione S-transferase (GST) (e.g., α-GST), chemokines (e.g., IL-8, IL-1β, etc.), prostacyclin, SB100B, neuron-specific enolase (NSE), myelin basic protein (MBP), hormones (e.g., testosterone, estradiol, progesterone, etc.), inhibin A/B, lactate dehydrogenase (LDH), and/or tumor necrosis factor (TNF).

In some embodiments, tissue construct and/or organoid is about 200 µm to about 350 µm in diameter, such as, for example, about 200, 250, 300, or 350 µm. The tissue construct and/or organoid may comprise about 1,500 or 2,000 to about 3,000 or 3,500 cells in total.

"Growth media" as used herein may be any natural or artificial growth media (typically an aqueous liquid) that sustains the cells used in carrying out the present disclosure. Examples include, but are not limited to, an essential media or minimal essential media (MEM), or variations thereof such as Eagle's minimal essential medium (EMEM) and Dulbecco's modified Eagle medium (DMEM), as well as blood, blood serum, blood plasma, lymph fluid, etc., including synthetic mimics thereof. In some embodiments, the growth media includes a pH color indicator (e.g., phenol red).

"Test compound" or "candidate compound" as used herein may be any compound for which a pharmacological or physiological activity, on cardiac tissue and/or other tissue, or an interaction between two test compounds, is to be determined. For demonstrative purposes, isoproterenol and quinidine are used separately below as test compounds to examine them independently, while propranolol and epinephrine are administered concurrently or in combination with one another as test compounds to examine the interaction there between. However, any compound may be used, typically organic compounds such as proteins, peptides, nucleic acids, and small organic compounds (aliphatic, aromatic, and mixed aliphatic/aromatic compounds) may be used. Candidate compounds may be generated by any suitable techniques, including randomly generated by combinatorial techniques, and/or rationally designed based on particular targets. Where a drug interaction is to be studied, two (or more) test compounds may be administered concurrently, and one (or both) may be known compounds, for which the possible combined effect is to be determined. In some embodiments, the test compound is a metal, such as, but not limited to, aluminum, lead, etc. In some embodiments, the test compound is a heavy metal, such as, but not limited to, arsenic, cadmium, chromium, lead, and/or mercury. In some embodiments, the test compound is a pesticide.

A composition of the present disclosure may be used to prepare a tissue construct of the present disclosure. Compositions of the present disclosure may comprise live cells in a "bioink," where the "bioink" is in turn comprised of a cross-linkable prepolymer, a post-deposition crosslinking group or agent; and other optional ingredients, including but not limited to growth factors, initiators (*e.g*., of cross-linking), water (to balance), etc. In some embodiments, the compositions are in the form of a hydrogel. Various components and properties of the compositions are discussed further below.

***Cells.*** As noted above, cells used to carry out the present disclosure may be animal cells *(e.g.,* bird, reptile, amphibian, etc.) and in some embodiments are mammalian cells *(e.g.,* dog, cat, mouse, rat, monkey, ape, human). The cells may be differentiated or undifferentiated cells, but are in some embodiments tissue cells (e.g., liver cells such as hepatocytes, pancreatic cells, cardiac muscle cells, skeletal muscle cells, etc.).

Choice of cells will depend upon the particular organoid being created. For example, for a liver organoid, liver hepatocyte cells may be used. For a peripheral or central nerve organoid, peripheral nerve cells, central nerve cells, glia cells, or combinations thereof may be used. For a bone organoid, bone osteoblast cells, bone osteoclast cells, or combinations thereof may be used. For a lung organoid, lung airway epithelial cells may be used. For a lymph node organoid, follicular dendritic lymph cells, fibroblastic reticular lymph cells, leukocytes, B cells, T cells, or combinations thereof may be used. For a smooth or skeletal muscle organoid, smooth muscle cells, skeletal muscle cells, or combinations thereof may be used. For a skin organoid, skin keratinocytes, skin melanocytes, or combinations thereof may be used. The cells may be differentiated upon initial incorporation into the composition, or undifferentiated cells that are subsequently differentiated may be used. Additional cells may be added to any of the compositions described above, and cancer cells as described below may be added to primary or "first" organoids, as described below.

Cancer cells optionally used in the present disclosure may be any type of cancer cell, including but not limited to melanoma, carcinoma, sarcoma, blastoma, glioma, and astrocytoma cells, etc.

In some embodiments, cells may be obtained from a subject, such as, for example, a subject or patient undergoing treatment for cancer and/or that has cancer and/or a subject that has a compromised immune system. In some embodiments, cells are tumor cells, such as, e.g., patient biopsy-derived tumor cells, and organoids prepared from such cells may be used to screen potentially effective drugs and/or treatments. The cells may be differentiated at least in part to a particular cell or tissue type, such as brain, liver, intestine, pancreas, lymph node, smooth muscle, skeletal muscle, central nerve, peripheral nerve, skin, immune system, etc.

In some embodiments, an organoid of the present disclosure is not prepared from and/or does not comprise cells from an immortalized cell line. Organoids of the present disclosure may comprise and/or be prepared using high functioning cells, such as, but not limited to, primary cells and/or stem cells, e.g., induced pluripotent stems and/or differentiated iPS-derived cells.

The cells may be incorporated into the composition in any suitable form, including as unencapsulated cells, or as cells previously encapsulated in spheroids, or pre-formed organoids (as noted above). Animal tissue cells encapsulated or contained in polymer spheroids can be produced in accordance with known techniques, or in some cases are commercially available (*see, e.g.,* Insphero AG, 3D Hepg2 Liver Microtissue Spheroids (2012); Inspherio AG, 3D InSight™ Human Liver Microtissues, (2012)).

An organoid of the present disclosure may be in any suitable shape and/or form such as, e.g., any three-dimensional shape or multi-layered shape. In some embodiments, an organoid of the present disclosure may be in the form of a spheroid. An organoid of the present disclosure may be self-organized in a suspension or medium and optionally may be in the form of a spheroid. In some embodiments, the cells forming and/or present in the organoid may be suspended in a composition and/or bioink of the present disclosure. In some embodiments, an organoid of the present disclosure such as, e.g., a lung and/or gut (e.g., colon) organoid, may comprise endothelial cells on one side of a semi-porous membrane and epithelial cells on the opposite side of the semi-porous membrane. Thus, the organoid may comprise a layer of endothelial cells and a layer of epithelial cells that are separated by a semi-porous membrane. The endothelial cells and epithelial cells may each be cultured on a surface (i.e., opposing surfaces) of the semi-porous membrane. The semi-porous membrane may be a silicon and/or polycarbonate-based membrane.

***Cross-linkable prepolymers.*** Any suitable prepolymer can be used to carry out the present disclosure, so long as it can be further cross-linked to increase the elastic modulus thereof after deposition when employed in the methods described herein.

In some embodiments, the prepolymer is formed from the at least partial crosslinking reaction of *(i)* an oligosaccharide (*e.g*., hyaluronic acid, collagen, combinations thereof and particularly thiol-substituted derivatives thereof) and *(ii)* a first crosslinking agent *(e.g.,* a thiol-reactive crosslinking agent, such as polyalkylene glycol diacrylate, polyalkylene glycol methacrylate, etc., and particularly polyethylene glycol diacrylate, etc.; thiolated crosslinking agent to create thiol-thiol disulfide bonds; gold nanoparticles gold functionalized crosslinkers forming thiol-gold bonds; etc., including combinations thereof).

***Cross-linking group.*** In some embodiments, the compositions include a post-deposition crosslinking group. Any suitable crosslinking groups can be used, including but not limited to multi-arm thiol-reactive crosslinking agent, such as polyethylene glycol dialkyne, other alkyne-functionalized groups, acrylate or methacrylate groups, etc., including combinations thereof.

***Initiators.*** Compositions of the disclosure may optionally, but in some embodiments preferably, include an initiator (*e.g*., a thermal or photoinitiator). Any suitable initiator that catalyzes the reaction between said prepolymer and the second (or post-deposition) crosslinking group (*e.g*., upon heating or upon exposure to light), may be employed.

***Growth factors.*** Compositions of the disclosure may optionally, but in some embodiments preferably, include at least one growth factor (*e.g*., appropriate for the particular cells included, and/or for the particular tissue substitute being produced). In some embodiments, growth factors and/or other growth promoting proteins may be provided in a decellularized extracellular matrix composition ("ECM") from a tissue corresponding to the tissue cells (*e.g*., decellularized extracellular liver matrix when the live animal cells are liver cells; decellularized extracellular cardiac muscle matrix when the live animal cells are cardiac muscle cells; decellularized skeletal muscle matrix when the live animal cells are skeletal muscle cells; etc.). Additional collagens, glycosaminoglycans, and/or elastin (*e.g*., which may be added to supplement the extracellular matrix composition), etc., may also be included.

***Elastic modulus.*** A composition of the present disclosure may have an elastic modulus, at room temperature and atmospheric pressure, sufficiently low such that it can be manipulated and deposited on a substrate by whatever deposition method is employed (*e.g*., extrusion deposition). Further, the composition optionally, but in some embodiments preferably, has an elastic modulus, again at room temperature and atmospheric pressure, sufficiently high so that it will substantially retain the shape or configuration in which it is deposited until subsequent cross-linking (whether that cross-linking be spontaneous, thermal or photo-initiated, etc.). In some embodiments, the composition, prior to deposition, has a stiffness of from 0.05, 0.1 or 0.5 to 1, 5 or 10 kiloPascals, or more, at room temperature and atmospheric pressure.

A method of making a cardiac construct, may comprise: depositing a mixture comprising live mammalian cardiac cells (*e.g*., individual cells, organoids, or spheroids), fibrinogen, gelatin, and water on a support to form an intermediate cardiac construct; optionally co-depositing a structural support material (*e.g*., polycaprolactone) with the mixture in a configuration that supports the intermediate construct; and then contacting thrombin to the construct in an amount effective to cross-link the fibrinogen and produce (with intervening incubation as necessary, depending on the maturity of the cardiac cells to begin with) a cardiac construct comprised of live cardiac cells that together spontaneously beat in a fibrin hydrogel.

In some embodiments, the cardiac cells are in the form of organoids produced by hanging-drop culture of cardiomyocytes. *See, e.g.,* US 2011/0287470 to Stoppini.

In some embodiments, the cardiac construct (specifically, the cardiac cells therein) exhibits spontaneous beating that is increased in frequency by the administration of isoproterenol in an effective amount and decreased in frequency by the administration of quinidine in an effective amount.

In some embodiments, the cardiac construct (specifically, the cardiac cells therein) express VEGF, actinin, and/or cardiac troponin-T.

As with the general bioink described above, unmodified gelatin can be added to the fibrinogen in order to thicken it into an extrudable material that can be bioprinted using bioprinting devices. As this gelatin is not crosslinked, upon incubation at physiological temperature (37 degrees C) after bioprinting a cardiac construct, the gelatin eventually dissolves and leaches out of the construct, leaving behind only the crosslinked fibrin and the beating cardiac construct.

Disclosed are methods of making a tissue construct in an apparatus of the present disclosure. In one non-limiting method, a composition of the present disclosure is used in a method of making a particular construct in an apparatus of the present disclosure. In some embodiments, a method of the present disclosure comprises the steps of:
*(a)* providing a reservoir containing an extrudable hydrogel composition as described above, then
*(b)* depositing the hydrogel composition onto a substrate (*e.g*., by extrusion through a syringe); and then
*(c)* optionally (as the secondary constructs may be produced by any suitable means) for general compositions and their tissue constructs, cross-linking the prepolymer with a second crosslinking group by an amount sufficient to increase the stiffness of said hydrogel and form said three-dimensional organ construct *(e.g*., by heating the hydrogel, irradiating the hydrogel composition with light (*e.g*., ambient light, UV light), altering the pH of the hydrogel; etc.); and
*(d)* for cardiac construct compositions, contacting the hydrogel with thrombin to cross-link the fibrinogen and form a fibrin hydrogel, as noted above.

The depositing step may be carried out with any suitable apparatus, including but not limited to 3d bioprinting techniques (including extrusion 3d bioprinting) such as that described in H.-W. Kang, S. J. Lee, A. Atala and J. J. Yoo, US Patent Application Pub. No. US 2012/0089238 (April 12, 2012). In some embodiments, the depositing step is a patterned depositing step: That is, deposition is carried out so that the deposited composition is deposited in the form of a regular or irregular pattern, such as a regular or irregular lattice, grid, spiral, etc.

In some embodiments, the hydrogel composition containing cells is applied to the central region of a preformed 3D organoid substrate without the cells, resulting in distinct cell-containing zones (*e.g*., tumor cell-containing zones) inside of outer organoid zones.

In some embodiments, cell-free gelatin-only channels may be formed in the organoid substrate, forming channels in the construct that may aid in diffusion.

In some embodiments of general constructs, the cross-linking step increases the stiffness of said hydrogel by from 1 or 5 to 10, 20 or 50 kiloPascals, or more, at room temperature and atmospheric pressure. In some such embodiments, the hydrogel has a stiffness after said cross-linking step *(c)* of from 1 or 5 to 10, 20 or 50 kiloPascals at room temperature and atmospheric pressure.

In some embodiments, the method further comprises the step of depositing a supporting polymer (*e.g*., poly-L-lactic acid, poly(glycolic acid), polycaprolactone; polystyrene; polyethylene glycol, etc., including copolymers thereof such as poly(lactic-co-glycolic acid)) on said substrate in a position adjacent that of said hydrogel composition (*e.g.*, concurrently with, after, or in alternating repetitions with, the step of depositing said hydrogel, and in some embodiments prior to the cross-linking step).

Any suitable substrate can be used for the deposition, including organic and inorganic substrates, and including substrates with or without features such as well, chambers, or channels formed thereon. For the particular products described herein, the substrate comprises a microfluidic device having at least two chambers (e.g., 2, 3, 4, 5, 6, 7, 8, or more chambers) (the chambers optionally but preferably associated with an inlet channel and/or an outlet channel) connected by a primary fluid conduit through which the growth media may circulate, and the depositing is carried out separately in each chamber. In an alternative, the substrate may comprise a first and second planar member (*e.g*., a microscope cover slip), the depositing step may be carried out on that planar member, and the method may further comprise the step of inserting each planar member into a separate chamber of a microfluidic device. Post-processing steps, such as sealing of chambers, and maintaining the viability of cells, may be carried out in accordance with known techniques.

While the present disclosure is described primarily with reference to a single secondary chamber, it will be appreciated that multiple secondary chambers, with the same or different organoids, may be included on the substrate if desired. Thus the secondary chambers can be connected to one another, and the primary chamber, in any suitable configuration, including in series, in parallel, or in combinations thereof.

In some embodiments, an apparatus of the present disclosure may comprise at least three chambers (e.g., 3, 4, 5, 6, 7, 8, or more chambers), with each chamber containing the same or different cells, tissues and/or organoids as another chamber. The at least three chambers may be provided in a single perfusion platform with the at least three chambers arranged and/or connected in any manner. In some embodiments, at least one of the at least three chambers and/or tissues in said at least three chambers comprises metastatic and/or malignant cells. An apparatus may comprise at least a first, second, and third chamber in fluid communication with one another, with, for example, a liver tissue in said first chamber; a cardiac muscle tissue in said second chamber; and a lung tissue in said third chamber. In some embodiments, an apparatus comprises at least six chambers with each chamber comprising one of six different tissues, such as, for example, a liver tissue, a heart tissue, a vasculature tissue, a lung tissue, a colon tissue, or either a testes tissue or an ovary tissue. In some embodiments, an apparatus comprises at least six chambers with each chamber comprising one of six different tissues, such as, for example, a liver tissue, a heart tissue, a brain tissue, a lung tissue, a colon tissue, or either a testes tissue or an ovary tissue. In some embodiments, an apparatus comprises at least five chambers with each chamber comprising one of five different tissues, such as, for example, a liver tissue, a heart tissue, a brain tissue, a lung/vasculature tissue, or either a testes tissue or an ovary tissue.In some embodiments, an apparatus comprises at least six chambers with each chamber comprising one of six different tissues, such as, for example, a liver tissue, a heart tissue, a brain tissue, a lung tissue, a vasculature tissue, or either a testes tissue or an ovary tissue.

In some embodiments, a common aqueous growth media is present in one or more of the chambers of the apparatus (e.g., the at least three or all). The common aqueous growth media may comprise a serum-free endothelial cell media and/or testicular cell media. The common aqueous growth media may be introduced into the at least three chambers in any manner. For example, in some embodiments, the common aqueous growth media may be introduced first into a chamber comprising a testes tissue, then into a chamber comprising a cardiac tissue, etc. The common aqueous growth media circulates and/or flow through each of the one or more chambers by circulating perfusion. The apparatus may draw the common aqueous growth media from a reservoir at a rate in a range of about 5 µL/min to about 50 µL/min, or any range and/or individual value therein, and circulate the media through the one or more chambers. In some embodiments, the apparatus may draw the common aqueous growth media from a reservoir at a rate of about 5, 10, 15, 20, 25, 30, 35, 40, 45, or 50 µL/min or any range and/or individual value therein.

The common aqueous growth media may comprise one or more additional ingredients including, but not limited to, glutamine, glucose, hydrocortisone, ascorbic acid, heparin, an antibacterial agent, an antimicrobial agent, serum, interleukins, neurotrophins, bone morphogenetic proteins, angiopoietin, erythropoietin, stem cell factor, and/or one or more growth factors (particularly mammalian, such as, e.g., human), such as, e.g., epithelial growth factor, vascular endothelial growth factor, fibroblast growth factor beta, hepatocyte growth factor, platelet-derived growth factor, glial cell line-derived neurotrophic factor, keratinocyte growth factor, placental growth factor, and/or Insulin-like growth factor 1.

In some embodiments, the common aqueous growth media may comprise glutamine, optionally glutamine in a concentration in a range of about 0.1 mM to about 5 mM.

The common aqueous growth media may comprise glucose, optionally glucose in a concentration in a range of about 5 mM to about 60 mM.

The common aqueous growth media may comprise (mammalian, particularly human) epithelial growth factor, optionally epithelial growth factor in a concentration in a range of about 0.1 ng/mL to about 20 ng/mL.

The common aqueous growth media may comprise hydrocortisone, optionally hydrocortisone in a concentration in a range of about 0.1 µg/mL to about 2 µg/mL.

The common aqueous growth media may comprise ascorbic acid, optionally ascorbic acid in a concentration in a range of about 0.1 µg/mL to about 50 µg/mL.

The common aqueous growth media may comprise (mammalian, particularly human) vascular endothelial growth factor, optionally vascular endothelial growth factor in a concentration in a range of about 0.1 ng/mL to about 10 ng/mL.

The common aqueous growth media may comprise (mammalian, particularly human) fibroblast growth factor beta, optionally fibroblast growth factor beta in a concentration in a range of about 0.1 to about 10 ng/mL.

The common aqueous growth media may comprise Insulin-like growth factor 1, optionally Insulin-like growth factor 1 in a concentration in a range of about 0.1 ng/mL to about 15 ng/mL.

In some embodiments, the common aqueous growth media may comprise heparin, optionally heparin in a concentration in a range of about 0.1 units/mL to about 5 units/mL.

The common aqueous growth media may comprise an antibacterial and/or antimicrobial agent (*e.g*., penicillin, streptomycin, gentamicin, amphotericin B), optionally the antibacterial and/or antimicrobial agent in a concentration in a range of about 0.1% to about 1% by volume.

In some embodiments, the common aqueous growth media comprises serum *(e.g.,* fetal bovine serum, calf serum, etc.), optionally serum in a concentration in a range of about 0.1% to about 10% by volume.

In some embodiments, the common aqueous growth media comprises a test compound, such as, e.g., a metal (e.g., a heavy metal), drug, and/or pesticide.

An apparatus of the present invention may comprise a sensor operatively associated with the common aqueous growth media. The sensor may be configured to sense toxic responses to drugs, drug candidates, chemical compounds, biological agents, chemical agents, etc., such as, e.g., by responding by reporting the presence, a reduction, and/or an increase in one or more of the following: (i) IL-8; (ii) prostacyclin; (iii) albumin; (iv) urea; (v) LDH; (vi) creatine kinase; (vii) alpha-glutathione-S-transferase; (viii) calcein AM stained viable cells; (ix) propidium iodide or ethidium homodimer stained dead cells; (x) ATP activity; and/or (xi) mitochondrial metabolism. The sensor may comprise an ELISA sensor; colorimetric assay sensor, real time antibody or aptamer sensor, Western blot sensor, trans-endothelial electrical resistance sensor, trans-epithelial electrical resistance sensor, optical video capture apparatus configured to show morphology of tissues, and/or an optical video capture apparatus configured to show dynamic beating or changes in beating behavior in cardiac tissue.

An apparatus of the present invention may be configured to provide a physiological or hyperphysiological fluid to tissue volume ratio. For example, one or more chambers in the apparatus have an average volume in a range of about 2 µL to about 10 µL. In some embodiments, one or more chambers in the apparatus may have an average volume of about 2, 3, 4, 5, 6, 7, 8, 9, or 10 µL. In some embodiments, an apparatus of the present invention (e.g., an apparatus comprising at least 6 chambers) uses liquid in the amount of and/or has a volume of less than about 100 µL, such as, e.g., about 95, 90, 85, 80, 75, 70, 65, 60, 55, 50, 45, 40 µL or less. The volume of the apparatus, as used herein, refers to the volume to fill the chamber(s) and channel(s) of the apparatus. In some embodiments, an apparatus of the present invention (e.g., an apparatus comprising at least 6 chambers) may use liquid in the amount of and/or have a volume of less than about 50 µL. In some embodiments, the volume of the apparatus may be increased by integration and/or use with an external fluid reservoir. The external fluid reservoir may increase the overall system volume and/or aid in controlling the volume of the apparatus.

An apparatus and/or method of the present invention/disclosure may comprise and/or provide one or more (e.g., 1, 2, 3, 4, 5, 6, 7, 8, or more) different tissues and/or organoids (e.g., 3D organoids) that each are viable for at least 1, 2, 3, 4, or more weeks. In some embodiments, an apparatus and/or method of the present invention/disclosure comprises and/or provides one or more (e.g., 1, 2, 3, 4, 5, 6, 7, 8, or more) different 3D tissue constructs that are in fluid communication with each other and a common aqueous growth media, and that are each viable for at least 1, 2, 3, 4, or more weeks. Thus, in some embodiments, two, three, four, five, six, or more different 3D tissue constructs are in fluid communication with each other and a common aqueous growth media, and each is viable for at least 1, 2, 3, 4, or more weeks. In some embodiments, one or more of the 3D tissue constructs may be viable and may comprise at least about 75% or more (e.g., about 80%, 85%, 90%, 95% or more) living cells based on the average number of cells present in the construct at 1, 2, 3, 4, or more weeks. The tissues and/or organoids may be generated by differentiation from a common cell sample *(e.g.,* a sample such as a skin sample collected from a subject). One or more of the tissue constructs may comprise cells in proportions similar to the proportions of cells present in the corresponding native (e.g., human) tissue. In some embodiments, at least one of the tissue constructs comprises metastatic and/or malignant cells. In some embodiments, a function and/or property of the tissue and/or organoid may be determined and/or measured and compared to the function and/or property of a corresponding native tissue (e.g., a property of a liver organoid may be measured and compared to the same property of a liver tissue in a subject). In some embodiments, a function and/or property of the tissue and/or organoid may be similar to the function and/or property of a corresponding native tissue.

The substrate carrying the primary and secondary chambers, associated organoids, inlets, outlets, and conduits, may be provided in the form of an independent "cartridge" or subcombination that may be installed within a larger apparatus in combination with additional components for use. Thus, in some such larger apparatus embodiments, the apparatus further includes a pump operatively associated with the primary chamber for circulating the growth media from the primary chamber to the secondary chamber.

An apparatus of the present invention may comprise a pump operatively associated with one or more of the chamber(s) of the apparatus. In some embodiments the pump may be associated with all of the chamber(s) of the apparatus. The pump may circulate the common aqueous growth media through the one or more (or all) of the chambers of the apparatus.

In some embodiments, the apparatus comprises an oxygenating chamber (*e.g*., a media reservoir) operatively associated with and in fluid communication with one or more (or all) of the chambers of the apparatus.

In some embodiments, the apparatus comprises a (manually operated or automatically controlled) fluid valve positioned next to one or more of the chambers of the apparatus and/or between one or more (e.g., 1, 2, 3, 4, etc.) chambers of the apparatus. The fluid valve may isolate one or a combination of tissue(s) present in the chamber from one another.

In some embodiments, the apparatus further includes *(c)* a cardiac monitor or beat monitor (*e.g*., a camera, electrode or electrode array, etc.) operatively associated with the cardiac construct (*e.g*., for monitoring the beat rate or frequency of the cardiac construct) and optionally operatively associated with the window.

In some embodiments, the apparatus further includes a growth media reservoir and/or bubble trap operatively associated with the primary chamber.

In some embodiments, the apparatus further includes a return conduit operatively associated with the primary and secondary chambers (and the pump, and reservoir and/or bubble trap when present) for returning growth media circulated through the secondary chambers to the primary chamber.

Once produced, subcombination or "cartridge" devices as described above may be used immediately, or prepared for storage and/or transport.

To store and transport the product, a transient protective support media that is a flowable liquid at room temperature (*e.g*., 25° C), but gels or solidifies at refrigerated temperatures (*e.g.*, 4° C), such as a gelatin mixed with water, may be added into the device to substantially or completely fill the chambers, and preferably also the associated conduits. Any inlet and outlet ports may be capped with a suitable capping element (*e.g*., a plug) or capping material (*e.g*., wax). The device may then be packaged together with a cooling element (*e.g.,* ice, dry ice, a thermoelectric chiller, etc.) and all placed in a (preferably insulated) package.

Alternatively, to store and transport the product, a transient protective support media that is a flowable liquid at cooled temperature (*e.g.*, 4° C), but gels or solidifies at warmed temperatures such as room temperature (*e.g*., 20° C) or body temperature (e.g., 37° C), such as poly(N-isopropylacrylamide and poly(ethylene glycol) block co-polymers, may be used.

Upon receipt, the end user may simply remove the device from the associated package and cooling element, allow the temperature to rise or fall (depending on the choice of transient protective support media), uncap any ports, and remove the transient protective support media with a syringe (*e.g*., by flushing with growth media).

An apparatus of the present invention may be used in a method of *in vitro* drug screening, toxicology screening, and/or disease modeling. The method may comprise providing an apparatus of the present invention; and detecting a pharmacological or toxicological response in at least one, or a plurality of tissues present in the apparatus. In some embodiments, an apparatus of the present invention may be used for screening at least one test compound for physiological activity and/or toxicity, by:
*(a)* providing an apparatus of the present invention;
*(b)* optionally circulating a growth medium (e.g., the common aqueous growth medium) from a first chamber to a second chamber;
*(c)* administering at least one test compound to the tissue constructs in the apparatus (*e.g.,* by adding the test compound to the growth medium); and
*(d)* detecting a pharmacological and/or toxicological response to the at least one test compound (e.g., determining a change in beat frequency of the cardiac construct such as, *e.g.,* with the cardiac monitor). In some embodiments, detecting the pharmacological and/or toxicological response may include comparing the response to the response (or lack of a response) observed when the test compound is not administered.

In some embodiments, the at least one test compound comprise at least two distinct test compounds that are administered concurrently with one another, for example, to test for drug interactions there between.

In some embodiments, the at least one test compound comprises a heavy metal, and may be used, for example, to model environmental heavy metal toxicity for each tissue construct.

In some embodiments, the test compound may comprise cadmium, such as, e.g., CdCl₂, optionally in a concentration in a range of about 0.1 µM to about 100 µM.

In some embodiments, the test compound may comprise chromium (VI), such as, e.g., CrO₃, optionally in a concentration in a range of about 0.1 µM to about 10 µM.

In some embodiments, the test compound may comprise chromium (III), optionally in a concentration in a range of about 1 µM to about 100 µM.

In some embodiments, the test compound may comprise lead, such as, e.g., PbCl₂, optionally in a concentration in a range of about 5 µM to about 5 mM.

In some embodiments, the test compound may comprise mercury, such as, e.g., HgCl₂, optionally in a concentration in a range of about 0.2 µM to about 20 µM.

In some embodiments, the test compound may comprise arsenic, such as, e.g., arsenic (III) (e.g., As₂O₃), optionally in a concentration in a range of about 0.05 µM to about 50 µM.

In some embodiments, the detecting step is carried out a plurality of times sequentially spaced from one another *(e.g.,* at least two occasions spaced at least a day apart).

In some embodiments, the method comprises circulating the growth media through all of the chambers in the apparatus for a time of one or more day(s), such as, for example, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more days. In some embodiments, the circulating step may be carried out for at least a time sufficient to allow biomarkers, such as, e.g., stress biomarkers, to achieve about non-cellular media control levels. The circulating step may be carried out for at least a time *(i)* sufficient for IL-8 and/or prostacyclin inflammatory marker concentrations in said common aqueous growth media to decline; *(ii)* during which albumin and/or urea concentrations in said media remain substantially stable; *(iii)* during which LDH and/or creatine kinase levels in said media remain substantially the same as the same media under control conditions; and/or *(iv)* during which alpha-glutathione-S-transferase levels in said media remain substantially the same as the same media under control conditions. In some embodiments, the at least one test compound may be administered after the circulating step is carried out for a sufficient time for non-cellular media control levels to be achieved and/or for *(i)-(iv)* as described above to be achieved.

In some embodiments, the method may comprise determining and/or measuring a function and/or property of a tissue and/or organoid in a chamber in the apparatus and comparing the function and/or property of the tissue and/or organoid to the function and/or property of a corresponding native tissue (e.g., a property of a liver organoid may be compared to the same property of a liver tissue in a subject).

In some embodiments, the method may comprise administering a chelating agent to the tissue construct and/or organoid in the apparatus, such as, e.g., by adding the chelating agent to the growth medium. Example chelating agents include, but are not limited to, dimercaprol, dimercaptosuccinic acid, dimercapto-propane sulfonate, penicillamine, ethylenediamine tetraacetic acid, cysteine, N-acetyl-L-cysteine, glutathione, alpha-lipoic acid, ascorbic acid, alpha-tocopherols, seleninium, quercetin, and/or caffeic acid.

The methods and/or apparatus of the present invention/disclosure may be used, among other things, for the assessment of cellular metabolism, including metabolism of a particular test compound, or cellular toxicity induced by said by a particular test compound, or an interaction thereof.

Aspects of the present invention/disclosure are explained further in the following non-limiting experimental examples.

### EXAMPLES

### Example 1

The use of bioengineered tissues to create a body-on-a-chip platform was investigated. We have previously bioengineered tissues that have been implanted in patients successfully ¹²⁻¹⁴. Implementation of highly specialized primary cells, such as hepatocytes ¹⁵ and cardiomyocytes ^{16,17} for drug discovery applications has proven to be a technically difficult and expensive process ¹⁸. In addition to being able to source the adequate tissue-specific human cells for our bioengineered constructs, incorporation of supportive cells, such as endothelial cells and fibroblasts, as well as structural matrix proteins, can allow for recapitulation of the dynamic interactions of cells with each other, and with the cellular microenvironment.

The next challenge is to combine multiple tissue or organ types within a single microfluidic device to model a simple organism-on-a-chip for drug and therapeutic studies. After all, in the human body, tissues and organs are interconnected and highly interdependent on one another. Organ-on-a-chip systems that comprehensively and accurately mimic human tissues and model disease are limited, and there are fewer still in which multiple organs are represented in an integrated fashion. This is an important technological limitation, as tissue and organ development and function in the human body does not occur in isolation. Conversely, it is essential that organs receive vascular, neural, metabolic and hormonal signals and support to function normally. In drug screening, for instance, toxic effects in secondary tissues can be as important as effects at the target site. If undetected, these effects can lead to an unnecessarily high rate of failure or withdrawal from the market due to negative side effects. Likewise, in tumor metastasis, in which malignant tumor cells migrate from one tissue to another location, multiple tissue or organ sites as well as a circulatory system (vascular or lymphatic) are key components of the system ¹⁹. As such, while useful for many applications, single organoid models have limited efficacy for recapitulating the complex physiological interactions between multiple tissues that occur naturally in the human body. Multi-organoid platforms are a necessity and the logical progression of organ-on-a-chip technology. Several recent such systems have been recently developed. In one such example, a 4-tissue system was developed in a pumpless perfusions platform that integrated 2D tissue cultures of liver, cardiac, skeletal muscle, and neuronal compartments within a single device. This platform was employed to demonstrate basic cell toxicity in drug screens with Doxorubicin, Atorvastatin, Valproic Acid, Acetaminophen and N-Acetyl-m-aminophenol.²⁰ In another example, a microphysiological microfluidic platform was developed containing preformed intestine and skin constructs, liver spheroids, and a kidney epithelial barrier tissue model, in which basic function, gene expression, and viability were maintained for 28 days.²¹ These examples represent important steps towards systems that can mimic complex responses and interactions between tissues during drug and toxicology screens.

In this study, we aim to further advance these technologies, and describe the development and testing of a six-tissue body-on-a-chip system comprising liver, cardiac, vascular, lung, testes, and colon models. Each tissue model was created with the cell types present in the native human tissue, with similar cell-type proportions if possible. Bioprinting ²²⁻²⁶ with customized tissue-specific "bio-inks" ^{27,28} and microfluidic technologies were employed to precisely and reproducibly integrate liver, cardiac, and testes tissue organoids into modular perfusable devices that were connected to lung and vascular barrier tissue modules, and a smooth muscle and epithelium colon construct. This system was then employed to assess physiological responses to drugs and toxic agents. Onboard this six-organoid body-on-a-chip system, organoids are capable of responding to a variety of external stimuli independently or in a concerted manner ²⁹, similar to organ dynamics found in the human body, during which integrated prototype biosensor systems can be employed for environmental and biological monitoring. We demonstrate physiological characterization, functional testing, and drug and toxicology screening, showcasing the utility of this new platform.

### RESULTS

### Design of the six-tissue body-on-a-chip platform

The goal of our work was to develop a highly functional microfluidic perfusion-driven six-tissue body-on-a-chip system, containing representations of liver, heart, vasculature, lung, testes, and colon **(****Fig. 1****, panels A-B)**. Importantly, these individual tissue types would be constructed in modular microfluidic hardware devices, or microreactors **(****Fig. 1****, panel B),** formed by conventional polydimethyl-siloxane (PDMS) soft lithography and molding,^{11,30,31} allowing simple "plug and play" capabilities for platform initialization. This strategy allows continual evolution of the overall platform to contain additional tissue constructs or organoids. Because of the specific individual requirements of each type of tissue model, a toolbox of biofabrication techniques was employed to create each tissue model **(****Fig. 1****, panels C-E)**. Briefly, using hanging drop methodology, spherical liver organoids were formed with primary human hepatocytes, stellate cells, and Kupffer cells, spherical cardiac organoids and testes organoids were formed using human induced pluripotent stem (iPS) cells. These spherical organoids were then bioprinted (**Fig. 7**) into the microreactors using a liver ECM-derived bio-ink, a fibrin and gelatin bio-ink, and a hyaluronic acid (HA) and gelatin bio-ink, employing crosslinking of the hydrogel bio-inks to immobilize the organoids as larger hydrogel-organoid constructs within the perfusion system. Vascular and lung modules were fabricated in a similar form factor in microreactors, but with semi-porous membranes immobilized within the devices on which human umbilical vein endothelial cells (vascular) or lung fibroblasts and lung epithelial cells were seeded. A custom-built trans-endothelial electrical resistance (TEER) sensor was employed, which was integrated into the endothelium module device itself. This sensor measures the effective resistance across the endothelium using 2 gold electrodes on either side of the monolayer, allowing monitoring of endothelial or epithelial integrity. Lastly, colon organoids were formed by molding collagen Type I gels with colon smooth muscle cells and epithelial cells, resulting in aligned collagen submucosal tissue containing epithelial acini structures **(****Fig. 1****, panel E)**. Following biofabrication, devices were sealed and connected to the circulatory perfusion system through a central fluid routing breadboard, driven by a micro-perisaltic pump. Extensive individual tissue model characterization data is described in **Figs. 8-17****,** including histology, immunostaining, metabolic assays, basic drug toxicity testing, and additional functional tests, demonstrating the physiological relevancy of each tissue model type.

### Six-tissue baseline characterization

Ultimately, a body-on-a-chip device should support a variety tissue types under a common medium under baseline conditions with little toxicity. The platform described above was assembled and initiated under circulating perfusion at 10 µL/min drawing media from a 5 mL reservoir via a micro-peristaltic pump. The common media contained a 1:1 mix of serum-free endothelial cell media and testicular cell media. Viability was shown to be relatively high at both days 7 and 14 as visualized by LIVE/DEAD staining and imaging by macro-confocal microsopy **(****Fig. 2****, panels A-B)** Interestingly, a panel of secreted biomarkers **(****Fig. 2****, panels C-H)** indicate an initial increase in inflammatory markers that attenuates, while throughout the study other markers of toxicity remain low while some functional markers are present and consistent. Specifically, baseline function of the liver (albumin and urea) remain relatively constant and significantly higher than media controls for the duration of the study, indicating good viability and function **(****Fig. 2****, panels C-D).** Likewise, LDH, a general marker of toxicity for a variety of tissue types when released from cells into the media, and CK, a marker of toxicity in cardiomyocytes, stay relatively low - at similar levels to the media controls - indicating low toxicity **(****Fig. 2****, panels E-F).** Interestingly, IL-8 and prostacyclin, proteins released from lung epithelium and vascular endothelium, respectively, initially spike, but decrease to baseline levels over time, suggesting that the lung and vascular constructs experienced stress upon system initiation, but improve over time **(****Fig.** 2, **panels G-H).** These data suggest that the multi-organoid system self-regulates itself, conditioning the media over time to better support each tissue type, perhaps similar to how the human body functions.

### Dynamic-responding vascular modules

Vascular endothelium modules **(****Fig. 1****, panel D)** expressed classical endothelial markers, including CD31, von Willebrand Factor (vWF), and VE-Cadherin (**Fig. 3****, panels a-c).** Additionally, viability of the endothelium was high, as demonstrated by LIVE/DEAD staining **(****Fig. 3****, panel d).** To assess functional response, a custom-built trans-endothelial electrical resistance (TEER) sensor was employed, which was integrated into the endothelium module device itself **(****Fig. 3****, panels e-f).** Briefly, this sensor measures the effective resistance across the endothelium using 2 gold electrodes on either side of the monolayer. An intact monolayer results in increased resistance, while a disrupted monolayer results in decreased resistance **(****Fig. 3****, panel f).** First, the TEER sensor was validated by assessing resistance across the endothelium as the monolayer became confluent over time. **Fig. 3****, panel g** shows an increase in resistance as the endothelial cells proliferate and populate the membrane versus a no-cell media control that stays constant.

Next, the TEER sensor was used to assess the effects of histamine on endothelium integrity. In the body, release of histamine from platelets results in disruption of the endothelium. Under normal media conditions, resistance across endothelium layers was consistently about 0.54 kQ. Upon adding 100 µM histamine, resistance dropped significantly (p < 0.05) to approximately 0.4 kQ, and remained constant for 10 minutes. When fresh media was added to wash the histamine out, resistance increased significantly (p < 0.05), returning to the original baseline level **(****Fig. 3****, panel h).**

One important function of the vascular endothelium is to serve as a selective barrier against both cells and molecules moving to the blood stream and into tissues, and vice versa. As such, we sought to further demonstrate the responsiveness of the vascular module together with the capability to modulate passage of soluble molecules from an adjacent organoid-containing space. New vascular modules were prepared with either fully confluent endothelial layers or non-confluent layers. For this experiment, liver organoids were immobilized in liver-specific bioink, as described, in the bottom chamber of the modules **(****Fig. 3****, panel f).** These were imaged in parallel to the mass transport experiment by macro-confocal microscopy, showing the endothelium on the semi-porous membrane (membrane not visible) and the 3D liver organoid below **(****Fig. 3****, panel g)**. Importantly, for the mass transport experiment, the bioink also contained a soluble fluorescent dye consisting of soluble 15 kDa gelatin conjugated with Alexa Fluor 594 maleimide tags in media. Upon addition of the liver organoids, bioink, and dye in the bottom chamber of the device, devices were sealed, and experiments were initiated. Modules were maintained under 25 µL/min flow with normal media coming from a media reservoir, during which aliquots from the outlet ports were collected every 5 minutes. After 20 minutes, the reservoir was replaced with a reservoir containing 100 µM histamine **(****Fig. 3****, panel h)**. Aliquots were analyzed following the experiment for fluorescent intensity. Aliquots from devices with confluent monolayers had low levels of fluorescence (50-90 RFUs) before introduction of histamine, after which fluorescent intensity increased gradually, reaching nearly 200 RFUs at 45 min **(****Fig. 3****, panel i)**. In particular, the RFU levels of aliquots from the final 3 time points (35, 40, and 45 min) were significantly greater (p < 0.05) than those from earlier time points (10, 15, and 20 min), demonstrating that disruption of endothelium integrity by histamine allowed transfer of the dyed media into the circulation above. Importantly, the non-confluent control devices failed to withhold dye transfer from the start of the experiment. As a result, increased RFU levels were observed at the first 3 time points, and decreased over the course of the experiment.

### Comparison of drug toxicity in 2D cell cultures versus 3D organoids

Unfortunately, many drugs have made it through preclinical studies and clinical trials, after which they existed on the market, for years in some cases, before being recalled by the Food and Drug Administration (FDA) for having toxic effects in humans. Notably, the 90% of drugs that are pulled from the market are pulled due to effects on the liver and the heart. To demonstrate the utility of our platform, we screened several of these drugs, which are summarized in **Table 1.** These include liver toxins troglitazone, mibefradil, bromfenac, and tienilic acid, as well as cardiac toxins, astemizole, pergoglide, terodiline, rofecoxib, and valdecoxib.

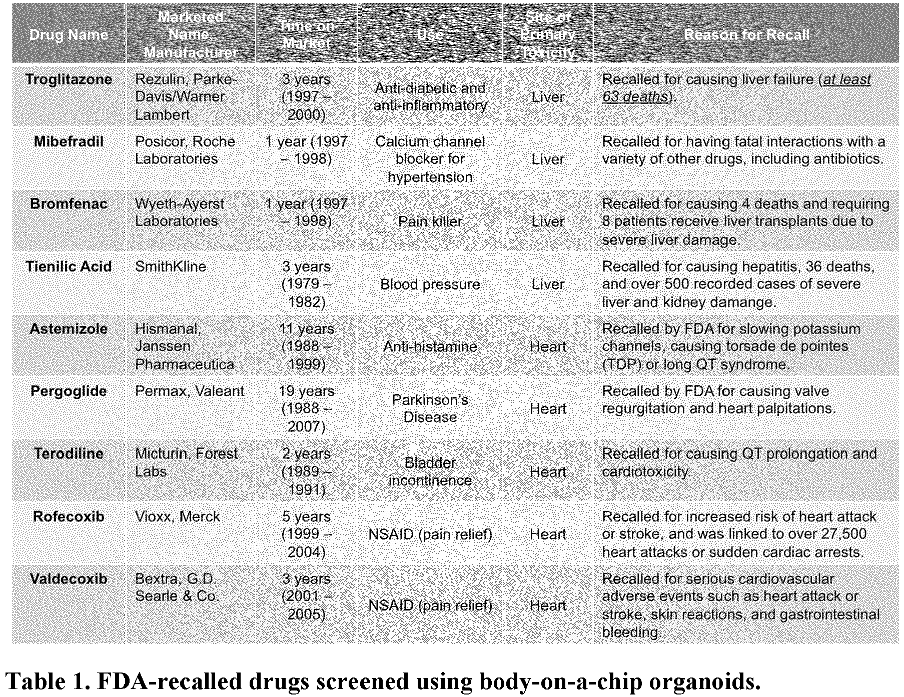

**Fig. 4** shows changes in cellular viability as a function drug concentration. Specifically, liver organoids experience increases in cell death as concentrations of troglitazone increase from 0 to 1, 2, and 3 µM and concentrations of mibefradil increase from 0 to 1, 10, and 100 µM **(****Fig. 4****, panel a).** Likewise, cardiac organoids experience increases in cell death as concentrations of astemizole increase from 0 to 100 nM, 1 µM, and 10 µM, concentrations of pergoglide increase from 0 to 1, 10, and 100 µM, and concentrations of terodiline increase from 0 to 100 µM (**Fig. 4****, panel b**).

In comparison to 3D organoid systems, we also screened these drugs using 2D primary hepatocyte and iPS cardiomyocyte cultures, and 2D hepg2 hepatoma cells and C2C12 myoblast cultures. **Fig. 5** summarizes the effects of these drug screens on ATP activity for each tissue model type. In order to allow comparisons between the different cell model form factors, which employed different numbers of cells (liver organoids - 1500; cardiac organoids - 2500; 2D hepatocytes - 20000; 2D cardiomyocytes - 12500; HepG2 and C2C12 cultures - 20000), ATP levels were normalized to no drug baseline levels.

These data demonstrate a key characteristic of 3D to 2D comparisons; namely that, while the sensitivities of these models to drug treatments often differ, the trend is not necessarily consistent. Specifically, with respect to liver drug screens, 3D organoids were significantly more sensitive to both 2D systems **(****Fig. 5****, panel a).** While, both 3D organoids and 2D hepatocyte cultures were more sensitive to mibefradil in comparison to 2D HepG2 cultures **(****Fig. 5****, panel b)** Conversely, both 3D organoids and 2D hepatocyte cultures were slightly more resistant to bromfenac in comparison to 2D HepG2 cultures **(****Fig. 5****, panel c).** Finally, in response to tielinic acid, the different form factors did not experience significantly different responses **(****Fig. 5****, panel d).**

With respect to cardiac drug treatments, similar variations in response were observed. The 3D cardiac organoids were significantly more sensitive to both astemizole and pergoglide at low to medium concentrations compared to the 2D systems **(****Fig. 5****, panels e-f)**. The 3D organoids were also slightly more sensitive to terodiline than the 2D systems, but only at the highest concentration of 100 µM **(****Fig. 5****, panel g)**. On the other hand, the 2D cardiomyocyte cultures showed increased sensitivity to both rofecoxib and valdecoxib compared to the 3D organoids and 2D C2C12 cultures, but only at low to medium drug concentrations **(****Fig. 5****, panels h-i)**. These results demonstrate that there are clear differences in cellular response to some types of drugs depending on whether or not the cells are in a 3D organoid architecture or in a 2D monolayer, or if the cells are high functioning primary cells or differentiated iPS-derived cells versus typically less functional, established immortalized cell lines.

With respect to cardiac drug screens, the results in **Fig. 5** certainly have some significant differences. However, in particular for terodiline, rofecoxib, and valdecoxib, the overall response trends are remarkably similar. It should be noted that most drugs that are recalled for causing cardiac toxicity are not actually recalled due to cardiac cell death, but rather for causing changes in cardiac function, such as beating kinetics. While 2D cardiomyocyte cultures can maintain beating activity, consistency and reproducibility can be difficult to maintain. Moreover, C2C12 cells are myoblasts and do not beat. These facts make it more difficult to compare beating kinetics between these cell model types. However, as demonstrated in **Fig. 15****, the** 3D cardiac organoids respond consistently to drug administration, and in fact, as shown in **Fig. 19**, also show detrimental changes in beating kinetics as a response to astemizole, pergoglide, and terodiline, which as described, were withdrawn for causing changes in heart beat kinetics in patients (**Table 1**).

### Importance of multi-organoid interactivity in drug testing

In the human body, organs interact with one another in complex ways. To demonstrate that the organoid platform can also support multi-organoid interactions, experiments were performed in which the functionality of the downstream cardiac construct was dependent on the upstream liver construct metabolism. The modular nature of the fluidic system was employed to construct such a platform. Real-time visual monitoring of cardiac organoids was achieved using an onboard LED and camera system that was customized to integrate with the cardiac construct microreactor housing (**Fig. 6****, panel a**) ³². This system allowed real time video capture **(****Fig. 6****, panel b).** Using custom written MatLab code with a series of MatLab functions, moving pixels in each frame were determined against time, generating a binary representation of beat propagation **(****Fig. 6****, panel c)** and plots visualizing beating rates.

Effects of epinephrine and propranolol were first tested independently with a cardiac-only system or the tandem system, before being tested jointly in both systems. Treatment with propranolol only (0.1 µM) resulted in a small (~10%), but significant (p < 0.05) decrease in beating rate in the cardiac-only system. However, in the presence of the liver construct, there was no decrease in beating rate, indicating metabolism of the drug **(****Fig. 6d****).** Similarly, treatment with epinephrine only (0.5 µM) resulted in a significant (~40%) increase in beating rate in the cardiac-only system. Addition of the liver component did not negate the epinephrine induced beating rate increase, but reduced the increase from approximately 40% to 30% (p < 0.05, **Fig. 6****, panel d**), further demonstrating the integrated organoid system response.

Next, the interplay between both drugs in the cardiac-only and tandem systems was assessed. Drug concentrations of 0.1 µM propranolol and 0.5 µM epinephrine were chosen based on the results described in **(****Fig. 14****).** Propranolol was administered first after which epinephrine was subsequently added, and depending on which organoids were present, the effect of epinephrine would vary **(****Fig. 6****, panel e).** Beating plots for each condition are depicted in **Fig. 6****, panels f-i**. In Group 1, which did not have liver organoids, 0.1 µM propranolol remained active, and successfully blocked the beta-adrenergic effects of 0.5 µM epinephrine. This was expected, as there was no liver component to metabolize the blocking agent. In Group 2, in which the liver component was present, a 1.25 fold increase in BPM was observed after the epinephrine was administered. This was compared to a 1.5 fold increase in cardiac BPM in experimental controls where no propranolol was administered prior to epinephrine treatment. This suggests that the 3D liver organoids metabolized enough of the propranolol to allow the epinephrine to activate a significant percentage of the beta adrenergic receptors of the cardiac organoids, inducing the equivalent of approximately 50% of the control epinephrine-only response. This highlights the advantage that multiple organoid systems have compared to single organoid systems. Interestingly, conditions in Group 2 were repeated using a 2D hepatocyte culture comprised of 1-2 million cells on tissue culture plastic versus the 50,000 cells used in making the 3D organoids within the microreactor. The 2D cultures failed to achieve any restoration of the epinephrine-induced increase in beat rate (**Fig. 6****, panel e**), further demonstrating the lack of sufficient metabolic activity in 2D cultures compared to 3D systems.

### DISCUSSION

Development of new drug candidates has been limited and made incredibly expensive due to the failure of accurately modeling human tissues *in vitro,* while animal models allow only limited manipulation, and are not necessarily predictive of results in humans. Traditionally, *in vitro* drug and toxicology testing has been performed using cell lines in 2D cultures. Despite yielding countless medical discoveries, 2D cultures fail to recapitulate the 3D microenvironment of *in vivo* tissues ^{4,6,7}. By transitioning to 3D systems such as tissue organoids, many of these shortcomings may be overcome. Organoids have the capability to respond to drugs and toxins in many of the same manners as actual human organs do, and as such, may provide an improved platform for drug screening applications. Furthermore, recent advances of platforms that integrate multiple models of different tissue types^{20,21} seek to provide additional capabilities that take into consideration the complex interactions that can occur between different tissues and organs in the body.

In the work presented here, we describe a platform that supports up to six distinct bioengineered tissue models - liver, cardiac, vascular, lung, testes, and colon - within a single recirculating perfusion system under a common media with high viability and few indications of toxicity **(****Fig. 2****).** We then describe applications of the components and combinations of the components of the platform to showcase the potential such a body-on-a-chip platform may have. These examples include a responsive modular vascular endothelial module **(****Fig. 3****),** a series of drug screens using FDA-recalled drugs (**Figs. 4****,** **5****,** and **19**), a demonstration of a two organoid interdependent dual drug response **(****Fig. 6****),** and a variety of individual organoid/tissue model characterization **(****Figs. 7-17****),** including substantial histological and functional assays, as well as a demonstration of acetaminophen toxicity and counteraction by N-acetyl-L-cysteine **(****Fig. 13****).**

Importantly, when the six tissue type constructs were integrated within a single perfusion platform, we were able to maintain relatively high viability in all six modules for 2 weeks. While other groups have demonstrated the capability to maintain multi-tissue model system viability for longer - up to 4 weeks - to date, these examples have been limited to up to 4 tissue types.^{20,21} Moreover, the majority of the organoids or tissue models in these systems have been in the form of 2D cultures or 3D cell lines. We had originally hypothesized that with each tissue type that was added to the body-on-a-chip platform, the more complex the media would have to be in order to maintain a sufficient level of viability in the cells. Surprisingly, we found that this did not appear to be the case. In our demonstration of the 6-tissue platform, we biased the common media towards what we believed would be the more sensitive cells populations; specifically, the endothelial cells which were in a planar organization, and the testes organoids. Remarkably, we observed that the other 4 tissue types in the system performed well. Viability assays showed low numbers of dead cells. Soluble biomarker analysis revealed an initial spike in IL-8, prostacyclin, and in some systems CK, representing stress experienced by the lung, vascular, and cardiac constructs, respectively. However, over the time course of the system run, these biomarkers returned to levels close to non-cellular media controls. This interesting result suggests that perhaps the presence of higher numbers of tissue types, results in conditioning of the media towards a naturally-derived, supportive universal media of sorts. Additionally, we believe that once cells are placed in physiologically relevant 3D architectures, they may be more robust, and therefore less influenced by changes media formulations. These theories are notable, because they suggest that multi-organoid platforms comprised of 3D tissue models may be able to move away from more expensive tissue-specific media formulations currently on the market, and towards more inexpensive and simple common media.

Importantly, we wish to discuss the differences between what might be considered simple cell aggregates and 3D organoids. The liver and cardiac organoids that we subsequently bioprint into tissue-on-a-chip constructs are formed by the hanging drop method, as is described. However, these organoids far surpass the functionality associated with simple cell aggregates. The term "cell aggregate" communicates a physical cluster of cells. Aggregates have been employed for many years, but traditionally do not necessarily have high tissue functionality, where as our organoids do. Furthermore, the customization we perform to provide a more supportive microenvironment, provide additional characteristics to the system that have beneficial biological effects ^{28,33}.

The majority of *in vitro* organoid models are being developed to screen drugs and toxins ^{9,10}. Importantly, these systems have the potential to identify toxic effects of drug candidates in development before they advance to clinical trials and are administered to patients. Unfortunately, many drugs pass clinical trials that do have detrimental side effects, reach market, and are used by patients before being recalled by the FDA. If technologies such as body-on-a-chip platforms were more available during development, and importantly, more widely accepted, researchers may have identified these problems early, saving actual human lives and billions of dollars in lawsuits. In this context, we chose to demonstrate our platform utility by screening a panel of FDA-recalled drugs for toxic effects. We successfully captured liver toxicity from troglitazone and mibefradil and cardiac toxicity and beating rate decreases from astemizole, pergogide, and terodiline. Complications with the liver and heart account for approximately 90% of all drug failures and recalls, and as such, being able to demonstrate the ability to capture these outcomes is incredibly important. Our parallel studies using 2D control cultures further demonstrate the potential value of our platform. For example, in the case of liver, 2D cultures of primary hepatocytes and the long-used HepG2 hepatoma cell line resulted in results that varied in comparison to the organoids. In some cases, the organoids were more sensitive and in some cases the organoids were more resistant to the drugs. Likewise, these kinds of variable results - increased sensitivity to some drugs and increased resistance to others - were observed in cardiac organoid drug screens. These results indicate that the relationships between drug toxicity in the body versus 3D organoids versus 2D cultures are complex. One cannot simply say that 3D systems are more sensitive than 2D cultures. The particular sensitivity, and assay appropriateness, are very much dependent on the particular drug in questions. We believe that this is due to several factors: The substantial surface area versus volume ratio differences between 3D and 2D cultures; The buffering that may occur within the interior of 3D organoid and construct volumes; and the specific biological mechanisms that each individual drug targets within the cells. As such, we are currently investigating these factors and the specific mechanisms involved.

More important than individual organoid responses to drugs is a multi-organoid system response, in which the responses of one organoid have implications on the responses of other organoids, like actual human physiology. To explore this concept, individual liver and cardiac organoids were assembled into a dual organ system. Since healthy liver can efficiently metabolize propranolol, rendering it ineffective at blocking cardiac beta-receptors, the effects of propranolol blocking and epinephrine-based beta receptor activation was evaluated with and without liver organoids. In systems with no liver organoids, propranolol remained in its active form and successfully blocked epinephrine from inducing cardiac beating rate increases. However, when 3D liver organoids were introduced into the system, they metabolized the propranolol, and upon administration of epinephrine, beating rates increased, indicating significant inactivation of the drug by the liver construct. This may be one of the first documented instances or integrated responses of multiple *in vitro* organoids within one system. Notably, when hepatocyte cultures in 2D were substituted for the 3D liver organoids, propranolol blocked epinephrine's effects as if no liver cells were present at all, despite a 40X increase in cell number in 2D.

The work described here demonstrates the creation of organoids and tissue constructs that show functionality similar to native human organs. The integration of six distinct bioengineered tissue models in single recirculating perfusion system, comprised of the liver, the heart, vasculature, lung, testes, and colon is also demonstrated. Importantly, the individual components of the platform respond appropriately to a panel of drugs, including a variety of drugs that were removed from the market by the FDA due to toxicity in humans. Even more importantly, when combinations of organoids are combined into a single platform, more complex integrated responses can be observed, where the functionality of one organoid influenced the response of another organoid to drugs was realized. This system, and others like it, based on 3D human-based tissue models with nuanced and complex response capabilities, has massive potential for influencing how *in vitro* drug and toxicology screening and disease modeling is performed in the future.

### REFERENCES:

1 Seruga, B., Ocana, A., Amir, E. & Tannock, I. F. Failures in Phase III: Causes and Consequences. Clin Cancer Res 21, 4552-4560, doi:10.1158/1078-0432.CCR-15-0124 (2015).
2 Jamieson, L. E., Harrison, D. J. & Campbell, C. J. Chemical analysis of multicellular tumour spheroids. Analyst 140, 3910-3920, doi:10.1039/c5an00524h (2015).
3 Sung, J. H. et al. Using physiologically-based pharmacokinetic-guided "body-on-a-chip" systems to predict mammalian response to drug and chemical exposure. Exp Biol Med (Maywood) 239, 1225-1239, doi:10.1177/1535370214529397 (2014).
4 Kunz-Schughart, L. A., Freyer, J. P., Hofstaedter, F. & Ebner, R. The use of 3-D cultures for high-throughput screening: the multicellular spheroid model. J Biomol Screen 9, 273-285, doi: 10.1177/1087057104265040 (2004).
5 Pasirayi, G. et al. Low cost microfluidic cell culture array using normally closed valves for cytotoxicity assay. Talanta 129, 491-498, doi:10.1016/j.talanta.2014.06.020 (2014).
6 Ho, W. J. et al. Incorporation of multicellular spheroids into 3-D polymeric scaffolds provides an improved tumor model for screening anticancer drugs. Cancer Sci 101, 2637-2643, doi:10.1111/j.1349-7006.2010.01723.x (2010).
7 Drewitz, M. et al. Towards automated production and drug sensitivity testing using scaffold-free spherical tumor microtissues. Biotechnol J 6, 1488-1496, doi:10.1002/biot.201100290 (2011).
8 Bhushan, A. et al. Towards a three-dimensional microfluidic liver platform for predicting drug efficacy and toxicity in humans. Stem Cell Res Ther 4 Suppl 1, S16, doi:10.1186/scrt377 (2013).
9 Polini, A. et al. Organs-on-a-chip: a new tool for drug discovery. Expert Opin Drug Discov 9, 335-352, doi:10.1517/17460441.2014.886562 (2014).
10 Benam, K. H. et al. Engineered in vitro disease models. Annu Rev Pathol 10, 195-262, doi:10.1146/annurev-pathol-012414-040418 (2015).
11 Skardal, A., Devarasetty, M., Soker, S. & Hall, A. R. In situ patterned micro 3D liver constructs for parallel toxicology testing in a fluidic device. Biofabrication 7, 031001, doi:10.1088/1758-5090/7/3/031001 (2015).
12 Atala, A., Bauer, S. B., Soker, S., Yoo, J. J. & Retik, A. B. Tissue-engineered autologous bladders for patients needing cystoplasty. Lancet 367, 1241-1246, doi:10.1016/S0140-6736(06)68438-9 (2006).
13 Raya-Rivera, A. et al. Tissue-engineered autologous urethras for patients who need reconstruction: an observational study. Lancet 377, 1175-1182, doi:10.1016/S0140-6736(10)62354-9 (2011).
14 Raya-Rivera, A. M. et al. Tissue-engineered autologous vaginal organs in patients: a pilot cohort study. Lancet 384, 329-336, doi:10.1016/S0140-6736(14)60542-0 (2014).
15 Jouin, D. et al. Cryopreserved human hepatocytes in suspension are a convenient high throughput tool for the prediction of metabolic clearance. Eur J Pharm Biopharm 63, 347-355, doi:10.1016/j.ejpb.2006.01.014 (2006).
16 Kaneko, T., Kojima, K. & Yasuda, K. An on-chip cardiomyocyte cell network assay for stable drug screening regarding community effect of cell network size. Analyst 132, 892-898, doi:10.1039/b704961g (2007).
17 Peters, M. F., Scott, C. W., Ochalski, R. & Dragan, Y. P. Evaluation of cellular impedance measures of cardiomyocyte cultures for drug screening applications. Assay Drug Dev Technol 10, 525-532, doi:10.1089/adt.2011.442 (2012).
18 Lin, C., Ballinger, K. R. & Khetani, S. R. The application of engineered liver tissues for novel drug discovery. Expert Opin Drug Discov 10, 519-540, doi:10.1517/17460441.2015.1032241 (2015).
19 Skardal, A., Devarasetty, M., Forsythe, S. D., Atala, A. & Soker, S. A reductionist metastasis-on-a-chip platform for in vitro tumor progression modeling and drug screening. Biotechnol Bioeng In press. (2016).
20 Oleaga, C. et al. Multi-Organ toxicity demonstration in a functional human in vitro system composed of four organs. Sci Rep 6, 20030, doi:10.1038/srep20030 (2016).
21 Maschmeyer, I. et al. A four-organ-chip for interconnected long-term co-culture of human intestine, liver, skin and kidney equivalents. Lab Chip 15, 2688-2699, doi:10.1039/c51c00392j (2015).
22 Kang, H. W. et al. A 3D bioprinting system to produce human-scale tissue constructs with structural integrity. Nat Biotechnol In Press.
23 Murphy, S. V. & Atala, A. 3D bioprinting of tissues and organs. Nat Biotechnol 32, 773-785, doi:10.1038/nbt.2958 (2014).
24 Skardal, A. & Atala, A. Biomaterials for integration with 3-d bioprinting. Ann Biomed Eng 43, 730-746, doi:10.1007/s10439-014-1207-1 (2015).
25 Skardal, A., Zhang, J. & Prestwich, G. D. Bioprinting vessel-like constructs using hyaluronan hydrogels crosslinked with tetrahedral polyethylene glycol tetracrylates. Biomaterials 31, 6173-6181, doi:S0142-9612(10)00561-2 [pii] 10.1016j.biomaterials.2010.04.045 (2010).
26 Skardal, A., Zhang, J., McCoard, L., Oottamasathien, S. & Prestwich, G. D. Dynamically crosslinked gold nanoparticle - hyaluronan hydrogels. Adv Mater 22, 4736-4740, doi:10.1002/adma.201001436 (2010).
27 Skardal, A. et al. A hydrogel bioink toolkit for mimicking native tissue biochemical and mechanical properties in bioprinted tissue constructs. Acta Biomater, doi:10.1016/j.actbio.2015.07.030 (2015).
28 Skardal, A. et al. Tissue specific synthetic ECM hydrogels for 3-D in vitro maintenance of hepatocyte function. Biomaterials 33, 4565-4575, doi:S0142-9612(12)00321-3 [pii] 10.1016j.biomaterials.2012.03.034 (2012).
29 Esch, M. B. et al. How multi-organ microdevices can help foster drug development. Adv Drug Deliv Rev 69-70, 158-169, doi:10.1016/j.addr.2013.12.003 (2014).
30 Xia, Y. & Whitesides, G. M. Soft Lithography. Annu Rev Mater Scie 28, 153-184 (1998).
31 Skardal, A., Devarasetty, M., Soker, S. & Hall, A. R. In situ patterned micro 3-D liver constructs for parallel toxicology testing in a fluidic device. Biofabrication In press (2015).
32 Zhang, Y. S. et al. A cost-effective fluorescence mini-microscope for biomedical applications. Lab Chip 15, 3661-3669, doi:10.1039/c51c00666j (2015).
33 Skardal, A. et al. A hydrogel bioink toolkit for mimicking native tissue biochemical and mechanical properties in bioprinted tissue constructs. Acta Biomater 25, 24-34, doi:10.1016/j.actbio.2015.07.030 (2015).

### Example 2

Environmental heavy metal studies are proposed and the tissue organoid types for the proposed environmental heavy metal studies have been chosen based on known bioaccumulation, biodistribution and toxicity in fish and mammal tissue. This provides some baseline for evaluating data collected from stand-alone and MPS.

### Tissue Organoid Production:

Organoids are comprised entirely of primary human cells or human iPS-derived cells, and are combined in physiologically accurate ratios to generate highly functional and responsive tissue models. They contain combinations of natural matrix and tunable mechanical properties that are tissue type specific and support both viability and function. The cellular compositions for each organoid type are described below.

Liver: Liver organoids are formed with primary human hepatocytes (Triangle Research Labs), stellate cells (ScienceCell), and Kupffer cells (Gibco), which are aggregated in non-adherent round bottom wells.

Heart: Cardiac organoids are formed using iPS cell-derived cardiomyocytes and primary fibroblasts (Stem Cell Theranostics), aggregated as above. These organoids differentiate into atrial, ventral and nodal cardiomyocyte subtypes.

Brain: Human iPS cells will be differentiated in a step-wise neural differentiation protocol through embryoid body formation. Embryoid bodies will be kept in suspension in neuronal precursor (hNPC) medium. After two weeks, embryoid bodies will be plated as single-cell adherent cultures. For neuronal differentiation, early passage neuronal precursor single-cell suspension will be cultured under constant gyratory shaking. After four days, the medium will be changed and aggregates will be kept in differentiation medium for up to 4 weeks to induce simultaneous differentiation of NPCs into glutamatergic, GABAergic, dopaminergic neurons as well as astrocytes and oligodendrocytes.

Vascular: Vascular modules are formed by culturing endothelial cells on one side of a semi-porous silicon membrane. The planar construct is fitted in a microreactor device with parallel circulation systems.

Lung: 3D airway organoids, modeled on the structure and cellular organization that are present in the airways, are fabricated in three layers, also on a semi-porous membrane. Organoids contain lower layer: lung microvasculature endothelial cells (Lonza), Middle layer: airway stromal mesenchymal cells (donor derived), and Upper layer: bronchial epithelial cells (Lonza) cultured directly upon the stromal layer.

Testis: Testis organoids are formed using primary cell culture of spermatogonia, leydig, sertoli and peritubular myloid cells, aggregated as above.

Ovary: Ovary follicle organoids are produced with human granulosa and theca cells that are isolated from human tissue and expanded in culture. These cells are then aggregated (in a layered construct: granulosa cells surrounded by theca cells) to form an engineered follicle. These structures produce the expected hormones and the level of hormone secretion is self-regulated by feedback from the medium. These structures are able to mature oocytes isolated from ovary tissue under specific culture conditions.

### Device Fabrication:

Thin, silicone tapes that can be self-aligned through folding and layered to form microfluidic structures will be used in these studies **(****Fig. 20A****)**. This approach removes the need for cleanroom processes or precise layer-by-layer alignment and can be achieved through a variety of widely-available and inexpensive patterning technologies that are amenable to mass production, including the use of a computer-controlled razor plotter. Lateral device resolution in these structures is sufficient for the intended purpose while depth is defined by the thickness of the adhesive film layers used. These dimensions are adequate for the support of sub-microliter fluid handling as well as cell culture constructs containing populations of cells such as those contained within the described tissue organoids. This design strategy is compatible with the valves, mixers, and other functional systems used to drive the microfluidic circuit. Additionally, one common flaw associated with many tissue-chip platforms is a non-physiological fluid-tissue volume ratio. Our fabrication system allows for creation of microreactor chambers with an average volume of 7 µL. In the case of this 6-tissue platform, in which 6 chambers and associated connecting channels exist, each device uses less than 100 µL of cell culture media.

### Organoid-Device Integration:

To address the challenges of integrating 3D tissue organoids within a microfluidic system, we have developed a methodology for *in situ* fabrication that utilizes a hyaluronic acid (HA) and gelatin-based hydrogel, HyStem (ESI-BIO, Alameda, CA). This material has been employed extensively in tissue engineering, and unlike conventional materials such as collagen, Matrigel, and alginate, is easily integrated with variety of biofabrication techniques. Furthermore, since HA and gelatin are natural components of native ECM, it provides a truly biomimetic substrate in the form of crosslinked HA polysaccharide chains and cell-adherent hydrolytically degraded collagen gel.

In the general approach to fabricating cell culture constructs, HA and gelatin components are mixed with tissue organoids, as well as a crosslinker and photoinitiator to support thiol-acrylate photopolymerization. Each organoid type is added to the ungelled substrate at a density such that the ratios of organoid volumes in the final integrated construct match the volume ratios of organs in the human body (e.g. the mass of liver organoids are five times the mass of heart organoids.) Each organoid laden substrate is introduced to the microfluidic chambers sequentially and patterning is accomplished using a positive-tone photomask to define the shape and location of each construct **(****Fig. 20B****).** The cross-linked hydrogel is adherent to the top and bottom surfaces of the chamber, allowing it to be retained under fluid flow conditions. This photo-patterning can be performed in an arbitrary number of independent microfluidic chambers. The resulting 3D constructs can subsequently be kept under circulating flow with long-term viability, and the total system is amenable to analytical investigation, including both biochemical assays and direct imaging on chip.

Additional patterning (e.g. with additional cell or organoid types) can also be used to produce multi-component structures, enabling significant system complexity. Notably, the HyStem platform also supports incorporation of solubilized extracellular matrix, supplying additional biomolecular factors specific to each tissue organoid as described above. It has been shown that such *in vitro* constructs recapitulate a broad range of physiological activities and reactions observed *in vivo,* highlighting the biomimetic nature of the system. Overall, this fabrication approach is rapid, inexpensive, and modular, with straightforward potential to be mass-produced for a large number of parallel experiments. **Fig. 20C** shows a complete device with a total footprint of a microscope slide containing the six constructs proposed for both the *male* and female version of the MPS platform (Though this system includes vasculature rather than gut.). **Fig. 20D** shows viability of each organoid prepared via this method at one week of culture.

### Evaluation of Single Organoid System:

Experiments will be performed on independent systems containing each of the five common tissue organoid types, as well as both the ovary and testicular organoids. Six-organoid constructs will be maintained for 5 days to allow time for stabilization of the system. Each heavy metal will be introduced to the system medium on day 5 in the chemical form and concentrations described below:
Cadmium: CdCh; Concentrations of 0.1, 1, 10, 100 µM.
Chromium(VI): CrO₃; Concentrations of 0.1, 1, 10 µM.
Chromium(III): Cr; Concentrations of 1, 10, 100, and 100 µM.
Lead: PbCh; Concentrations of 5, 50, and 500 µM, and 5 mM.
Mercury: HgCh; Concentrations of 0.2, 2, 20 µM.
Arsenic(III): As₂O₃; Concentrations of 0.05, 0.5, 5, 50 µM.
During the course of the project, these doses may be shifted or expanded, as necessary.

### Basic Assessment:

Twenty-five percent partial medium changes will be performed every five days, and heavy metals will be included in the added medium at the appropriate concentration. Organoids will be harvested at various time points after heavy metal exposure. Media aliquots will be reserved and frozen for offline metabolomics and biomarker assays. Organoids will be assessed for viability by LIVE/DEAD staining and ATP activity. Preliminary data for liver, cardiac, and testes are shown in **Figs. 21** **and** **22** using thallium, lead, mercury, and glyphosate (active agent in Roundup); demonstrate that these organoids do indeed respond to acute heavy metal (and other environmental toxin) exposures. In general, traditional soluble biomarkers **(Table 2)** will be assessed by onboard electrochemical antibody or aptamer biosensing. ELISA or colorimetric assays will be performed on media collected at each time point as controls to ensure accuracy of the electrochemical monitors.

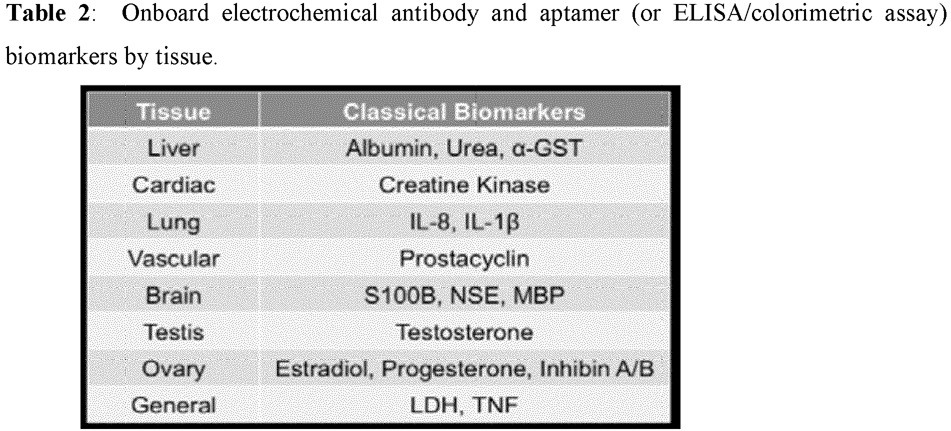

### Bioaccumulation of environmental metals within each organoid type:

Tissue Organoids will be harvested from the microreactors at the indicated time points. The organoids will be washed and homogenized in buffer. Heavy metal content in the organoid lysates will be measured by inductively coupled plasma spectroscopy (Intertek Allentown Analytical Services, Allentown, PA). This technique is quantitative for multiple metals, simultaneously, and can be normalized by the ratio of organoid volume for each tissue type. An additional advantage of using plasma spectroscopy is the ability to measure iron, copper and zinc, as heavy metals can displace these physiologically necessary metals in enzymes and other proteins; perhaps contributing to toxicity.

### Histological assessment:

Fluorescent cell death assays will be performed along with immunohistochemical identification of individual cell types. These data will be used to give a distribution of cell viability for each cell type. Additionally, histopathological evaluation of each organoid type will be used to identify cellular and microanatomical changes within metal exposed organoids.

### Gene Induction:

Organoids will be processed for RNA extraction by standard methods. Transcriptome analysis will performed using a stress response array. mRNA levels of 370 key genes which are involved in toxicological adverse outcomes including oxidative Stress and others will be measured by qPCR with the Qiagen Human Molecular Toxicology PathwayFinder RT² Profiler^{™} Results will be calculated as Fold-Change using the recommended control genes on the array. Data will be analyzed with AmiGO 2 and PANTHER overrepresentation in the Gene Ontology database and pathway enrichment. Results will be verified and Complemented by GORILLA.

Additionally, whole transcriptome sequencing will be conducted for key experiments to identify other stress response gene sets that may be upregulated in response to heavy metal exposure. Organoids will be processed for RNA extraction by standard methods. Transcriptomes will be measure using Agilent RNA microarray analysis (performed by WFUHS Genomics core). Whole genome transcription data will then be integrated and analyzed for molecular network interactions including bio-functional, toxicological and canonical pathways using standard software packages such as KEGG, Agilent Genespring and WGCNA by weighted gene correlation network analysis.

### Free Radical Production:

All of the heavy metals used in the proposed studies have the potential to drastically alter the redox chemistry within and surrounding tissue organoids. The redox potential of the medium within the microfluidic circuit will be monitored by an in line redox sensor developed by our team. Superoxide anions in the medium will be detected by highly sensitive electrochemical sensors based on nanoporous gold immobilized with cytochrome-c. Oxidation of this substrate by free radicals in the medium will result in changes in the measured current levels that can be used to calculate the amount of free radicals in the medium.

### Lipid Partitioning:

A portion of the harvested organoids will be lysed by sonication. Ultracentrifugation will be used to isolate organoid microsomes. Heavy metal concentrations within the microsome fraction will be compared to heavy metal content in the aqueous cellular component identified and quantitated by inductively coupled plasma spectroscopy will be used to determine lipid partitioning within each tissue organoid type.

### Statistical Analysis:

Experiments will be performed with n=3 samples or greater; based on variance identified in early experiments. Data will be presented as mean ± standard deviations. Two main statistical approaches will be used as appropriate: 1) One-way ANOVA models to detect whether measures are changing over time or not; and 2) Paired t-tests to compare outcomes between two groups or specific time points. Our team has access to several statistical cores, including the Biostatistics, Epidemiology, and Research Design Core and biostatistician consultants at Wake Forest College of Medicine.

### Data interpretation:

Heavy metal accumulation, stress gene induction, and toxicity for each heavy metal will be compared to published data for each of these experimental readouts.

### Evaluation of the integrated MPS platform:

A six organoid-organoid integrated MPS will be assembled in a microfluidic chip. The microfluidic chips reported to date have not taken into account that the gut (splanchnic) circulatory system is partially independent from the peripheral circulation. Microfluidic circuit patterning on the bioreactor chip will recirculate media through the liver bioreactor with a small portion of this circulation being fed into the general circulation. Microfluidic circuits will be fabricated to divert 90% of the first microfluidic circuit back through the liver construct. This will ensure that introduced compounds make several passes across the liver module before entering the general circulation. Data collected from these microcircuits will be compared to standard uniform fluid distribution microcircuits to determine if inter-organoid circulation dynamics affect modeling of heavy metal toxicity. Systems will contain the five common organoid types, plus either ovary or testis, in series. Acute toxicity, heavy metal bioaccumulation, biodistribution of metals among organoids, and histological/molecular response characterizations will be performed as described previously.

### Evaluation of Metal Chelation Therapy:

The goal of this experiment is to identify toxicity associated with the use of the chelators, and to determine if this possible toxicity is outweighed by the beneficial effects of chelation on MPS exposed to heavy metals at various concentrations. Notably, while chelators have been investigated for beneficial effects in cases of lead, mercury, and arsenic poisoning, no studies with these heavy metals have been performed using 3D organoid models. Moreover, to date there is no known specific antidote for chromium or cadmium toxicity.

### Chelating Agents, Concentrations, and Associated Targets:

Effects of the chelators alone will serve as a control for negative consequences of chelation therapy. Additionally, while these agents have not been shown to be effective at treating chromium and cadmium poisoning, we will screen these agents following chromium and cadmium exposure in order to generate a baseline from which future combinatorial therapies will be tested. The chelating agents to be tested are provided in **Table 3.**

**Table 3: Chelating agents to be tested for efficacy in the MPS models and integrated system.**

| **Chelating Agent** | **Concentration Range** | **Target** |
|---|---|---|
| Dimercaprol | 0.1 uM- 10 mM | Arsenic, Mercury, Lead |
| Dimercaptosuccinic acid | 0.1 uM- 10 mM | Arsenic, Mercury, Lead |
| Dimercapto-propane sulfonate | 0.1 uM- 10 mM | Arsenic, Mercury |
| Penicillamine | 0.1 uM- 10 mM | Arsenic, Lead |
| Ethylenediamine tetraacetic acid | 0.1 uM- 10 mM | Lead |

### Side Effect Assessment:

Liver enzymes, liver metabolism, neural toxicity, inflammation, cardiac arrhythmias, altered neurochemical signal transduction, and barrier function of lung are potential effects of the chelating agents that will be monitored during and after compound screens.

### Heavy Metal Toxicity:

Toxicity, heavy metal bioaccumulation, biodistribution of metals among organoids, and histological/molecular response characterizations will be performed as described previously.

### Data interpretation:

Results will be compared among systems treated with chelators alone, chelators administered concurrently with heavy metals, and chelators administered subsequent to metal exposure. These comparisons will be used to identify negative side effects, prophylactic sequestration of circulating metals, and removal of metals from tissue organoids, respectively.

### Evaluation of supplements:

The six-organoid MPS will be used to determine the effects of supplemental therapies for heavy metal exposure. Generally, these supplements increase the free radical scavenging potential in tissues and serum. Additionally, several over the counter treatments that claim to remove heavy metals from tissue will be evaluated. These compounds are available through the internet or health stores, but have not been approved by the FDA for heavy metal treatment. Several of these include low doses of known chelators, some do not. The agents to be tested are provided in **Table 4.**

**Table 4: Additional agents that act against free radicals and reactive oxygen species that will be tested individually and in concert with chelating agents in the MPS models and integrated system.**

| **Supplement Compound** | **Concentration Range** |
|---|---|
| Cysteine | 5 uM - 50 mM |
| N-acetyl-L-cysteine | 5 uM - 50 mM |
| Glutathione | 5 uM - 50 mM |
| Alpha-lipoic acid | 5 uM - 5 mM |
| Ascorbic acid | 0.5 uM - 50 mM |
| Alpha-tocopherols | 0.5 uM - 50 mM |
| Seleninium | 0.05 uM - 5 mM |
| Quercetin | 0.5 uM - 5 mM |
| Caffeic acid | 0.5 uM - 50 mM |

### Supplements to Chelating Agents:

Sulfhydryl-containing compounds have long been known to aid in metal chelation. As such, these compounds will be screened individually, and in combination with the chelating agents described in the previous aim. Concentrations are equivalent to human doses.

### Over the counter chelation therapy (OTC):

OTC heavy metal chelators will be selected based on groupings of common ingredients and claimed mechanism of action. Several concentrations will be selected based on manufacturer recommended dose. Examples include KelaminHM ^{™} (EDTA based), Pure Encapsulations Detoxification ^{®} - HM Complex (pectin based), Mercury Detox 60 ^{™} (amino acids and other natural macromolecules).

### Data interpretation:

Results will be compared among systems treated with either supplements or OTC chelators alone. These data will indicate potential toxic effects of the agents. OTC chelators administered concurrently with or subsequent to heavy metal administration will be used to determine prophylactic sequestration of circulating metals, and removal of metals from tissue organoids, respectively. Combined chelation/supplement treated systems will be compared to chelation therapy alone to determine whether these supplements decrease toxicity or affect metal chelation. Together, these experiments will test the safety and efficacy of antioxidant supplements and OTC chelation therapy for the treatment of heavy metal intoxication in an integrated human MPS.

### Example 3

The intent of these experiments is to assess how heavy metal toxicity affects each of the organoids in an five organoid MPS comparing to traditional cell culture and animal reference. The five organoid MPS will contain the five organoid types - liver, heart, lung/vascular, brain, and testes - in series. This comparison will describe the fidelity of MPS for modeling heavy metal toxicity. All experiments will be performed on integrated systems containing each of the five tissue organoid types.

MPS Strategy: A 5 organoid-organoid integrated MPS will be assembled in a microfluidic chip as described above in Example 2. Microfluidic circuit patterning on the bioreactor chip will recirculate media through the liver bioreactor with a small portion of this circulation being fed into the general circulation. Microfluidic circuits will divert 90% of the first microfluidic circuit back through the liver construct. This will ensure that introduced compounds make several passes across the liver module before entering the general circulation. Data collected from these microcircuits will be compared to standard uniform fluid distribution microcircuits to determine if inter-organoid circulation dynamics affect modeling of heavy metal toxicity. Systems will contain the five organoid types - liver, heart, lung/vascular, brain, and testes - in series.

Heavy Metal Administration: 5-organoid MPS devices will be maintained for 5 days to allow time for stabilization of the system. Each heavy metal will be introduced on day 5 in the chemical form and concentrations described below. Compounds will initially be administered to the MPS platforms at the following concentrations which were based on values described in the literature and preliminary acute toxicity studies. During the course of the project, these doses may be shifted or expanded, as necessary.
Cadmium: CdCl2; Concentrations of 0.1, 1, 10, 100 µM.
Chromium(VI): CrO3; Concentrations of 0.1, 1, 10 µM.
Chromium(III): Cr; Concentrations of 1, 10, 100, and 100 µM.
Lead: PbCl2; Concentrations of 5, 50, and 500 µM, and 5 mM.
Mercury: HgCl2; Concentrations of 0.2, 2, 20 µM.
Arsenic(III): As2O3; Concentrations of 0.05, 0.5, 5, 50 µM.

Basic assessment: ¼ partial medium changes will be performed every five days, and heavy metals will be included in the added medium at the appropriate concentration. Organoids will be harvested at various time points after heavy metal exposure. Media aliquots will be reserved and frozen for offline biomarker assays **(Table 2)**. Organoids will be assessed for viability by LIVE/DEAD staining and ATP activity. Preliminary data for liver, cardiac, and testes are shown in **Fig. 23** using thallium, lead, mercury, and glyphosate (active agent in Roundup) demonstrate that these organoids do indeed respond to acute heavy metal (and other environmental toxin) exposures.

Bioaccumulation of environmental metals within each organoid type: Organoids will be harvested from the MPS the end of studies, at which heavy metal content in the organoid lysates will be measured by inductively coupled plasma spectroscopy (Intertek Allentown Analytical Services, Allentown, PA). This technique is quantitative for multiple metals, simultaneously, and can be normalized by the ratio of organoid volume for each tissue type. An additional advantage of using plasma spectroscopy is the ability to measure iron, copper and zinc, as heavy metals can displace these physiologically necessary metals in enzymes and other proteins, perhaps contributing to toxicity.

Histological assessment: Fluorescent cell death assays (annexin V [apoptosis] vs. Ki67 [proliferation]) will be performed along with immunohistochemical identification of individual cell types. These data will be used to give a distribution of cell viability for each cell type. Additionally, histopathological evaluation of each organoid type will be used to identify cellular and microanotomical changes within metal exposed organoids. Outcomes, possible challenges, and proposed solutions (SA 1) Based on our work described above, in which our team has successfully maintained a six-organoid platform, it is anticipated that that 3D organoids platform will condition the common media over time, creating an environment that is hospitable to all of the specific cell types within the included organoids. While the volume of the system used is quite low (<50 ul), the fluid to tissue volume ratio is hyperphysiologic. It is possible that published biodistribution of heavy metals is based on the order in which organs are exposed to these metals within an organism. This may explain why gut and liver problems are encountered more frequently than muscle problems. Because of the high fluid to volume ratio in the MPS, it may be difficult to test this notion. The order of organoids may be switched to determine if this order affects biodistribution. Additionally, data from single organoid systems may be generated to determine if organ order is affecting bioaccumulation of heavy metals.

The single organoid MPS will be used to model the effects of the chelators alone, as well as and the combined effect of chelators with environmental heavy toxins. The main goal of this study is to identify toxicity associated with the use of the chelators, and to determine if this possible toxicity is outweighed by the beneficial effects of chelation on MPS exposed to heavy metals at various concentrations. Notably, while chelators have been investigated for beneficial effects in cases of lead, mercury, and arsenic poisoning, no studies with these heavy metals have been performed using 3D organoid models. Moreover, to date there is no known specific antidote for chromium or cadmium toxicity. As such, this creates a significant opportunity to deploy the integrated MPS platform to investigate potential therapies for exposure to these toxins. 5-organoid MPS will be used to determine the effects of supplemental therapies for heavy metal exposure. Generally, these supplements increase the free radical scavenging potential in tissues and serum.

Chelating Agents: Effects of the chelators alone **(Table 3)** will serve as a control for negative consequences of chelation therapy. Additionally, while these agents have not been shown to be effective at treating chromium and cadmium poisoning, we will screen these agents following chromium and cadmium exposure in order to generate a baseline from which future combinatorial therapies will be tested.

Supplements to Chelating Agents: Sulfhydryl-containing and anti-oxidant compounds have long been known to aid in metal chelation.31 As such, these compounds **(Table 4)** will be screened individually, and in combination with chelating agents described above.

Side Effect Assessment: Liver enzymes, liver metabolism, neural toxicity, inflammation, cardiac arrhythmias, altered neurochemical signal transduction, and barrier function of lung are potential effects of the chelating agents that will be monitored during and after compound screens.

Heavy Metal Toxicity: Toxicity, heavy metal bioaccumulation, biodistribution of metals among organoids, and histological/molecular response characterizations will be performed as described previously. Outcomes, possible challenges, and proposed solutions (SA 2) Supplements that act as antioxidants may behave differently in MPS as compared to humans due to the hyperphysiological fluid to tissue ratio in MPS. The large volume of antioxidant containing medium surrounding the tissue organoids may actually be more effective at scavenging free radicals. A range of doses will be used, scaled down dose based on the relationship between human fluid:tissue and MPS fluid:tissue (~1/100). This study will focus on antioxidant supplements and OTC chelators. However, several other agents have been suggested as possible treatments for heavy metal exposure. Additional agents that may aid in heavy metal clearance, or mitigate side effects associated with chelation therapy may be tested as resources allow. For example, some chelators have been linked to the side effects such as liver damage, neural function, inflammation, cardiac arrhythmias, and loss of tissue barrier function. Potential treatments for these side effects will be identified using the National Library of Medicine Drug Information Portal database.

Data Interpretation and Statistical Analysis: Data collected from the metal exposed integrated MPS will be compared to published values for acute toxicity and bioaccumulation in traditional two-dimensional culture, experimental animals, and humans. Initial studies will validate that the 6-organoid MPS maintains high viability and stable tissue function. Subsequent toxicity screens will assess 1) whether individual organoid metal bioaccumulation and toxicity correlates with responses measured in the integrated system, and 2) Unexpected tissue organoid responses resulting from heavy metal exposure in the integrated system.

### Example 4-5 organoid integrated drug screening

Aim of Experiment: To assess dependence of multi organoid toxicity on liver organoid metabolic function. As an example, the metabolic activity of the liver can heavily influence the outcome and efficacy of some drugs. For instance, 5FU, a common 1^{st} line cancer drug is cytotoxic to many cells in the body, not only tumors. As such, it is usually given in the form of a prodrug, capecitabine, which is essentially inert until it passes through the liver and is metabolized into its active form.

Design and Results of Experiments: Five tissue platforms were initiated containing liver, cardiac, lung, testis, and brain organoids and maintained for 7 days prior to the start of the drug study. At this point, the liver modules were removed from half of the platforms. Capecitabine (20 uM) was administered to all platforms after which viability was assessed through live/dead staining or cardiac beating behavior after 7 days of exposure (day 14 of total study).

The results demonstrated that with liver present, capecitabine is metabolized into the toxic drug 5-FU, causing heart and lung toxicity (**Fig. 24**). Without liver, this metabolism does not occur, and cardiac and lung organoid viability does not decrease. Surprisingly, brain organoids suffered in both groups (**Fig. 24**). The LIVE/DEAD results show that liver organoids are required to metabolize capecitabine to the active 5-FU form of the drug, inducing increased cell death in cardiac and lung organoids.

Soluble biomarkers, including urea, albumin, α-GST, IL-8, and IL-1β, were quantifed from media aliquots at days 1, 3, 5, 7, 9, 11, 13, and 15. (**Fig. 26**). Media aliquots were sent for mass spectrometry analysis to verify metabolism of capecitabine to 5-FU.

### Example 5 - 5 organoid integrated drug screening in a miniaturized microfluidic platform

A microfluidic platform having a miniaturized footprint and fluid volume was prepared and it was assessed whether the same complex drug studies are possible on this platform. Minimizing fluid volume may result in increased signal to noise ratio of pertinent biomarkers in the system circulation.

Design and Results of Experiments: Adhesive film-based microfluidic platforms were fabricated as described below. As with the full-scale platform described in Example 4, five tissue constructs were initiated containing liver, cardiac, lung, testis, and brain organoids and maintained for 7 days prior to the start of the drug study. At this point, the liver modules were removed from half of the platforms. Capecitabine (20 uM) was administered to all platforms after which viability was assessed through live/dead staining or cardiac beating behavior after 7 days of exposure (day 14 of total study).

In the miniaturized system, with liver present, capecitabine is metabolized into the toxic drug 5-FU, causing heart and lung toxicity (**Fig. 25**). Without liver, this metabolism does not occur, and cardiac and lung organoid viability does not decrease. Importantly, in this platform the brain organoid viability is high (**Fig. 5**). The LIVE/DEAD results show that liver organoids are required to metabolize capecitabine to the active 5-FU form of the drug, inducing increased cell death in cardiac and lung organoids. Moreover, brain organoids are viable, suggesting, but not wishing to be bound to any particular theory, that perhaps the scaled down volume in the system results in a better conditioned media that supports brain organoid viability. This demonstrates that the brain organoids can be maintained in this system together with the other organoid types.

Soluble biomarkers, including urea, albumin, α-GST, IL-8, and IL-1β, were quantifed from media aliquots at days 7 and 15 (**Fig. 26**). Media aliquots were sent for mass spectrometry analysis to verify metabolism of capecitabine to 5-FU.

### Miniaturized system design:

This system reduces or eliminates the surface area of PDMS exposed to media compared to other systems, such as the standard size system, thereby reducing chances for adsorption or absorption of drug compounds, toxins, soluble proteins, and secreted compounds onto or into the device walls. The device manufacturing strategy is based on tape microfluidics, laser cut PMMA, and some PDMS molding, thereby significantly minimizing the amount of PDMS surface area in contact with the media of the device. **Fig. 27** provides an overview of this fabrication methodology.

### Example 6 - Environmental Toxin Screening

Liver and cardiac organoids were subjected to 48-hour incubations with thallium nitrate (1 µM, 10 µM, 100 µM), lead chloride (100 µM, 1 mM, and 10 mM), glyphosate, the active agent in RoundUp (500 µM, 5 mM, 50 mM), or mercury chloride (2 µM, 20 mM, 200 mM) dissolved in hepatocyte culture media (Lonza) or cardiac maintenance media (Stem Cell Theranostics), respectively. Following compound exposures, organoids were assessed for viability with LIVE/DEAD staining or ATP activity. Organoids displayed dose dependent decreases in viability and ATP activity in response to all three compounds. In addition, cardiac organoids were assessed of impacts of beat rate kinetics.

**Fig. 28** shows the results from the liver organoid environmental toxin screens with panels A-D showing the normalized ATP activity and IC50 value estimation, and panel E showing the LIVE/DEAD staining and macro-confocal imaging of liver organoids treated with glyphosate, lead, thallium, and mercury. * p < 0.05 in comparison to the no drug condition for each toxin screen. **Fig. 29** shows the results from the cardiac organoid environmental toxin screens with panel A-D showing the normalized ATP activity and IC50 value estimation, and panel E showing the LIVE/DEAD staining and macro-confocal imaging of liver organoids treated with glyphosate, lead, thallium, and mercury. * p < 0.05 in comparison to the no drug condition for each toxin screen. **Fig. 30** shows changes in cardiac organoid beat rates as an effect of environmental toxin exposure.

## Claims

1. A multi-tissue body-on-a-chip apparatus, comprising:
a first chamber comprising a first organoid;
a second chamber comprising a second organoid;
a primary fluid conduit that connects said first and second chambers; and
a common aqueous growth media in said first chamber, said second chamber, and said primary fluid conduit;
wherein said first and second chambers are connected in series and are in fluid communication with one another via said primary fluid conduit;
wherein, in operation, the media flows through said first chamber, said second chamber, and said primary fluid conduit by recirculating perfusion;
wherein said first and second organoids are different from each other,
wherein the volume of the apparatus is less than 100 µL,
wherein each of said first chamber and said second chamber has a volume in a range of 2 µL to 10 µL, and
wherein the apparatus provides a hyperphysiological fluid to tissue volume ratio.

2. The apparatus of claim 1, wherein at least one of said first and second organoids is present in a hydrogel, optionally wherein the hydrogel comprises thiolated hyaluronic acid and/or thiolated collagen.

3. The apparatus of claim 2, wherein the hydrogel further comprises a polyethylene glycol (PEG) crosslinker, optionally wherein the PEG crosslinker comprises an alkyne.

4. The apparatus of claim 1, wherein said first and second organoids are viable in said apparatus for at least two weeks.

5. The apparatus of claim 1, wherein the media comprises serum, optionally wherein serum is present in the media in a concentration in a range of 0.1% to 10% by volume.

6. The apparatus of claim 1, wherein the media comprises serum-free endothelial cell media and/or testicular cell media.

7. The apparatus of claim 1, wherein the media flows through said first chamber, said second chamber, and said primary fluid conduit at a flow rate in a range of 5 µL/min to 50 µL/min.

8. The apparatus of claim 1, wherein at least one of said first and second organoids comprises primary cells and/or stem cells.

9. The apparatus of claim 1, wherein at least one of said first and second organoids does not comprise cells from an immortalized cell line.

10. The apparatus of claim 1, wherein at least one of said first and second organoids is a liver organoid.

11. The apparatus of claim 1, wherein said first organoid is a liver organoid and said second organoid is a cardiac organoid.

12. The apparatus of claim 1, further comprising a third chamber comprising a third organoid, wherein the third organoid is different than the first and second organoids;
wherein said first, second, and third chambers are in fluid communication with one another and connected by said primary fluid conduit; and
wherein, in operation, the media flows through said first chamber, second chamber, third chamber, and said primary fluid conduit by recirculating perfusion, optionally wherein said third organoid comprises cancer cells

13. The apparatus of claim 12, wherein said first chamber comprises a liver organoid, said second chamber comprises a cardiac organoid, said third chamber comprises lung organoid and said apparatus further comprises a fourth chamber comprising a testicular organoid or ovarian organoid, a fifth chamber comprising a brain organoid, and a sixth chamber comprising an intestinal organoid.

14. The apparatus of claim 1, wherein the apparatus comprises an adhesive film and/or double sided tape.

## Patentansprüche

1. Body-on-a-Chip-Vorrichtung mit verschiedenem Gewebe, umfassend:
eine erste Kammer, die ein erstes Organoid umfasst;
eine zweite Kammer, die ein zweites Organoid umfasst:
eine primäre Fluidleitung, die die erste und die zweite Kammer miteinander verbindet; und
ein gemeinsames wässriges Wachstumsmedium in der ersten Kammer, in der zweiten Kammer und in der primären Fluidleitung;
wobei die erste und die zweite Kammer in Reihe geschaltet sind und über die primäre Fluidleitung in Fluidverbindung miteinander stehen;
wobei im Betrieb das Medium durch die erste Kammer, die zweite Kammer und die primäre Fluidleitung durch rezirkulierende Perfusion fließt;
wobei die ersten und zweiten Organoiden voneinander verschieden sind,
wobei das Volumen der Vorrichtung weniger als 100 µL beträgt,
wobei sowohl die erste Kammer als auch die zweite Kammer ein Volumen im Bereich von 2 µL bis 10 µL aufweisen, und
wobei die Vorrichtung ein hyperphysiologisches Fluid-Gewebe-Volumenverhältnis bereitstellt.

2. Vorrichtung nach Anspruch 1, wobei mindestens eines des ersten und zweiten Organoids in einem Hydrogel vorhanden ist, wobei das Hydrogel optional thiolierte Hyaluronsäure und/oder thioliertes Kollagen umfasst.

3. Vorrichtung nach Anspruch 2, wobei das Hydrogel ferner einen Polyethylenglykol(PEG)-Vemetzer umfasst, wobei der PEG-Vernetzer optional ein Alkin umfasst.

4. Vorrichtung nach Anspruch 1, wobei das erste und zweite Organoid in der Vorrichtung für mindestens zwei Wochen lebensfähig sind.

5. Vorrichtung nach Anspruch 1, wobei das Medium Serum umfasst, wobei Serum optional in einer Konzentration im Bereich von 0,1 bis 10 Vol.-% in dem Medium vorhanden ist.

6. Vorrichtung nach Anspruch 1, wobei das Medium serumfreies Endothelzellmedium und/oder Hodenzellmedium umfasst.

7. Vorrichtung nach Anspruch 1, wobei das Medium durch die erste Kammer, die zweite Kammer und die primäre Fluidleitung mit einer Durchflussrate im Bereich von 5 µL/min bis 50 µL/min fließt.

8. Vorrichtung nach Anspruch 1, wobei mindestens eines des ersten und zweiten Organoids Primärzellen und/oder Stammzellen umfasst.

9. Vorrichtung nach Anspruch 1, wobei mindestens eines des ersten und zweiten Organoids keine Zellen aus einer immortalisierten Zelllinie umfasst.

10. Vorrichtung nach Anspruch 1, wobei mindestens eines des ersten und zweiten Organoids ein Leberorganoid ist.

11. Vorrichtung nach Anspruch 1, wobei es sich bei dem ersten Organoid um ein Leberorganoid und bei dem zweiten Organoid um ein Herzorganoid handelt.

12. Vorrichtung nach Anspruch 1, ferner umfassend eine dritte Kammer, die ein drittes Organoid umfasst, wobei sich das dritte Organoid von dem ersten und zweiten Organoid unterscheidet;
wobei die erste, zweite und dritte Kammer in Fluidverbindung miteinander stehen und durch die primäre Fluidleitung verbunden sind; und
wobei im Betrieb das Medium durch die erste Kammer, die zweite Kammer, die dritte Kammer und die primäre Fluidleitung durch rezirkulierende Perfusion fließt, wobei das dritte Organoid optional Krebszellen umfasst.

13. Vorrichtung nach Anspruch 12, wobei die erste Kammer ein Leberorganoid umfasst, die zweite Kammer ein Herzorganoid umfasst, die dritte Kammer ein Lungenorganoid umfasst und die Vorrichtung ferner eine vierte Kammer, die ein Hodenorganoid oder ein Eierstockorganoid umfasst, eine fünfte Kammer, die ein Hirnorganoid umfasst, und eine sechste Kammer umfasst, die ein Darmorganoid umfasst.

14. Vorrichtung nach Anspruch 1, wobei die Vorrichtung einen Klebefilm und/oder ein doppelseitiges Klebeband umfasst.

## Revendications

1. Appareil Body-on-a-Chip multi-tissu, comprenant :
une première chambre comprenant un premier organoïde ;
une deuxième chambre comprenant un deuxième organoïde ;
un conduit de fluide primaire qui relie lesdites première et deuxième chambres ; et
un milieu de croissance aqueux commun dans ladite première chambre, ladite deuxième chambre et ledit conduit de fluide primaire ;
dans lequel lesdites première et deuxième chambres sont reliées en série et sont en communication fluidique l'une avec l'autre *via* ledit conduit de fluide primaire ;
dans lequel, en fonctionnement, le milieu s'écoule à travers ladite première chambre, ladite deuxième chambre et ledit conduit de fluide primaire par perfusion recirculante ;
dans lequel lesdits premier et deuxième organoïdes sont différents l'un de l'autre,
dans lequel le volume de l'appareil est inférieur à 100 µL,
dans lequel chacune de ladite première chambre et de ladite deuxième chambre présente un volume dans une plage allant de 2 µL à 10 µL, et
dans lequel l'appareil fournit un rapport volumique hyperphysiologique de fluide sur tissu.

2. Appareil selon la revendication 1, dans lequel au moins un desdits premier et deuxième organoïdes est présent dans un hydrogel, facultativement dans lequel l'hydrogel comprend de l'acide hyaluronique thiolaté et/ou du collagène thiolaté.

3. Appareil selon la revendication 2, dans lequel l'hydrogel comprend en outre un agent de réticulation de polyéthylène glycol (PEG), facultativement dans lequel l'agent de réticulation de PEG comprend un alcyne.

4. Appareil selon la revendication 1, dans lequel lesdits premier et deuxième organoïdes sont viables dans ledit appareil pendant au moins deux semaines.

5. Appareil selon la revendication 1, dans lequel le milieu comprend du sérum, facultativement dans lequel du sérum est présent dans le milieu à une concentration dans une plage allant de 0,1 % à 10 % en volume.

6. Appareil selon la revendication 1, dans lequel le milieu comprend du milieu de cellules endothéliales et/ou du milieu de cellules testiculaires sans sérum.

7. Appareil selon la revendication 1, dans lequel le milieu s'écoule à travers ladite première chambre, ladite deuxième chambre et ledit conduit de fluide primaire à un débit d'écoulement dans une plage allant de 5 µL/min à 50 µL/min.

8. Appareil selon la revendication 1, dans lequel au moins l'un desdits premier et deuxième organoïdes comprend des cellules primaires et/ou des cellules souches.

9. Appareil selon la revendication 1, dans lequel au moins l'un desdits premier et deuxième organoïdes ne comprend pas de cellules provenant d'une lignée cellulaire immortalisée.

10. Appareil selon la revendication 1, dans lequel au moins l'un desdits premier et deuxième organoïdes est un organoïde hépatique.

11. Appareil selon la revendication 1, dans lequel ledit premier organoïde est un organoïde hépatique et ledit deuxième organoïde est un organoïde cardiaque.

12. Appareil selon la revendication 1, comprenant en outre une troisième chambre comprenant un troisième organoïde, dans lequel le troisième organoïde est différent des premier et deuxième organoïdes ;
dans lequel lesdites première, deuxième et troisième chambres sont en communication fluidique les unes avec les autres et sont reliées par ledit conduit de fluide primaire ; et
dans lequel, en fonctionnement, le milieu s'écoule à travers ladite première chambre, ladite deuxième chambre, ladite troisième chambre et ledit conduit de fluide primaire par perfusion recirculante, facultativement dans lequel ledit troisième organoïde comprend des cellules cancéreuses.

13. Appareil selon la revendication 12, dans lequel ladite première chambre comprend un organoïde hépatique, ladite deuxième chambre comprend un organoïde cardiaque, ladite troisième chambre comprend un organoïde pulmonaire et ledit appareil comprend en outre une quatrième chambre comprenant un organoïde testiculaire ou un organoïde ovarien, une cinquième chambre comprenant un organoïde cérébral et une sixième chambre comprenant un organoïde intestinal.

14. Appareil selon la revendication 1, dans lequel l'appareil comprend un film adhésif et/ou un ruban double face.
